# EUROPÄISCHE PATENTSCHRIFT

(11) **EP 1 585 742 B1**
(45) Veröffentlichungstag und Bekanntmachung des Hinweises auf die Patenterteilung: **08.08.2007**
(21) Anmeldenummer: 03789245.2
(22) Anmeldetag: 12.12.2003
(51) Int. Cl.: C07D 401/12, C07D 307/18, C07D 307/20, C07D 309/06, C07D 319/06, A01N 43/56

(54) **SUBSTITUIERTE BENZOYLDERIVATE ALS HERBIZIDE**
SUBSTITUTED BENZOYL DERIVATIVES USES AS HERBICIDES
DERIVES DE BENZOYLE SUBSTITUES UTILISES COMME HERBICIDES

(30) Priorität: 09.01.2003 DE 10301110
(43) Veröffentlichungstag der Anmeldung: 19.10.2005
(73) Patentinhaber: Bayer CropScience AG, 40789 Monheim (DE)
(72) Erfinder: VAN ALMSICK, Andreas, 61184 Karben (DE); WILLMS, Lothar, 65719 Hofheim (DE); Dr.AULER, Thomas, 42799 Leichlingen (DE); BIERINGER, Hermann, 65817 Eppstein (DE); MENNE, Hubert, 55252 Mainz-Kastel (DE)
(86) Internationale Anmeldenummer: PCT/EP2003/014129
(87) Internationale Veröffentlichungsnummer: WO 2004/063187

(56) Entgegenhaltungen:
- WO-A-99/10327
- WO-A-99/10328

## Beschreibung

Die Erfindung betrifft das technische Gebiet der Herbizide, insbesondere das der Herbizide aus der Gruppe der Benzoylcyclohexandione und Benzoylpyrazole zur selektiven Bekämpfung von Unkräutern und Ungräsem in Nutzpflanzenkulturen, insbesondere in Reiskulturen.

Aus verschiedenen Schriften ist bereits bekannt, daß bestimmte Benzoylderivate herbizide Eigenschaften besitzen. So sind aus WO 99/10327 und WO 99/10328 Benzoylcyclohexandione und Benzoylpyrazolone bekannt, die in 3-Position des Phenylrings einen über eine mehratomige Brücke gebundenen Heterocyclyl- oder Heteroaryl-Rest tragen.

Die aus diesen Schriften bekannten Verbindungen zeigen jedoch häufig eine nicht ausreichende herbizide Wirksamkeit.

Aufgabe der vorliegenden Erfindung ist die Bereitstellung von weiteren herbizid wirksamen Verbindungen mit - gegenüber den im Stand der Technik offenbarten Verbindungen - verbesserten herbiziden Eigenschaften.

Es wurde nun gefunden, daß Benzoylderivate, die in 3-Position des Phenylrings bestimmte über eine zweiatomige Brücke gebundene Heterocyclyl-Reste tragen, als Herbizide besonders gut geeignet sind. Ein Gegenstand der vorliegenden Erfindung sind daher Verbindungen der Formel (1) oder deren Salze 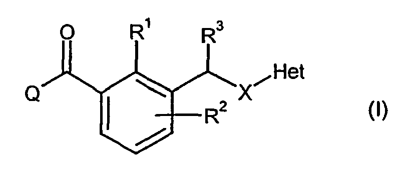 worin die Reste und Indizes folgende Bedeutungen haben:
- R¹, R²: bedeuten unabhängig voneinander Wasserstoff, Mercapto, Nitro, Halogen, Cyano, Rhodano, C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₃-C₆-Cycloalkyl, OR⁴, OCOR⁴, OSO₂R⁴, S(O)ₙR⁴, SO₂OR⁴, SO₂N(R⁴)₂, NR⁴SO₂R⁴, NR⁴COR⁴, C₁-C₆-Alkyl-S(O)ₙR⁴, C₁-C₆-Alkyl-OR⁴, C₁-C₆-Alkyl-OCOR⁴, C₁-C₆-Alkyl-OSO₂R⁴, C₁-C₆-Alkyl-SO₂OR⁴, C₁-C₆-Alkyl-SO₂N(R⁴)₂ oder C₁-C₆-Alkyl-NR⁴COR⁴;
- R³: bedeutet Wasserstoff, C₁-C₆-Alkyl, C₂-C₆-Alkenyl oder C₂-C₆-Alkinyl;
- R⁴: bedeutet Wasserstoff, C₁-C₆-Alkyl, C₂-C₆-Alkenyl, C₂-C₆-Alkinyl, C₃-C₆-Cycloalkyl, Phenyl oder Phenyl- C₁-C₆-alkyl, wobei die sechs letztgenannten Reste durch s Reste der Gruppe Hydroxy, Mercapto, Amino, Cyano, Nitro, Rhodano, OR³, SR³, N(R³)₂, =NOR³, OCOR³, SCOR³, NR³COR³, CO₂R³, COSR³, CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl und C₁-C₄-Alkylsulfonyl substituiert sind;
- Het: bedeutet eine vollständig gesättigte heterocyclische Gruppe, deren Ringatome aus Kohlenstoff- und Sauerstoffatomen bestehen, wobei
p bedeutet die Gesamtzahl der Ringatome,
r bedeutet die Anzahl der Sauerstoffatome,
(p-r) bedeutet die Anzahl der Kohlenstoffatome, und
Het durch n Reste R⁵ substituiert sein kann;
- n: bedeutet 0, 1 oder 2;
- p: bedeutet 5, 6 oder 7;
- r: bedeutet 1 oder 2;
- s: bedeutet 0,1, 2 oder 3;
- X: bedeutet 0 oder S(O)ₙ;
- R⁵: bedeutet Hydroxy, Mercapto, Amino, Cyano, Nitro, Halogen, Formyl, C₁-C₆-Alkylamino, C₁-C₆-Dialkylamino, C₁-C₆-Alkoxycarbonyl, C₁-C₆-Alkylcarbonyl, C₁-C₄-Alkylcarbonyloxy, C₁-C₆-Alkyl, C₁-C₆-Halogenalkyl, C₁-C₆-Alkylthio, C₁-C₆-Halogenalkylthio, C₁-C₆-Alkoxy, C₁-C₆-Halogenalkoxy oder R⁵ bildet zusammen mit dem Kohlenstoffatom, an dem es gebunden ist, eine Carbonylgruppe;
- Q: bedeutet einen Rest Q1 oder Q2; 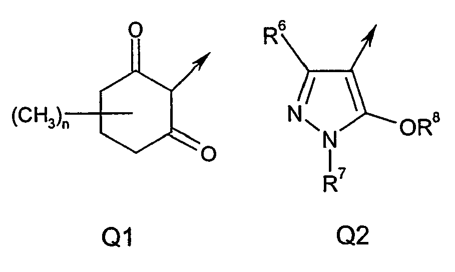
- R⁶, R⁷: bedeuten unabhängig voneinander Wasserstoff, C₁-C₆-Alkyl, C₁-C₆-Halogenalkyl oder C₃-C₆-Cyclopropyl;
- R⁸: bedeutet Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenyloxycarbonyl oder Phenylsulfonyl, wobei der Phenylkern der vier letztgenannten Reste durch s Reste aus der Gruppe Halogen, Nitro, Cyano, C₁-C₆-Alkyl, C₁-C₆-Halogenalkyl, C₁-C₆-Alkoxy und C₁-C₆-Halogenalkoxy substituiert ist.

In Abhängigkeit von äußeren Bedingungen, wie Lösungsmittel und pH-Wert, können die erfindungsgemäßen Verbindungen der Formel (I) in unterschiedlichen tautomeren Strukturen auftreten. Je nach Art der Substituenten enthalten die Verbindungen der Formel (I) ein acides Proton, das durch Umsetzung mit einer Base entfernt werden kann. Als Basen eignen sich beispielsweise Hydride, Hydroxide und Carbonate von Alkali- und Erdalkalimetallen, wie Lithium, Natrium, Kalium, Magnesium und Calcium, sowie Ammoniak und organische Amine wie Triethylamin und Pyridin. Solche Salze sind ebenfalls Gegenstand der Erfindung.

In Formel (I) und allen nachfolgenden Formeln können Alkylreste mit mehr als zwei C-Atomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, bevorzugt Methyl oder Ethyl.

Ist eine Gruppe mehrfach durch Reste substituiert, so ist darunter zu verstehen, daß diese Gruppe durch ein oder mehrere gleiche oder verschiedene der genannten Reste substituiert ist.

Cycloalkyl bedeutet ein carbocyclisches, gesättigtes Ringsystem mit drei bis neun C-Atomen, z.B. Cyclopropyl, Cyclopentyl oder Cyclohexyl. Analog bedeutet Cycloalkenyl eine monocyclische Alkenylgruppe mit drei bis neun Kohlenstoffringgliedern, z.B. Cyclopropenyl, Cyclobutenyl, Cyclpentenyl und Cyclohexenyl, wobei sich die Doppelbindung an beliebiger Position befinden kann. Im Falle zusammengesetzter Reste, wie Cycloalkylalkenyl, kann sich der erstgenannte Rest an beliebiger Position des zweitgenannten befinden.

Unter der heterocyclischen Gruppe sind Reste wie 2-Tetrahydrofuranyl, 3-Tetrahydrofuranyl, 2-Tetrahydropyranyl, 3-Tetrahydropyranyl, 4-Tetrahydropyranyl, 2-Hexahydrooxepanyl, 3-Hexahydrooxepanyl, 4-Hexahydrooxepanyl, 1,3-Dioxolan-4-yl, 1,3-Dioxan-4-yl, 1,3-Dioxan-5-yl und 1,4-Dioxan-2-yl zu verstehen. Bevorzugt ist Het unsubsubstituiert oder durch 1, 2, 3 oder 4 Methylgruppen und/oder 1 oder 2 Carbonylgruppen substituiert.

Im Falle einer zweifach substituierten Aminogruppe, wie Dialkylamino, können diese beiden Substituenten gleich oder verschieden sein.

Halogen bedeutet Fluor, Chlor, Brom oder Iod. Halogenalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl, CH=CHCl, CH=CCl₂, C≡CCH₂Cl; Halogenalkoxy ist z.B. OCF₃, OCHF₂, OCH₂F, CF₃CF₂O, OCH₂CF₃ und OCH₂CH₂Cl; entsprechendes gilt für Halogenalkenyl und andere durch Halogen substituierte Reste.

Ist eine Gruppe mehrfach substituiert, so ist darunter zu verstehen, daß bei der Kombination der verschiedenen Substituenten die allgemeinen Grundsätze des Aufbaus chemischer Verbindungen zu beachten sind, d.h. daß nicht Verbindungen gebildet werden, von denen der Fachmann weiß, daß sie chemisch instabil oder nicht möglich sind.

Die Verbindungen der Formel (I) können je nach Art und Verknüpfung der Substituenten als Stereoisomere vorliegen. Sind beispielsweise ein oder mehrere asymmetrische C-Atome vorhanden, so können Enantiomere und Diastereomere auftreten. Stereoisomere lassen sich aus den bei der Herstellung anfallenden Gemischen nach üblichen Trennmethoden, z.B. durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Als vorteilhaft haben sich Verbindungen der Formel (I) herausgestellt, worin
- R¹, R²: bedeuten unabhängig voneinander Wasserstoff, Nitro, Halogen, C₁-C₄-Alkyl, C₁-C₄-Halogenalkyl, C₂-C₆-Alkenyl, C₂-C₆-Halogenalkenyl, C₂-C₆-Alkinyl, C₂-C₆-Halogenalkinyl, C₃-C₆-Cycloalkyl, -OR⁴, S(O)ₙR⁴, SO₂OR⁴, SO₂N(R⁴)₂, NR⁴SO₂R⁴ oder C₁-C₆-Alkyl-S(O)ₙR⁴;
- R⁴: bedeutet Wasserstoff, C₁-C₄-Alkyl, C₂-C₄-Alkenyl, C₂-C₄-Alkinyl, C₃-C₆-Cycloalkyl, Phenyl oder Phenyl- C₁-C₄-alkyl, wobei die sechs letztgenannten Reste durch s Reste der Gruppe Cyano, Nitro, R³, OR³, SR³ oder N(R³)₂ substituiert sind, und die anderen Substituenten und Indices jeweils die weiter oben genannten Bedeutungen haben.

Bevorzugt sind Verbindungen der allgemeinen Formel (I), worin
- R³: bedeutet Wasserstoff;
- R⁵: bedeutet Cyano, Nitro, Halogen, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₆-Alkoxy, C₁-C₆-Halogenalkoxy oder R⁵ bildet zusammen mit dem Kohlenstoffatom, an dem es gebunden ist, eine Carbonylgruppe; insbesondere bedeutet R⁵ Methyl, Methoxy oder R⁵ bildet zusammen mit dem Kohlenstoffatom, an dem es gebunden ist, eine Carbonylgruppe, und die anderen Substituenten und Indices jeweils die weiter oben genannten Bedeutungen haben.

Besonders bevorzugt sind Verbindungen der allgemeinen Formel (I), worin
- R⁶, R⁷: bedeuten unabhängig voneinander Wasserstoff oder C₁-C₄-Alkyl, insbesonders Methyl oder Ethyl, oder Cyclopropyl;
- R⁸: bedeutet Wasserstoff, C₁-C₄-Alkyl, C₁-C₄-Halogenalkyl, C₁-C₄-Alkylcarbonyl, C₁-C₄-Halogenalkylcarbonyl, C₁-C₄-Alkoxycarbonyl, C₁-C₄-Alkylsulfonyl, C₁-C₄-Halogenalkylsulfonyl, Phenylcarbonyl, Phenylcarbonylmethyl, Phenyloxycarbonyl oder Phenylsulfonyl, wobei der Phenylkern der vier letztgenannten Reste durch s Reste aus der Gruppe Halogen, Nitro, Cyano, C₁-C₄-Alkyl, C₁-C₄-Halogenalkyl, C₁-C₄-Alkoxy und C₁-C₄-Halogenalkoxy substituiert ist,
und die anderen Substituenten und Indices jeweils die weiter oben genannten Bedeutungen haben.

Ganz besonders bevorzugt sind Verbindungen der allgemeinen Formel (I), worin
- R¹: bedeutet Chlor, Brom, Iod, Nitro, Methyl, Thiomethyl, Thioethyl, Methylsulfonyl, Ethylsulfonyl oder Methoxy;
- R²: bedeutet Brom, Chlor, Methylsulfonyl oder Ethylsulfonyl;
- R²: befindet sich in der 4- Position des Phenylreings;
- R⁸: bedeutet Wasserstoff;
- Het: bedeutet 3-Tetrahydrofuranyl, 3-Tetrahydropyranyl, 4-Tetrahydropyranyl, 1,3-Dioxan-5-yl oder γ-Butyrolacton-2-yl,
und die anderen Substituenten und Indices jeweils die weiter oben genannten Bedeutungen haben.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen Q für Q1 steht, können beispielsweise nach der in Schema 1 angegebenen Methode durch basenkatalysierte Umsetzung einer Verbindung der Formel (IIIa), in der T für Halogen, Hydroxy oder Alkoxy steht, mit einem Cyclohexandion (I) in Anwesenheit einer Cyanid-Quelle hergestellt werden. Solche Methoden sind beispielsweise in EP-A 0 369 803 und EP-B 0 283 261 beschrieben.

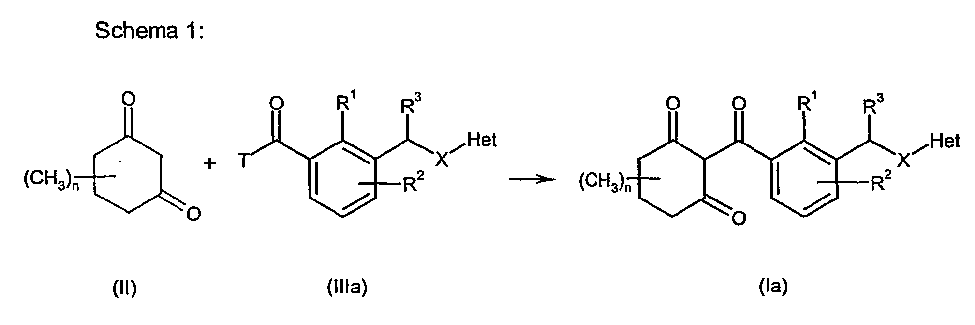

Erfindungsgemäße Verbindungen, in denen Q für Q2 und R8 für Wasserstoff steht, können beispielsweise nach der in Schema 2 angegebenen Methode hergestellt werden. Dazu wird eine Verbindung der Formel (IIIa) entweder in Gegenwart wasserentziehender Mittel, wie DCC, oder nach Überführung in ihr Säurechlorid, basenkatalysiert mit einem Pyrazol der Formel (IV) umgesetzt und schließlich mit einer Cyanid-Quelle behandelt. Diese Methoden sind beispielsweise in EP-A 0 369 803 beschrieben.

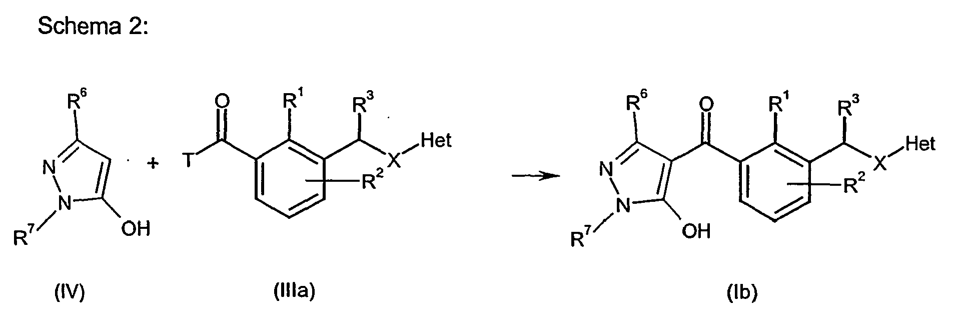

Erfindungsgemäße Verbindungen der Formel (I), in der R⁸ für andere Reste als Wasserstoff steht, können beispielsweise gemäß Schema 3 durch dem Fachmann an sich bekannte Substitutionsreaktionen hergestellt werden. Dazu werden Verbindungen der Formel (1b) mit Verbindungen der Formel (V), in der E für eine nucleophil austauschbare Abgangsgruppe steht, umgesetzt. Solche Methoden sind z. B. aus WO 99/10328 bekannt.

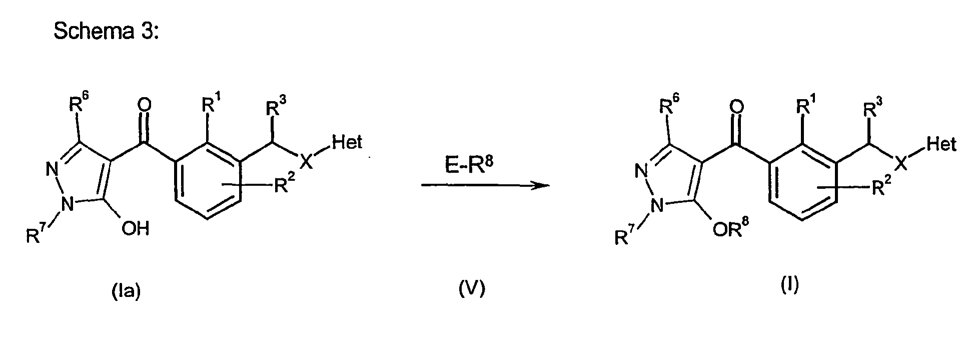

Verbindungen der Formel (IIIa), in der T für OH steht, können beispielsweise gemäß Schema 4 aus Verbindungen der Formel (1b), in der Hal für Halogen und R¹⁰ für Alkoxy oder OH steht, hergestellt werden.

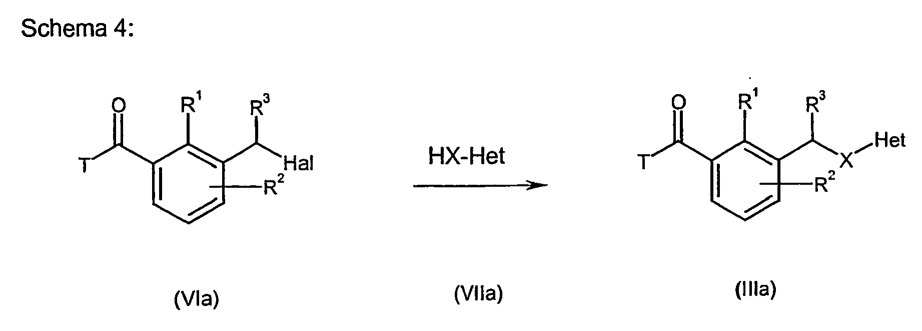

Verbindungen der allgemeinen Formel (IIIa) sind auch nach Reaktionen gemäß Schema 5 zugänglich.

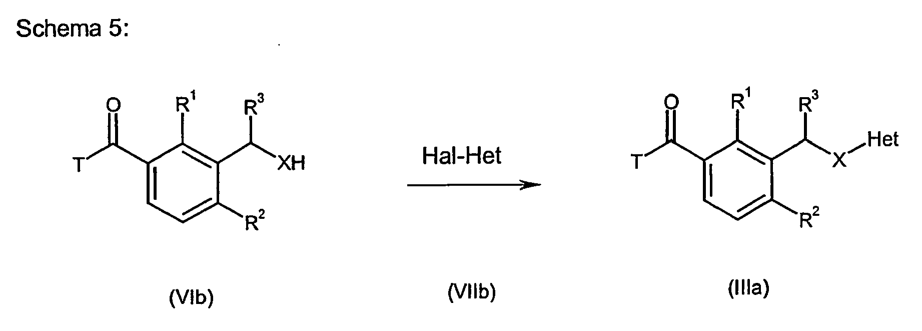

Verbindungen der Formel (VIa) und (VIb) sind literaturbekannt oder können nach bekannten Methoden, wie sie beispielsweise in WO 96/26200 und in der prioritätsälteren und nicht vorveröffentlichten deutschen Patentanmeldung Nr. 10144412.5 beschrieben sind, hergestellt werden.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Amaranthus retroflexus, Avena sp., Echinochloa sp., Cyperus serotinus, Lolium multiflorum, Setaria viridis, Sagittaria pygmaea, Scirpus juncoides, Sinapis sp. und Stellaria media.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Weizen, Mais und Reis auf. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen-bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vor-gegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. lnc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können z.B. verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäure-ester oder Polyoxethylensorbitanester wie z.B. Polyoxyethylen-sorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie-können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103..

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11 th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; mesotrione; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methjrl-5-benzyloxypyrazol; neburon; nicosulfuron; nipyraclophen; nitralin; nitrofen; nitrofluorfen; nortlurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; suclotrione ; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung von 2-(2-Chlor-3-(3-tetrahydrofuranyl)oxymethyl-4-methylsulfonylbenzoyl)-cyclohexan-1,3-dion (Tabellenbeispiel.Nr. 1.1)

### Schritt 1: 2-Chlor-3-(3-tetrahydrofuranyl)oxymethyl-4-methylsulfonylbenzoesäure

25 ml DMF und 3,25 g (28 mmol) Kalium-tert.-butylat wurden bei 0°C vorgelegt und mit 2,5 g (27,5 mmol) 3-Hydroxytetrahydrofuran vermischt. Die Lösung wurde auf - 15 °C gekühlt und mit 4,7 g (140 mmol) 3-Brommethyl-2-chlor-4-methylsulfonylbenzoesäure versetzt. Dann wurde 1 Stunde bei 15 bis 20 °C nachgerührt. Der Ansatz wurde auf 45 g Eis/Wasser gegeben, mit 2N HCl sauer gestellt und mit EE extrahiert. Die organischen Phasen wurden mit MgSO₄ getrocknet, filtriert und eingeengt. Man erhielt 5,41 g zähflüssiges Öl mit einer Reinheit von ca. 66 % nach HPLC. Ausbeute ca. 60 %

### Schritt 2: 3-Oxo-1-cyclohexenyl-2-chlor-3-(3-tetrahydrofuranyl)oxymethyl-4-methylsulfonyl-benzoat

5,41 g rohe 2-Chlor-3-(3-tetrahydrofuranyl)oxymethyl-4-methylsulfonyl-benzoesäure wurden in 30 ml CH₂Cl₂ gelöst und mit 2,5 ml (28 mmol) Oxalylchlorid langsam versetzt. Das Gemisch wurde ca. 30 min bis zur Beendigung der Gasentwicklung nachgerührt. Die Lösung wurde zu einem Gemisch aus 2 g (17,3 mmol) 1,3-Cyclohexandion und 5 g Net₃ in 20 ml CH₂Cl₂ unter Kühlung unterhalb von 15 °C zugetropft. Anschließend wurde 1 Stunde bei RT nachgerührt. Das Gemisch wurde filtriert, die Lösungsmittel einrotiert und der Rückstand anschließend mit 30 ml EE versetzt. Es wurde zuerst mit 5 % HCl, dann mit 2%-iger NaHCO₃-Lösung und 2 mal mit Wasser gewaschen. Die organische Phase wurde mit MgSO₄ getrocknet, filtriert und einrotiert. Man erhielt 5 g dickflüssiges Öl, welches chromatographisch gereinigt wurde (SiO₂/n-Heptan:EE, 1:3). Man erhielt 2,95 g farblosen Feststoff mit einer Reinheit von ca. 99 % nach HPLC.

### Schritt 3: 2-(2-Chlor-3-(3-tetrahydrofuranyl)oxymethyl-4-methylsulfonyl-benzoyl)-cyclohexan-1,3-dion

8 g (18,5 mmol) 3-Oxo-1-cyclohexenyl-2-chlor-3-(3-tetrahydrofuranyl)oxymethyl-4-methylsulfonyl-benzoat wurden in 50 ml CH₃CN suspendiert und unter Rühren mit 2,25 g (21,8 mmol) NEt₃ und 0,13 g (1,5 mmol) Acetoncyanhydrin versetzt. Man ließ 3 Stunden bei RT rühren und rotierte anschließend ein. Zum öligen Rückstand wurde Wasser gegeben und mit gesättigter NaHCO₃-Lösung ein pH-Wert von > 8 eingestellt. Die basische Lösung wurde mit 20 ml EE gewaschen. Die wässrige Lösung wurde dann mit 2N HCl sauer gestellt und mit 2 x 50 ml EE extrahiert. Die Lösung wurde mit NaHCO₃-Lösung gewaschen. Die organische Lösung wurde mit MgSO₄ getrocknet, filtriert und einrotiert. Aus der konzentrierten Lösung kristallisierte das Produkt langsam aus. Der Feststoff wurde abgesaugt und mit kaltem EE nachgewaschen. Man erhielt 6,81 g (15,9 mmol) Produkt mit einer Reinheit von 99,8 % nach HPLC und einem Festpunkt von 126 °C. Die Ausbeute betrug 85 %.

Die hier verwendeten Abkürzungen bedeuten:

| | | |
|---|---|---|
| cPr = cyclo-Propyl | nPr = n-Propyl | nBu = n-Butyl |
| Et = Ethyl | Me = Methyl | Ph = Phenyl |
| EE = Ethylacetat | Fp. = Festpunkt | RT = Raumtemperatur |

**Tabelle 1: Erfindungsgemäße Verbindungen der allgemeinen Formel (I), worin die Substituenten und Symbole folgende Bedeutungen haben:**

| | | | | | |
|---|---|---|---|---|---|
| | R³ = H | | Q = Q1 | | p = 0 |
| 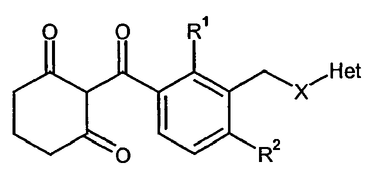 | | | | | |

| **Nr.** | **R¹** | **R²** | **X** | **Het** | **Physikal. Daten** |
|---|---|---|---|---|---|
| 1.1 | Cl | SO₂Me | O | 3-Tetrahydrofuranyl | Fp.:126°C |
| 1.2 | Cl | SO₂Et | O | 3-Tetrahydrofuranyl | Öl |
| 1.3 | Cl | Cl | O | 3-Tetrahydrofuranyl | |
| 1.4 | Br | SO₂Me | O | 3-Tetrahydrofuranyl | |
| 1.5 | Br | SO₂Et | O | 3-Tetrahydrofuranyl | |
| 1.6 | Br | Cl | O | 3-Tetrahydrofuranyl | |
| 1.7 | I | SO₂Me | O | 3-Tetrahydrofuranyl | |
| 1.8 | I | SO₂Et | O | 3-Tetrahydrofuranyl | |
| 1.9 | I | Cl | O | 3-Tetrahydrofuranyl | |
| 1.10 | Me | SO₂Me | O | 3-Tetrahydrofuranyl | |
| 1.11 | Me | SO₂Et | O | 3-Tetrahydrofuranyl | |
| 1.12 | Me | Cl | O | 3-Tetrahydrofuranyl | |
| 1.13 | SMe | SO₂Me | O | 3-Tetrahydrofuranyl | |
| 1.14 | SMe | SO₂Et | O | 3-Tetrahydrofuranyl | |
| 1.15 | SMe | Cl | O | 3-Tetrahydrofuranyl | |
| 1.16 | SO₂Me | SO₂Me | O | 3-Tetrahydrofuranyl | |
| 1.17 | SO₂Me | SO₂Et | O | 3-Tetrahydrofuranyl | |
| 1.18 | SO₂Me | Cl | O | 3-Tetrahydrofuranyl | |
| 1.19 | NO₂ | SO₂Me | O | 3-Tetrahydrofuranyl | |
| 1.20 | NO₂ | SO₂Et | O | 3-Tetrahydrofuranyl | |
| 1.21 | NO₂ | Cl | O | 3-Tetrahydrofuranyl | |
| 1.22 | OMe | Cl | O | 3-Tetrahydrofuranyl | |
| 1.23 | SO₂Et | Cl | O | 3-Tetrahydrofuranyl | |
| 1.24 | SEt | Cl | O | 3-Tetrahydrofuranyl | |
| 1.25 | Cl | SO₂Me | O | 4-Tetrahydropyranyl | |
| 1.26 | Cl | SO₂Et | O | 4-Tetrahydropyranyl | |
| 1.27 | Cl | Cl | O | 4-Tetrahydropyranyl | |
| 1.28 | Br | SO₂Me | O | 4-Tetrahydropyranyl | |
| 1.29 | Br | SO₂Et | O | 4-Tetrahydropyranyl | |
| 1.30 | Br | Cl | O | 4-Tetrahydropyranyl | |
| 1.31 | I | SO₂Me | O | 4-Tetrahydropyranyl | |
| 1.32 | I | SO₂Et | O | 4-Tetrahydropyranyl | |
| 1.33 | I | Cl | O | 4-Tetrahydropyranyl | |
| 1.34 | Me | SO₂Me | O | 4-Tetrahydropyranyl | |
| 1.35 | Me | SO₂Et | O | 4-Tetrahydropyranyl | |
| 1.36 | Me | Cl | O | 4-Tetrahydropyranyl | |
| 1.37 | SMe | SO₂Me | O | 4-Tetrahydropyranyl | |
| 1.38 | SMe | SO₂Et | O | 4-Tetrahydropyranyl | |
| 1.39 | SMe | Cl | O | 4-Tetrahydropyranyl | |
| 1.40 | SO₂Me | SO₂Me | O | 4-Tetrahydropyranyl | |
| 1.41 | SO₂Me | SO₂Et | O | 4-Tetrahydropyranyl | |
| 1.42 | SO₂Me | Cl | O | 4-Tetrahydropyranyl | |
| 1.43 | NO₂ | SO₂Me | O | 4-Tetrahydropyranyl | |
| 1.44 | NO₂ | SO₂Et | O | 4-Tetrahydropyranyl | |
| 1.45 | NO₂ | Cl | O | 4-Tetrahydropyranyl | |
| 1.46 | OMe | Cl | O | 4-Tetrahydropyranyl | |
| 1.47 | SO₂Et | Cl | O | 4-Tetrahydropyranyl | |
| 1.48 | SEt | Cl | O | 4-Tetrahydropyranyl | |
| 1.49 | Cl | SO₂Me | O | 3-Tetrahydropyranyl | |
| 1.50 | Cl | SO₂Et | O | 3-Tetrahydropyranyl | |
| 1.51 | Cl | Cl | O | 3-Tetrahydropyranyl | |
| 1.52 | Br | SO₂Me | O | 3-Tetrahydropyranyl | |
| 1.53 | Br | SO₂Et | O | 3-Tetrahydropyranyl | |
| 1.54 | Br | Cl | O | 3-Tetrahydropyranyl | |
| 1.55 | I | SO₂Me | O | 3-Tetrahydropyranyl | |
| 1.56 | I | SO₂Et | O | 3-Tetrahydropyranyl | |
| 1.57 | I | Cl | O | 3-Tetrahydropyranyl | |
| 1.58 | Me | SO₂Me | O | 3-Tetrahydropyranyl | |
| 1.59 | Me | SO₂Et | O | 3-Tetrahydropyranyl | |
| 1.60 | Me | Cl | O | 3-Tetrahydropyranyl | |
| 1.61 | SMe | SO₂Me | O | 3-Tetrahydropyranyl | |
| 1.62 | SMe | SO₂Et | O | 3-Tetrahydropyranyl | |
| 1.63 | SMe | Cl | O | 3-Tetrahydropyranyl | |
| 1.64 | SO₂Me | SO₂Me | O | 3-Tetrahydropyranyl | |
| 1.65 | SO₂Me | SO₂Et | O | 3-Tetrahydropyranyl | |
| 1.66 | SO₂Me | Cl | O | 3-Tetrahydropyranyl | |
| 1.67 | NO₂ | SO₂Me | O | 3-Tetrahydropyranyl | |
| 1.68 | NO₂ | SO₂Et | O | 3-Tetrahydropyranyl | |
| 1.69 | NO₂ | Cl | O | 3-Tetrahydropyranyl | |
| 1.70 | OMe | Cl | O | 3-Tetrahydropyranyl | |
| 1.71 | SO₂Et | Cl | O | 3-Tetrahydropyranyl | |
| 1.72 | SEt | Cl | O | 3-Tetrahydropyranyl | |
| 1.73 | Cl | SO₂Me | O | 1,3-Dioxan-5 yl | |
| 1.74 | Cl | SO₂Et | O | 1,3-Dioxan-5 yl | |
| 1.75 | Cl | Cl | O | 1,3-Dioxan-5 yl | |
| 1.76 | Sr | SO₂Me | O | 1,3-Dioxan-5 yl | |
| 1.77 | Br | SO₂Et | O | 1,3-Dioxan-5 yl | |
| 1.78 | Br | Cl | O | 1,3-Dioxan-5 yl | |
| 1.79 | I | SO₂Me | O | 1,3-Dioxan-5 yl | |
| 1.80 | I | SO₂Et | O | 1,3-Dioxan-5 yl | |
| 1.81 | I | Cl | O | 1,3-Dioxan-5 yl | |
| 1.82 | Me | SO₂Me | O | 1,3-Dioxan-5 yl | |
| 1.83 | Me | SO₂Et | O | 1,3-Dioxan-5 yl | |
| 1.84 | Me | Cl | O | 1,3-Dioxan-5 yl | |
| 1.85 | SMe | SO₂Me | O | 1,3-Dioxan-5 yl | |
| 1.86 | SMe | SO₂Et | O | 1,3-Dioxan-5 yl | |
| 1.87 | SMe | Cl | O | 1,3-Dioxan-5 yl | |
| 1.88 | SO₂Me | SO₂Me | O | 1,3-Dioxan-5 yl | |
| 1.89 | SO₂Me | SO₂Et | O | 1,3-Dioxan-5 yl | |
| 1.90 | SO₂Me | Cl | O | 1,3-Dioxan-5 yl | |
| 1.91 | NO₂ | SO₂Me | O | 1,3-Dioxan-5 yl | |
| 1.92 | NO₂ | SO₂Et | O | 1,3-Dioxan-5 yl | |
| 1.93 | NO₂ | Cl | O | 1,3-Dioxan-5 yl | |
| 1.94 | OMe | Cl | O | 1,3-Dioxan-5 yl | |
| 1.95 | SO₂Et | Cl | O | 1,3-Dioxan-5 yl | |
| 1.96 | SEt | Cl | O | 1,3-Dioxan-5 yl | |
| 1.97 | Cl | SO₂Me | O | γ-butyrolacton-2-yl | Öl |
| 1.98 | Cl | SO₂Et | O | γ-butyrolacton-2-yl | |
| 1.99 | Cl | Cl | O | γ-butyrolacton-2-yl | |
| 1.100 | Br | SO₂Me | O | γ-butyrolacton-2-yl | |
| 1.101 | Br | SO₂Et | O | γ-butyrolacton-2-yl | |
| 1.102 | Br | Cl | O | γ-butyrolacton-2-yl | |
| 1.103 | I | SO₂Me | O | γ-butyrolacton-2-yl | |
| 1.104 | I | SO₂Et | O | γ-butyrolacton-2-yl | |
| 1.105 | I | Cl | O | γ-butyrolacton-2-yl | |
| 1.106 | Me | SO₂Me | O | γ-butyrolacton-2-yl | |
| 1.107 | Me | SO₂Et | O | γ-butyrolacton-2-yl | |
| 1.108 | Me | Cl | O | γ-butyrolacton-2-yl | |
| 1.109 | SMe | SO₂Me | O | γ-butyrolacton-2-yl | |
| 1.110 | SMe | SO₂Et | O | γ-butyrolacton-2-yl | |
| 1.111 | SMe | Cl | O | γ-butyrolacton-2-yl | |
| 1.112 | SO₂Me | SO₂Me | O | γ-butyrolacton-2-yl | |
| 1.113 | SO₂Me | SO₂Et | O | γ-butyrolacton-2-yl | |
| 1.114 | SO₂Me | Cl | O | γ-butyrolacton-2-yl | |
| 1.115 | NO₂ | SO₂Me | O | γ-butyrolacton-2-yl | |
| 1.116 | NO₂ | SO₂Et | O | γ-butyrolacton-2-yl | |
| 1.117 | NO₂ | Cl | O | γ-butyrolacton-2-yl | |
| 1.118 | OMe | Cl | O | γ-butyrolacton-2-yl | |
| 1.119 | SO₂Et | Cl | O | γ-butyrolacton-2-yl | |
| 1.120 | SEt | Cl | O | γ-butyrolacton-2-yl | |
| 1.121 | Cl | SO₂Me | S | 3-Tetrahydrofuranyl | |
| 1.122 | Cl | SO₂Et | S | 3-Tetrahydrofuranyl | |
| 1.123 | Cl | Cl | S | 3-Tetrahydrofuranyl | |
| 1.124 | Br | SO₂Me | S | 3-Tetrahydrofuranyl | |
| 1.125 | Br | SO₂Et | S | 3-Tetrahydrofuranyl | |
| 1.126 | Br | Cl | S | 3-Tetrahydrofuranyl | |
| 1.127 | I | SO₂Me | S | 3-Tetrahydrofuranyl | |
| 1.128 | I | SO₂Et | S | 3-Tetrahydrofuranyl | |
| 1.129 | I | Cl | S | 3-Tetrahydrofuranyl | |
| 1.130 | Me | SO₂Me | S | 3-Tetrahydrofuranyl | |
| 1.131 | Me | SO₂Et | S | 3-Tetrahydrofuranyl | |
| 1.132 | Me | Cl | S | 3-Tetrahydrofuranyl | |
| 1.133 | SMe | SO₂Me | S | 3-Tetrahydrofuranyl | |
| 1.134 | SMe | SO₂Et | S | 3-Tetrahydrofuranyl | |
| 1.135 | SMe | Cl | S | 3-Tetrahydrofuranyl | |
| 1.136 | SO₂Me | SO₂Me | S | 3-Tetrahydrofuranyl | |
| 1.137 | SO₂Me | SO₂Et | S | 3-Tetrahydrofuranyl | |
| 1.138 | SO₂Me | Cl | S | 3-Tetrahydrofuranyl | |
| 1.139 | NO₂ | SO₂Me | S | 3-Tetrahydrofuranyl | |
| 1.140 | NO₂ | SO₂Et | S | 3-Tetrahydrofuranyl | |
| 1.141 | NO₂ | Cl | S | 3-Tetrahydrofuranyl | |
| 1.142 | OMe | Cl | S | 3-Tetrahydrofuranyl | |
| 1.143 | SO₂Et | Cl | S | 3-Tetrahydrofuranyl | |
| 1.144 | SEt | Cl | S | 3-Tetrahydrofuranyl | |
| 1.145 | Cl | SO₂Me | S | 4-Tetrahydropyranyl | |
| 1.146 | Cl | SO₂Et | S | 4-Tetrahydropyranyl | |
| 1.147 | Cl | Cl | S | 4-Tetrahydropyranyl | |
| 1.148 | Br | SO₂Me | S | 4-Tetrahydropyranyl | |
| 1.149 | Br | SO₂Et | S | 4-Tetrahydropyranyl | |
| 1.150 | Br | Cl | S | 4-Tetrahydropyranyl | |
| 1.151 | I | SO₂Me | S | 4-Tetrahydropyranyl | |
| 1.152 | I | SO₂Et | S | 4-Tetrahydropyranyl | |
| 1.153 | I | Cl | S | 4-Tetrahydropyranyl | |
| 1.154 | Me | SO₂Me | S | 4-Tetrahydropyranyl | |
| 1.155 | Me | SO₂Et | S | 4-Tetrahydropyranyl | |
| 1.156 | Me | Cl | S | 4-Tetrahydropyranyl | |
| 1.157 | SMe | SO₂Me | S | 4-Tetrahydropyranyl | |
| 1.158 | SMe | SO₂Et | S | 4-Tetrahydropyranyl | |
| 1.159 | SMe | Cl | S | 4-Tetrahydropyranyl | |
| 1.160 | SO₂Me | SO₂Me | S | 4-Tetrahydropyranyl | |
| 1.161 | SO₂Me | SO₂Et | S | 4-Tetrahydropyranyl | |
| 1.162 | SO₂Me | Cl | S | 4-Tetrahydropyranyl | |
| 1.163 | NO₂ | SO₂Me | S | 4-Tetrahydropyranyl | |
| 1.164 | NO₂ | SO₂Et | S | 4-Tetrahydropyranyl | |
| 1.165 | NO₂ | Cl | S | 4-Tetrahydropyranyl | |
| 1.166 | OMe | Cl | S | 4-Tetrahydropyranyl | |
| 1.167 | SO₂Et | Cl | S | 4-Tetrahydropyranyl | |
| 1.168 | SEt | Cl | S | 4-Tetrahydropyranyl | |
| 1.169 | Cl | SO₂Me | S | 3-Tetrahydropyranyl | |
| 1.170 | Cl | SO₂Et | S | 3-Tetrahydropyranyl | |
| 1.171 | Cl | Cl | S | 3-Tetrahydropyranyl | |
| 1.172 | Br | SO₂Me | S | 3-Tetrahydropyranyl | |
| 1.173 | Br | SO₂Et | S | 3-Tetrahydropyranyl | |
| 1.174 | Br | Cl | S | 3-Tetrahydropyranyl | |
| 1.175 | I | SO₂Me | S | 3-Tetrahydropyranyl | |
| 1.176 | I | SO₂Et | S | 3-Tetrahydropyranyl | |
| 1.177 | I | Cl | S | 3-Tetrahydropyranyl | |
| 1.178 | Me | SO₂Me | S | 3-Tetrahydropyranyl | |
| 1.179 | Me | SO₂Et | S | 3-Tetrahydropyranyl | |
| 1.180 | Me | Cl | S | 3-Tetrahydropyranyl | |
| 1.181 | SMe | SO₂Me | S | 3-Tetrahydropyranyl | |
| 1.182 | SMe | SO₂Et | S | 3-Tetrahydropyranyl | |
| 1.183 | SMe | Cl | S | 3-Tetrahydropyranyl | |
| 1.184 | SO₂Me | SO₂Me | S | 3-Tetrahydropyranyl | |
| 1.185 | SO₂Me | SO₂Et | S | 3-Tetrahydropyranyl | |
| 1.186 | SO₂Me | Cl | S | 3-Tetrahydropyranyl | |
| 1.187 | NO₂ | SO₂Me | S | 3-Tetrahydropyranyl | |
| 1.188 | NO₂ | SO₂Et | S | 3-Tetrahydropyranyl | |
| 1.189 | NO₂ | Cl | S | 3-Tetrahydropyranyl | |
| 1.190 | OMe | Cl | S | 3-Tetrahydropyranyl | |
| 1.191 | SO₂Et | Cl | S | 3-Tetrahydropyranyl | |
| 1.192 | SEt | Cl | S | 3-Tetrahydropyranyl | |
| 1.193 | Cl | SO₂Me | S | 1,3-Dioxan-5 yl | |
| 1.194 | Cl | SO₂Et | S | 1,3-Dioxan-5 yl | |
| 1.195 | Cl | Cl | S | 1,3-Dioxan-5 yl | |
| 1.196 | Br | SO₂Me | S | 1,3-Dioxan-5 yl | |
| 1.197 | Br | SO₂Et | S | 1,3-Dioxan-5 yl | |
| 1.198 | Br | Cl | S | 1,3-Dioxan-5 yl | |
| 1.199 | I | SO₂Me | S | 1,3-Dioxan-5 yl | |
| 1.200 | I | SO₂Et | S | 1,3-Dioxan-5 yl | |
| 1.201 | I | Cl | S | 1,3-Dioxan-5 yl | |
| 1.202 | Me | SO₂Me | S | 1,3-Dioxan-5 yl | |
| 1.203 | Me | SO₂Et | S | 1,3-Dioxan-5 yl | |
| 1.204 | Me | Cl | S | 1,3-Dioxan-5 yl | |
| 1.205 | SMe | SO₂Me | S | 1,3-Dioxan-5 yl | |
| 1.206 | SMe | SO₂Et | S | 1,3-Dioxan-5 yl | |
| 1.207 | SMe | Cl | S | 1,3-Dioxan-5 yl | |
| 1.208 | SO₂Me | SO₂Me | S | 1,3-Dioxan-5 yl | |
| 1.209 | SO₂Me | SO₂Et | S | 1,3-Dioxan-5 yl | |
| 1.210 | SO₂Me | Cl | S | 1,3-Dioxan-5 yl | |
| 1.211 | NO₂ | SO₂Me | S | 1,3-Dioxan-5 yl | |
| 1.212 | NO₂ | SO₂Et | S | 1,3-Dioxan-5 yl | |
| 1.213 | NO₂ | Cl | S | 1,3-Dioxan-5 yl | |
| 1.214 | OMe | Cl | S | 1,3-Dioxan-5 yl | |
| 1.215 | SO₂Et | Cl | S | 1,3-Dioxan-5 yl | |
| 1.216 | SEt | Cl | S | 1,3-Dioxan-5 yl | |
| 1.217 | Cl | SO₂Me | S | γ-butyrolacton-2-yl | |
| 1.218 | Cl | SO₂Et | S | γ-butyrolacton-2-yl | |
| 1.219 | Cl | Cl | S | γ-butyrolacton-2-yl | |
| 1.220 | Br | SO₂Me | S | γ-butyrolacton-2-yl | |
| 1.221 | Br | SO₂Et | S | γ-butyrolacton-2-yl | |
| 1.222 | Br | Cl | S | γ-butyrolacton-2-yl | |
| 1.223 | I | SO₂Me | S | γ-butyrolacton-2-yl | |
| 1.224 | I | SO₂Et | S | γ-butyrolacton-2-yl | |
| 1.225 | I | Cl | S | γ-butyrolacton-2-yl | |
| 1.226 | Me | SO₂Me | S | γ-butyrolacton-2-yl | |
| 1.227 | Me | SO₂Et | S | γ-butyrolacton-2-yl | |
| 1.228 | Me | Cl | S | γ-butyrolacton-2-yl | |
| 1.229 | SMe | SO₂Me | S | γ-butyrolacton-2-yl | |
| 1.220 | SMe | SO₂Et | S | γ-butyrolacton-2-yl | |
| 1.231 | SMe | Cl | S | γ-butyrolacton-2-yl | |
| 1.232 | SO₂Me | SO₂Me | S | γ-butyrolacton-2-yl | |
| 1.233 | SO₂Me | SO₂Et | S | γ-butyrolacton-2-yl | |
| 1.234 | SO₂Me | Cl | S | γ-butyrolacton-2-yl | |
| 1.235 | NO₂ | SO₂Me | S | γ-butyrolacton-2-yl | |
| 1.236 | NO₂ | SO₂Et | S | γ-butyrolacton-2-yl | |
| 1.237 | NO₂ | Cl | S | γ-butyrolacton-2-yl | |
| 1.238 | OMe | Cl | S | γ-butyrolacton-2-yl | |
| 1.239 | SO₂Et | Cl | S | γ-butyrolacton-2-yl | |
| 1.240 | SEt | Cl | S | γ-butyrolacton-2-yl | |

**Tabelle 2: Erfindungsgemäße Verbindungen der allgemeinen Formel (I), worin die Substituenten und Symbole folgende Bedeutungen haben:**

| | | | | | |
|---|---|---|---|---|---|
| R³ = H | | | Q = Q1 | | p = 2 |
| 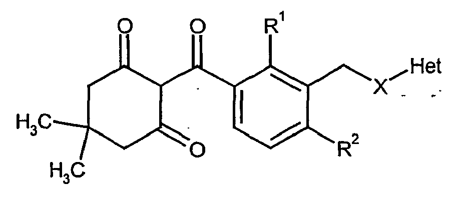 | | | | | |

| **Nr.** | **R¹** | **R²** | **X** | **Het** | **Physikal. Daten** |
|---|---|---|---|---|---|
| 2.1 | Cl | SO₂Me | O | 3-Tetrahydrofuranyl | Öl |
| 2.2 | Cl | SO₂Et | O | 3-Tetrahydrofuranyl | Öl |
| 2.3 | Cl | Cl | O | 3-Tetrahydrofuranyl | |
| 2.4 | Br | SO₂Me | O | 3-Tetrahydrofuranyl | |
| 2.5 | Br | SO₂Et | O | 3-Tetrahydrofuranyl | |
| 2.6 | Br | Cl | O | 3-Tetrahydrofuranyl | |
| 2.7 | I | SO₂Me | O | 3-Tetrahydrofuranyl | |
| 2.8 | I | SO₂Et | O | 3-Tetrahydrofuranyl | |
| 2.9 | I | Cl | O | 3-Tetrahydrofuranyl | |
| 2.10 | Me | SO₂Me | O | 3-Tetrahydrofuranyl | |
| 2.11 | Me | SO₂Et | O | 3-Tetrahydrofuranyl | |
| 2.12 | Me | Cl | O | 3-Tetrahydrofuranyl | |
| 2.13 | SMe | SO₂Me | O | 3-Tetrahydrofuranyl | |
| 2.14 | SMe | SO₂Et | O | 3-Tetrahydrofuranyl | |
| 2.15 | SMe | Cl | O | 3-Tetrahydrofuranyl | |
| 2.16 | SO₂Me | SO₂Me | O | 3-Tetrahydrofuranyl | |
| 2.17 | SO₂Me | SO₂Et | O | 3-Tetrahydrofuranyl | |
| 2.18 | SO₂Me | Cl | O | 3-Tetrahydrofuranyl | |
| 2.19 | NO₂ | SO₂Me | O | 3-Tetrahydrofuranyl | |
| 2.20 | NO₂ | SO₂Et | O | 3-Tetrahydrofuranyl | |
| 2.21 | NO₂ | Cl | O | 3-Tetrahydrofuranyl | |
| 2.22 | Cl | SO₂Me | O | 4-Tetrahydropyranyl | |
| 2.23 | Cl | SO₂Et | O | 4-Tetrahydropyranyl | |
| 2.24 | Cl | Cl | O | 4-Tetrahydropyranyl | |
| 2.25 | Br | SO₂Me | O | 4-Tetrahydropyranyl | |
| 2.26 | Br | SO₂Et | O | 4-Tetrahydropyranyl | |
| 2.27 | Br | Cl | O | 4-Tetrahydropyranyl | |
| 2.28 | I | SO₂Me | O | 4-Tetrahydropyranyl | |
| 2.29 | I | SO₂Et | O | 4-Tetrahydropyranyl | |
| 2.30 | I | Cl | O | 4-Tetrahydropyranyl | |
| 2.31 | Me | SO₂Me | O | 4-Tetrahydropyranyl | |
| 2.32 | Me | SO₂Et | O | 4-Tetrahydropyranyl | |
| 2.33 | Me | Cl | O | 4-Tetrahydropyranyl | |
| 2.34 | SMe | SO₂Me | O | 4-Tetrahydropyranyl | |
| 2.35 | SMe | SO₂Et | O | 4-Tetrahydropyranyl | |
| 2.36 | SMe | Cl | O | 4-Tetrahydropyranyl | |
| 2.37 | SO₂Me | SO₂Me | O | 4-Tetrahydropyranyl | |
| 2.38 | SO₂Me | SO₂Et | O | 4-Tetrahydropyranyl | |
| 2.39 | SO₂Me | Cl | O | 4-Tetrahydropyranyl | |
| 2.40 | NO₂ | SO₂Me | O | 4-Tetrahydropyranyl | |
| 2.41 | NO₂ | SO₂Et | O | 4-Tetrahydropyranyl | |
| 2.42 | NO₂ | Cl | O | 4-Tetrahydropyranyl | |
| 2.43 | Cl | SO₂Me | O | 3-Tetrahydropyranyl | |
| 2.44 | Cl | SO₂Et | O | 3-Tetrahydropyranyl | |
| 2.45 | Cl | Cl | O | 3-Tetrahydropyranyl | |
| 2.46 | Br | SO₂Me | O | 3-Tetrahydropyranyl | |
| 2.47 | Br | SO₂Et | O | 3-Tetrahydropyranyl | |
| 2.48 | Br | Cl | O | 3-Tetrahydropyranyl | |
| 2.49 | I | SO₂Me | O | 3-Tetrahydropyranyl | |
| 2.50 | I | SO₂Et | O | 3-Tetrahydropyranyl | |
| 2.51 | I | Cl | O | 3-Tetrahydropyranyl | |
| 2.52 | Me | SO₂Me | O | 3-Tetrahydropyranyl | |
| 2.53 | Me | SO₂Et | O | 3-Tetrahydropyranyl | |
| 2.54 | Me | Cl | O | 3-Tetrahydropyranyl | |
| 2.55 | SMe | SO₂Me | O | 3-Tetrahydropyranyl | |
| 2.56 | SMe | SO₂Et | O | 3-Tetrahydropyranyl | |
| 2.57 | SMe | Cl | O | 3-Tetrahydropyranyl | |
| 2.58 | SO₂Me | SO₂Me | O | 3-Tetrahydropyranyl | |
| 2.59 | SO₂Me | SO₂Et | O | 3-Tetrahydropyranyl | |
| 2.60 | SO₂Me | Cl | O | 3-Tetrahydropyranyl | |
| 2.61 | NO₂ | SO₂Me | O | 3-Tetrahydropyranyl | |
| 2.62 | NO₂ | SO₂Et | O | 3-Tetrahydropyranyl | |
| 2.63 | NO₂ | Cl | O | 3-Tetrahydropyranyl | |
| 2.64 | Cl | SO₂Me | O | 1,3-Dioxan-5 yl | |
| 2.65 | Cl | SO₂Et | O | 1,3-Dioxan-5 yl | |
| 2.66 | Cl | Cl | O | 1,3-Dioxan-5 yl | |
| 2.67 | Br | SO₂Me | O | 1,3-Dioxan-5 yl | |
| 2.68 | Br | SO₂Et | O | 1,3-Dioxan-5 yl | |
| 2.69 | Br | Cl | O | 1,3-Dioxan-5 yl | |
| 2.70 | I | SO₂Me | O | 1,3-Dioxan-5 yl | |
| 2.71 | I | SO₂Et | O | 1,3-Dioxan-5 yl | |
| 2.72 | I | Cl | O | 1,3-Dioxan-5 yl | |
| 2.73 | Me | SO₂Me | O | 1,3-Dioxan-5 yl | |
| 2.74 | Me | SO₂Et | O | 1,3-Dioxan-5 yl | |
| 2.75 | Me | Cl | O | 1,3-Dioxan-5 yl | |
| 2.76 | SMe | SO₂Me | O | 1,3-Dioxan-5 yl | |
| 2.77 | SMe | SO₂Et | O | 1,3-Dioxan-5 yl | |
| 2.78 | SMe | Cl | O | 1,3-Dioxan-5 yl | |
| 2.79 | SO₂Me | SO₂Me | O | 1,3-Dioxan-5 yl | |
| 2.80 | SO₂Me | SO₂Et | O | 1,3-Dioxan-5 yl | |
| 2.81 | SO₂Me | Cl | O | 1,3-Dioxan-5 yl | |
| 2.82 | NO₂ | SO₂Me | O | 1,3-Dioxan-5 yl | |
| 2.83 | NO₂ | SO₂Et | O | 1,3-Dioxan-5 yl | |
| 2.84 | NO₂ | Cl | O | 1,3-Dioxan-5 yl | |
| 2.85 | Cl | SO₂Me | O | γ-butyrolacton-2-yl | |
| 2.86 | Cl | SO₂Et | O | γ-butyrolacton-2-yl | |
| 2.87 | Cl | Cl | O | γ-butyrolacton-2-yl | |
| 2.88 | Br | SO₂Me | O | γ-butyrolacton-2-yl | |
| 2.89 | Br | SO₂Et | O | γ-butyrolacton-2-yl | |
| 2.90 | Br | Cl | O | γ-butyrolacton-2-yl | |
| 2.91 | I | SO₂Me | O | γ-butyrolacton-2-yl | |
| 2.92 | I | SO₂Et | O | γ-butyrolacton-2-yl | |
| 2.93 | I | Cl | O | γ-butyrolacton-2-yl | |
| 2.94 | Me | SO₂Me | O | γ-butyrolacton-2-yl | |
| 2.95 | Me | SO₂Et | O | γ-butyrolacton-2-yl | |
| 2.96 | Me | Cl | O | γ-butyrolacton-2-yl | |
| 2.97 | SMe | SO₂Me | O | γ-butyrolacton-2-yl | |
| 2.98 | SMe | SO₂Et | O | γ-butyrolacton-2-yl | |
| 2.99 | SMe | Cl | O | γ-butyrolacton-2-yl | |
| 2.100 | SO₂Me | SO₂Me | O | γ-butyrolacton-2-yl | |
| 2.101 | SO₂Me | SO₂Et | O | γ-butyrolacton-2-yl | |
| 2.102 | SO₂Me | Cl | O | γ-butyrolacton-2-yl | |
| 2.103 | NO₂ | SO₂Me | O | γ-butyrolacton-2-yl | |
| 2.104 | NO₂ | SO₂Et | O | γ-butyrolacton-2-yl | |
| 2.105 | NO₂ | Cl | O | γ-butyrolacton-2-yl | |
| 2.106 | Cl | SO₂Me | S | 3-Tetrahydrofuranyl | |
| 2.107 | Cl | SO₂Et | S | 3-Tetrahydrofuranyl | |
| 2.108 | Cl | Cl | S | 3-Tetrahydrofuranyl | |
| 2.109 | Br | SO₂Me | S | 3-Tetrahydrofuranyl | |
| 2.110 | Br | SO₂Et | S | 3-Tetrahydrofuranyl | |
| 2.111 | Br | Cl | S | 3-Tetrahydrofuranyl | |
| 2.112 | I | SO₂Me | S | 3-Tetrahydrofuranyl | |
| 2.113 | I | SO₂Et | S | 3-Tetrahydrofuranyl | |
| 2.114 | I | Cl | S | 3-Tetrahydrofuranyl | |
| 2.115 | Me | SO₂Me | S | 3-Tetrahydrofuranyl | |
| 2.116 | Me | SO₂Et | S | 3-Tetrahydrofuranyl | |
| 2.117 | Me | Cl | S | 3-Tetrahydrofuranyl | |
| 2.118 | SMe | SO₂Me | S | 3-Tetrahydrofuranyl | |
| 2.119 | SMe | SO₂Et | S | 3-Tetrahydrofuranyl | |
| 2.120 | SMe | Cl | S | 3-Tetrahydrofuranyl | |
| 2.121 | SO₂Me | SO₂Me | S | 3-Tetrahydrofuranyl | |
| 2.122 | SO₂Me | SO₂Et | S | 3-Tetrahydrofuranyl | |
| 2.123 | SO₂Me | Cl | S | 3-Tetrahydrofuranyl | |
| 2.124 | NO₂ | SO₂Me | S | 3-Tetrahydrofuranyl | |
| 2.125 | NO₂ | SO₂Et | S | 3-Tetrahydrofuranyl | |
| 2.126 | NO₂ | Cl | S | 3-Tetrahydrofuranyl | |
| 2.127 | Cl | SO₂Me | S | 4-Tetrahydropyranyl | |
| 2.128 | Cl | SO₂Et | S | 4-Tetrahydropyranyl | |
| 2.129 | Cl | Cl | S | 4-Tetrahydropyranyl | |
| 2.130 | Br | SO₂Me | S | 4-Tetrahydropyranyl | |
| 2.131 | Br | SO₂Et | S | 4-Tetrahydropyranyl | |
| 2.132 | Br | Cl | S | 4-Tetrahydropyranyl | |
| 2.133 | I | SO₂Me | S | 4-Tetrahydropyranyl | |
| 2.134 | I | SO₂Et | S | 4-Tetrahydropyranyl | |
| 2.135 | I | Cl | S | 4-Tetrahydropyranyl | |
| 2.136 | Me | SO₂Me | S | 4-Tetrahydropyranyl | |
| 2.137 | Me | SO₂Et | S | 4-Tetrahydropyranyl | |
| 2.138 | Me | Cl | S | 4-Tetrahydropyranyl | |
| 2.139 | SMe | SO₂Me | S | 4-Tetrahydropyranyl | |
| 2.140 | SMe | SO₂Et | S | 4-Tetrahydropyranyl | |
| 2.141 | SMe | Cl | S | 4-Tetrahydropyranyl | |
| 2.142 | SO₂Me | SO₂Me | S | 4-Tetrahydropyranyl | |
| 2.143 | SO₂Me | SO₂Et | S | 4-Tetrahydropyranyl | |
| 2.144 | SO₂Me | Cl | S | 4-Tetrahydropyranyl | |
| 2.145 | NO₂ | SO₂Me | S | 4-Tetrahydropyranyl | |
| 2.146 | NO₂ | SO₂Et | S | 4-Tetrahydropyranyl | |
| 2.147 | NO₂ | Cl | S | 4-Tetrahydropyranyl | |
| 2.148 | Cl | SO₂Me | S | 3-Tetrahydropyranyl | |
| 2.149 | Cl | SO₂Et | S | 3-Tetrahydropyranyl | |
| 2.150 | Cl | Cl | S | 3-Tetrahydropyranyl | |
| 2.151 | Br | SO₂Me | S | 3-Tetrahydropyranyl | |
| 2.152 | Br | SO₂Et | S | 3-Tetrahydropyranyl | |
| 2.153 | Br | Cl | S | 3-Tetrahydropyranyl | |
| 2.154 | I | SO₂Me | S | 3-Tetrahydropyranyl | |
| 2.155 | I | SO₂Et | S | 3-Tetrahydropyranyl | |
| 2.156 | I | Cl | S | 3-Tetrahydropyranyl | |
| 2.157 | Me | SO₂Me | S | 3-Tetrahydropyranyl | |
| 2.158 | Me | SO₂Et | S | 3-Tetrahydropyranyl | |
| 2.159 | Me | Cl | S | 3-Tetrahydropyranyl | |
| 2.160 | SMe | SO₂Me | S | 3-Tetrahydropyranyl | |
| 2.161 | SMe | SO₂Et | S | 3-Tetrahydropyranyl | |
| 2.162 | SMe | Cl | S | 3-Tetrahydropyranyl | |
| 2.163 | SO₂Me | SO₂Me | S | 3-Tetrahydropyranyl | |
| 2.164 | SO₂Me | SO₂Et | S | 3-Tetrahydropyranyl | |
| 2.165 | SO₂Me | Cl | S | 3-Tetrahydropyranyl | |
| 2.166 | NO₂ | SO₂Me | S | 3-Tetrahydropyranyl | |
| 2.167 | NO₂ | SO₂Et | S | 3-Tetrahydropyranyl | |
| 2.168 | NO₂ | Cl | S | 3-Tetrahydropyranyl | |
| 2.169 | Cl | SO₂Me | S | 1,3-Dioxan-5 yl | |
| 2.170 | Cl | SO₂Et | S | 1,3-Dioxan-5 yl | |
| 2.171 | Cl | Cl | S | 1,3-Dioxan-5 yl | |
| 2.172 | Br | SO₂Me | S | 1,3-Dioxan-5 yl | |
| 2.173 | Br | SO₂Et | S | 1,3-Dioxan-5 yl | |
| 2.174 | Br | Cl | S | 1,3-Dioxan-5 yl | |
| 2.175 | I | SO₂Me | S | 1,3-Dioxan-5 yl | |
| 2.176 | I | SO₂Et | S | 1,3-Dioxan-5 yl | |
| 2.177 | I | Cl | S | 1,3-Dioxan-5 yl | |
| 2.178 | Me | SO₂Me | S | 1,3-Dioxan-5 yl | |
| 2.179 | Me | SO₂Et | S | 1,3-Dioxan-5 yl | |
| 2.180 | Me | Cl | S | 1,3-Dioxan-5 yl | |
| 2.181 | SMe | SO₂Me | S | 1,3-Dioxan-5 yl | |
| 2.182 | SMe | SO₂Et | S | 1,3-Dioxan-5 yl | |
| 2.183 | SMe | Cl | S | 1,3-Dioxan-5 yl | |
| 2.184 | SO₂Me | SO₂Me | S | 1,3-Dioxan-5 yl | |
| 2.185 | SO₂Me | SO₂Et | S | 1,3-Dioxan-5 yl | |
| 2.186 | SO₂Me | Cl | S | 1,3-Dioxan-5 yl | |
| 2.187 | NO₂ | SO₂Me | S | 1,3-Dioxan-5 yl | |
| 2.188 | NO₂ | SO₂Et | S | 1,3-Dioxan-5 yl | |
| 2.189 | NO₂ | Cl | S | 1,3-Dioxan-5 yl | |
| 2.190 | Cl | SO₂Me | S | γ-butyrolacton-2-yl | |
| 2.191 | Cl | SO₂Et | S | γ-butyrolacton-2-yl | |
| 2.192 | Cl | Cl | S | γ-butyrolacton-2-yl | |
| 2.193 | Br | SO₂Me | S | γ-butyrolacton-2-yl | |
| 2.194 | Br | SO₂Et | S | γ-butyrolacton-2-yl | |
| 2.195 | Br | Cl | S | γ-butyrolacton-2-yl | |
| 2.196 | I | SO₂Me | S | γ-butyrolacton-2-yl | |
| 2.197 | I | SO₂Et | S | γ-butyrolacton-2-yl | |
| 2.198 | I | Cl | S | γ-butyrolacton-2-yl | |
| 2.199 | Me | SO₂Me | S | γ-butyrolacton-2-yl | |
| 2.200 | Me | SO₂Et | S | γ-butyrolacton-2-yl | |
| 2.201 | Me | Cl | S | γ-butyrolacton-2-yl | |
| 2.202 | SMe | SO₂Me | S | γ-butyrolacton-2-yl | |
| 2.203 | SMe | SO₂Et | S | γ-butyrolacton-2-yl | |
| 2.204 | SMe | Cl | S | γ-butyrolacton-2-yl | |
| 2.205 | SO₂Me | SO₂Me | S | γ-butyrolacton-2-yl | |
| 2.206 | SO₂Me | SO₂Et | S | γ-butyrolacton-2-yl | |
| 2.207 | SO₂Me | Cl | S | γ-butyrolacton-2-yl | |
| 2.208 | NO₂ | SO₂Me | S | γ-butyrolacton-2-yl | |
| 2.209 | NO₂ | SO₂Et | S | γ-butyrolacton-2-yl | |
| 2.210 | NO₂ | Cl | S | γ-butyrolacton-2-yl | |
| 2.211 | SEt | Cl | S | 3-Tetrahydrofuranyl | |
| 2.212 | SEt | Cl | S | 3-Tetrahydropyranyl | |
| 2.213 | SEt | Cl | S | 4-Tetrahydropyranyl | |
| 2.214 | SEt | Cl | S | 1,3-Dioxan-5 yl | |
| 2.215 | SEt | Cl | S | γ-butyrolacton-2-yl | |
| 2.216 | SO₂Et | Cl | S | 3-Tetrahydrofuranyl | |
| 2.217 | SO₂Et | Cl | S | 3-Tetrahydropyranyl | |
| 2.218 | SO₂Et | Cl | S | 4-Tetrahydropyranyl | |
| 2.219 | SO₂Et | Cl | S | 1,3-Dioxan-5 yl | |
| 2.220 | SO₂Et | Cl | S | γ-butyrolacton-2-yl | |
| 2.221 | OMe | Cl | S | 3-Tetrahydrofuranyl | |
| 2.222 | OMe | Cl | S | 3-Tetrahydropyranyl | |
| 2.223 | OMe | Cl | S | 4-Tetrahydropyranyl | |
| 2.224 | OMe | Cl | S | 1,3-Dioxan-5 yl | |
| 2.225 | OMe | Cl | S | γ-butyrolacton-2-yl | |

**Tabelle 3: Erfindungsgemäße Verbindungen der allgemeinen Formel (I), worin die Substituenten und Symbole folgende Bedeutungen haben:**

| | | | | | |
|---|---|---|---|---|---|
| | R³ = H | | Q = Q1 | | p = 2 |
| 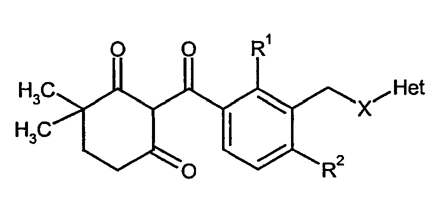 | | | | | |

| **Nr** | **R¹** | **R²** | **X** | **Het** | **Physikal. Daten** |
|---|---|---|---|---|---|
| 3.1 | Cl | SO₂Me | O | 3-Tetrahydrofuranyl | Öl |
| 3.2 | Cl | SO₂Et | O | 3-Tetrahydrofuranyl | Öl |
| 3.3 | Cl | Cl | O | 3-Tetrahydrofuranyl | |
| 3.4 | Br | SO₂Me | O | 3-Tetrahydrofuranyl | |
| 3.5 | Br | SO₂Et | O | 3-Tetrahydrofuranyl | |
| 3.6 | Br | Cl | O | 3-Tetrahydrofuranyl | |
| 3.7 | I | SO₂Me | O | 3-Tetrahydrofuranyl | |
| 3.8 | I | SO₂Et | O | 3-Tetrahydrofuranyl | |
| 3.9 | I | Cl | O | 3-Tetrahydrofuranyl | |
| 3.10 | Me | SO₂Me | O | 3-Tetrahydrofuranyl | |
| 3.11 | Me | SO₂Et | O | 3-Tetrahydrofuranyl | |
| 3.12 | Me | Cl | O | 3-Tetrahydrofuranyl | |
| 3.13 | SMe | SO₂Me | O | 3-Tetrahydrofuranyl | |
| 3.14 | SMe | SO₂Et | O | 3-Tetrahydrofuranyl | |
| 3.15 | SMe | Cl | O | 3-Tetrahydrofuranyl | |
| 3.16 | SO₂Me | SO₂Me | O | 3-Tetrahydrofuranyl | |
| 3.17 | SO₂Me | SO₂Et | O | 3-Tetrahydrofuranyl | |
| 3.18 | SO₂Me | Cl | O | 3-Tetrahydrofuranyl | |
| 3.19 | NO₂ | SO₂Me | O | 3-Tetrahydrofuranyl | |
| 3.20 | NO₂ | SO₂Et | O | 3-Tetrahydrofuranyl | |
| 3.21 | NO₂ | Cl | O | 3-Tetrahydrofuranyl | |
| 3.22 | Cl | SO₂Me | O | 4-Tetrahydropyranyl | |
| 3.23 | Cl | SO₂Et | O | 4-Tetrahydropyranyl | |
| 3.24 | Cl | Cl | O | 4-Tetrahydropyranyl | |
| 3.25 | Br | SO₂Me | O | 4-Tetrahydropyranyl | |
| 3.26 | Br | SO₂Et | O | 4-Tetrahydropyranyl | |
| 3.27 | Br | Cl | O | 4-Tetrahydropyranyl | |
| 3.28 | I | SO₂Me | O | 4-Tetrahydropyranyl | |
| 3.29 | I | SO₂Et | O | 4-Tetrahydropyranyl | |
| 3.30 | I | Cl | O | 4-Tetrahydropyranyl | |
| 3.31 | Me | SO₂Me | O | 4-Tetrahydropyranyl | |
| 3.32 | Me | SO₂Et | O | 4-Tetrahydropyranyl | |
| 3.33 | Me | Cl | O | 4-Tetrahydropyranyl | |
| 3.34 | SMe | SO₂Me | O | 4-Tetrahydropyranyl | |
| 3.35 | SMe | SO₂Et | O | 4-Tetrahydropyranyl | |
| 3.36 | SMe | Cl | O | 4-Tetrahydropyranyl | |
| 3.37 | SO₂Me | SO₂Me | O | 4-Tetrahydropyranyl | |
| 3.38 | SO₂Me | SO₂Et | O | 4-Tetrahydropyranyl | |
| 3.39 | SO₂Me | Cl | O | 4-Tetrahydropyranyl | |
| 3.40 | NO₂ | SO₂Me | O | 4-Tetrahydropyranyl | |
| 3.41 | NO₂ | SO₂Et | O | 4-Tetrahydropyranyl | |
| 3.42 | NO₂ | Cl | O | 4-Tetrahydropyranyl | |
| 3.43 | Cl | SO₂Me | O | 3-Tetrahydropyranyl | |
| 3.44 | Cl | SO₂Et | O | 3-Tetrahydropyranyl | |
| 3.45 | Cl | Cl | O | 3-Tetrahydropyranyl | |
| 3.46 | Br | SO₂Me | O | 3-Tetrahydropyranyl | |
| 3.47 | Br | SO₂Et | O | 3-Tetrahydropyranyl | |
| 3.48 | Br | Cl | O | 3-Tetrahydropyranyl | |
| 3.49 | I | SO₂Me | O | 3-Tetrahydropyranyl | |
| 3.50 | I | SO₂Et | O | 3-Tetrahydropyranyl | |
| 3.51 | I | Cl | O | 3-Tetrahydropyranyl | |
| 3.52 | Me | SO₂Me | O | 3-Tetrahydropyranyl | |
| 3.53 | Me | SO₂Et | O | 3-Tetrahydropyranyl | |
| 3.54 | Me | Cl | O | 3-Tetrahydropyranyl | |
| 3.55 | SMe | SO₂Me | O | 3-Tetrahydropyranyl | |
| 3.56 | SMe | SO₂Et | O | 3-Tetrahydropyranyl | |
| 3.57 | SMe | Cl | O | 3-Tetrahydropyranyl | |
| 3.58 | SO₂Me | SO₂Me | O | 3-Tetrahydropyranyl | |
| 3.59 | SO₂Me | SO₂Et | O | 3-Tetrahydropyranyl | |
| 3.60 | SO₂Me | Cl | O | 3-Tetrahydropyranyl | |
| 3.61 | NO₂ | SO₂Me | O | 3-Tetrahydropyranyl | |
| 3.62 | NO₂ | SO₂Et | O | 3-Tetrahydropyranyl | |
| 3.63 | NO₂ | Cl | O | 3-Tetrahydropyranyl | |
| 3.64 | Cl | SO₂Me | O | 1,3-Dioxan-5 yl | |
| 3.65 | Cl | SO₂Et | O | 1,3-Dioxan-5 yl | |
| 3.66 | Cl | Cl | O | 1,3-Dioxan-5 yl | |
| 3.67 | Br | SO₂Me | O | 1,3-Dioxan-5 yl | |
| 3.68 | Br | SO₂Et | O | 1,3-Dioxan-5 yl | |
| 3.69 | Br | Cl | O | 1,3-Dioxan-5 yl | |
| 3.70 | I | SO₂Me | O | 1,3-Dioxan-5 yl | |
| 3.71 | I | SO₂Et | O | 1,3-Dioxan-5 yl | |
| 3.72 | I | Cl | O | 1,3-Dioxan-5 yl | |
| 3.73 | Me | SO₂Me | O | 1,3-Dioxan-5 yl | |
| 3.74 | Me | SO₂Et | O | 1,3-Dioxan-5 yl | |
| 3.75 | Me | Cl | O | 1,3-Dioxan-5 yl | |
| 3.76 | SMe | SO₂Me | O | 1,3-Dioxan-5 yl | |
| 3.77 | SMe | SO₂Et | O | 1,3-Dioxan-5 yl | |
| 3.78 | SMe | Cl | O | 1,3-Dioxan-5 yl | |
| 3.79 | SO₂Me | SO₂Me | O | 1,3-Dioxan-5 yl | |
| 3.80 | SO₂Me | SO₂Et | O | 1,3-Dioxan-5 yl | |
| 3.81 | SO₂Me | Cl | O | 1,3-Dioxan-5 yl | |
| 3.82 | NO₂ | SO₂Me | O | 1,3-Dioxan-5 yl | |
| 3.83 | NO₂ | SO₂Et | O | 1,3-Dioxan-5 yl | |
| 3.84 | NO₂ | Cl | O | 1,3-Dioxan-5 yl | |
| 3.85 | Cl | SO₂Me | O | γ-butyrolacton-2-yl | |
| 3.86 | Cl | SO₂Et | O | γ-butyrolacton-2-yl | |
| 3.87 | Cl | Cl | O | γ-butyrolacton-2-yl | |
| 3.88 | Br | SO₂Me | O | γ-butyrolacton-2-yl | |
| 3.89 | Br | SO₂Et | O | γ-butyrolacton-2-yl | |
| 3.90 | Br | Cl | O | γ-butyrolacton-2-yl | |
| 3.91 | I | SO₂Me | O | γ-butyrolacton-2-yl | |
| 3.92 | I | SO₂Et | O | γ-butyrolacton-2-yl | |
| 3.93 | I | Cl | O | γ-butyrolacton-2-yl | |
| 3.94 | Me | SO₂Me | O | γ-butyrolacton-2-yl | |
| 3.95 | Me | SO₂Et | O | γ-butyrolacton-2-yl | |
| 3.96 | Me | Cl | O | γ-butyrolacton-2-yl | |
| 3.97 | SMe | SO₂Me | O | γ-butyrolacton-2-yl | |
| 3.98 | SMe | SO₂Et | O | γ-butyrolacton-2-yl | |
| 3.99 | SMe | Cl | O | γ-butyrolacton-2-yl | |
| 3.100 | SO₂Me | SO₂Me | O | γ-butyrolacton-2-yl | |
| 3.101 | SO₂Me | SO₂Et | O | γ-butyrolacton-2-yl | |
| 3.102 | SO₂Me | Cl | O | γ-butyrolacton-2-yl | |
| 3.103 | NO₂ | SO₂Me | O | γ-butyrolacton-2-yl | |
| 3.104 | NO₂ | SO₂Et | O | γ-butyrolacton-2-yl | |
| 3.105 | NO₂ | Cl | O | γ-butyrolacton-2-yl | |
| 3.106 | Cl | SO₂Me | S | 3-Tetrahydrofuranyl | |
| 3.107 | Cl | SO₂Et | S | 3-Tetrahydrofuranyl | |
| 3.108 | Cl | Cl | S | 3-Tetrahydrofuranyl | |
| 3.109 | Br | SO₂Me | S | 3-Tetrahydrofuranyl | |
| 3.110 | Br | SO₂Et | S | 3-Tetrahydrofuranyl | |
| 3.111 | Br | Cl | S | 3-Tetrahydrofuranyl | |
| 3.112 | I | SO₂Me | S | 3-Tetrahydrofuranyl | |
| 3.113 | I | SO₂Et | S | 3-Tetrahydrofuranyl | |
| 3.114 | I | Cl | S | 3-Tetrahydrofuranyl | |
| 3.115 | Me | SO₂Me | S | 3-Tetrahydrofuranyl | |
| 3.116 | Me | SO₂Et | S | 3-Tetrahydrofuranyl | |
| 3.117 | Me | Cl | S | 3-Tetrahydrofuranyl | |
| 3.118 | SMe | SO₂Me | S | 3-Tetrahydrofuranyl | |
| 3.119 | SMe | SO₂Et | S | 3-Tetrahydrofuranyl | |
| 3.120 | SMe | Cl | S | 3-Tetrahydrofuranyl | |
| 3.121 | SO₂Me | SO₂Me | S | 3-Tetrahydrofuranyl | |
| 3.122 | SO₂Me | SO₂Et | S | 3-Tetrahydrofuranyl | |
| 3.123 | SO₂Me | Cl | S | 3-Tetrahydrofuranyl | |
| 3.124 | NO₂ | SO₂Me | S | 3-Tetrahydrofuranyl | |
| 3.125 | NO₂ | SO₂Et | S | 3-Tetrahydrofuranyl | |
| 3.126 | NO₂ | Cl | S | 3-Tetrahydrofuranyl | |
| 3.127 | Cl | SO₂Me | S | 4-Tetrahydropyranyl | |
| 3.128 | Cl | SO₂Et | S | 4-Tetrahydropyranyl | |
| 3.129 | Cl | Cl | S | 4-Tetrahydropyranyl | |
| 3.130 | Br | SO₂Me | S | 4-Tetrahydropyranyl | |
| 3.131 | Br | SO₂Et | S | 4-Tetrahydropyranyl | |
| 3.132 | Br | Cl | S | 4-Tetrahydropyranyl | |
| 3.133 | I | SO₂Me | S | 4-Tetrahydropyranyl | |
| 3.134 | I | SO₂Et | S | 4-Tetrahydropyranyl | |
| 3.135 | I | Cl | S | 4-Tetrahydropyranyl | |
| 3.136 | Me | SO₂Me | S | 4-Tetrahydropyranyl | |
| 3.137 | Me | SO₂Et | S | 4-Tetrahydropyranyl | |
| 3.138 | Me | Cl | S | 4-Tetrahydropyranyl | |
| 3.139 | SMe | SO₂Me | S | 4-Tetrahydropyranyl | |
| 3.140 | SMe | SO₂Et | S | 4-Tetrahydropyranyl | |
| 3.141 | SMe | Cl | S | 4-Tetrahydropyranyl | |
| 3.142 | SO₂Me | SO₂Me | S | 4-Tetrahydropyranyl | |
| 3.143 | SO₂Me | SO₂Et | S | 4-Tetrahydropyranyl | |
| 3.144 | SO₂Me | Cl | S | 4-Tetrahydropyranyl | |
| 3.145 | NO₂ | SO₂Me | S | 4-Tetrahydropyranyl | |
| 3.146 | NO₂ | SO₂Et | S | 4-Tetrahydropyranyl | |
| 3.147 | NO₂ | Cl | S | 4-Tetrahydropyranyl | |
| 3.148 | Cl | SO₂Me | S | 3-Tetrahydropyranyl | |
| 3.149 | Cl | SO₂Et | S | 3-Tetrahydropyranyl | |
| 3.150 | Cl | Cl | S | 3-Tetrahydropyranyl | |
| 3.151 | Br | SO₂Me | S | 3-Tetrahydropyranyl | |
| 3.152 | Br | SO₂Et | S | 3-Tetrahydropyranyl | |
| 3.153 | Br | Cl | S | 3-Tetrahydropyranyl | |
| 3.154 | I | SO₂Me | S | 3-Tetrahydropyranyl | |
| 3.155 | I | SO₂Et | S | 3-Tetrahydropyranyl | |
| 3.156 | I | Cl | S | 3-Tetrahydropyranyl | |
| 3.157 | Me | SO₂Me | S | 3-Tetrahydropyranyl | |
| 3.158 | Me | SO₂Et | S | 3-Tetrahydropyranyl | |
| 3.159 | Me | Cl | S | 3-Tetrahydropyranyl | |
| 3.160 | SMe | SO₂Me | S | 3-Tetrahydropyranyl | |
| 3.161 | SMe | SO₂Et | S | 3-Tetrahydropyranyl | |
| 3.162 | SMe | Cl | S | 3-Tetrahydropyranyl | |
| 3.163 | SO₂Me | SO₂Me | S | 3-Tetrahydropyranyl | |
| 3.164 | SO₂Me | SO₂Et | S | 3-Tetrahydropyranyl | |
| 3.165 | SO₂Me | Cl | S | 3-Tetrahydropyranyl | |
| 3.166 | NO₂ | SO₂Me | S | 3-Tetrahydropyranyl | |
| 3.167 | NO₂ | SO₂Et | S | 3-Tetrahydropyranyl | |
| 3.168 | NO₂ | Cl | S | 3-Tetrahydropyranyl | |
| 3.169 | Cl | SO₂Me | S | 1,3-Dioxan-5 yl | |
| 3.170 | Cl | SO₂Et | S | 1,3-Dioxan-5 yl | |
| 3.171 | Cl | Cl | S | 1,3-Dioxan-5 yl | |
| 3.172 | Br | SO₂Me | S | 1,3-Dioxan-5 yl | |
| 3.173 | Br | SO₂Et | S | 1,3-Dioxan-5 yl | |
| 3.174 | Br | Cl | S | 1,3-Dioxan-5 yl | |
| 3.175 | I | SO₂Me | S | 1,3-Dioxan-5 yl | |
| 3.176 | I | SO₂Et | S | 1,3-Dioxan-5 yl | |
| 3.177 | I | Cl | S | 1,3-Dioxan-5 yl | |
| 3.17 | Me | SO₂Me | S | 1,3-Dioxan-5 yl | |
| 3.179 | Me | SO₂Et | S | 1,3-Dioxan-5 yl | |
| 3.180 | Me | Cl | S | 1,3-Dioxan-5 yl | |
| 3.181 | SMe | SO₂Me | S | 1,3-Dioxan-5 yl | |
| 3.182 | SMe | SO₂Et | S | 1,3-Dioxan-5 yl | |
| 3.183 | SMe | Cl | S | 1,3-Dioxan-5 yl | |
| 3.184 | SO₂Me | SO₂Me | S | 1,3-Dioxan-5 yl | |
| 3.185 | SO₂Me | SO₂Et | S | 1,3-Dioxan-5 yl | |
| 3.186 | SO₂Me | Cl | S | 1,3-Dioxan-5 yl | |
| 3.187 | NO₂ | SO₂Me | S | 1,3-Dioxan-5 yl | |
| 3.188 | NO₂ | SO₂Et | S | 1,3-Dioxan-5 yl | |
| 3.189 | NO₂ | Cl | S | 1,3-Dioxan-5 yl | |
| 3.190 | Cl | SO₂Me | S | γ-butyrolacton-2-yl | |
| 3.191 | Cl | SO₂Et | S | γ-butyrolacton-2-yl | |
| 3.192 | Cl | Cl | S | γ-butyrolacton-2-yl | |
| 3.193 | Br | SO₂Me | S | γ-butyrolacton-2-yl | |
| 3.194 | Br | SO₂Et | S | γ-butyrolacton-2-yl | |
| 3.195 | Br | Cl | S | γ-butyrolacton-2-yl | |
| 3.196 | I | SO₂Me | S | γ-butyrolacton-2-yl | |
| 3.197 | I | SO₂Et | S | γ-butyrolacton-2-yl | |
| 3.198 | I | Cl | S | γ-butyrolacton-2-yl | |
| 3.199 | Me | SO₂Me | S | γ-butyrolacton-2-yl | |
| 3.200 | Me | SO₂Et | S | γ-butyrolacton-2-yl | |
| 3.201 | Me | Cl | S | γ-butyrolacton-2-yl | |
| 3.202 | SMe | SO₂Me | S | γ-butyrolacton-2-yl | |
| 3.203 | SMe | SO₂Et | S | γ-butyrolacton-2-yl | |
| 3.204 | SMe | Cl | S | γ-butyrolacton-2-yl | |
| 3.205 | SO₂Me | SO₂Me | S | γ-butyrolacton-2-yl | |
| 3.206 | SO₂Me | SO₂Et | S | γ-butyrolacton-2-yl | |
| 3.207 | SO₂Me | Cl | S | γ-butyrolacton-2-yl | |
| 3.208 | NO₂ | SO₂Me | S | γ-butyrolacton-2-yl | |
| 3.209 | NO₂ | SO₂Et | S | γ-butyrolacton-2-yl | |
| 3.210 | NO₂ | Cl | S | γ-butyrolacton-2-yl | |

**Tabelle 4: Erfindungsgemäße Verbindungen der allgemeinen Formel (I), worin die Substituenten und Symbole folgende Bedeutungen haben:**

| | | | | | |
|---|---|---|---|---|---|
| | R³ = H | | Q = Q2 | | R⁶ = Me |
| | R⁷ = Me | | R⁸ = H | | |
| 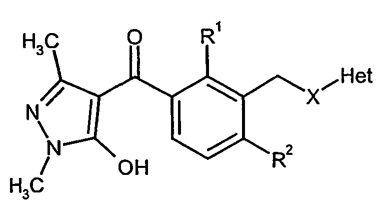 | | | | | |

| **Nr.** | **R¹** | **R²** | **X** | **Het** | **Physikal. Daten** |
|---|---|---|---|---|---|
| 4.1 | Cl | SO₂Me | O | 3-Tetrahydrofuranyl | Öl |
| 4.2 | Cl | SO₂Et | O | 3-Tetrahydrofuranyl | Öl |
| 4.3 | Cl | Cl | O | 3-Tetrahydrofuranyl | |
| 4.4 | Br | SO₂Me | O | 3-Tetrahydrofuranyl | |
| 4.5 | Br | SO₂Et | O | 3-Tetrahydrofuranyl | |
| 4.6 | Br | Cl | O | 3-Tetrahydrofuranyl | |
| 4.7 | I | SO₂Me | O | 3-Tetrahydrofuranyl | |
| 4.8 | I | SO₂Et | O | 3-Tetrahydrofuranyl | |
| 4.9 | I | Cl | O | 3-Tetrahydrofuranyl | |
| 4.10 | Me | SO₂Me | O | 3-Tetrahydrofuranyl | |
| 4.11 | Me | SO₂Et | O | 3-Tetrahydrofuranyl | |
| 4.12 | Me | Cl | O | 3-Tetrahydrofuranyl | |
| 4.13 | SMe | SO₂Me | O | 3-Tetrahydrofuranyl | |
| 4.14 | SMe | SO₂Et | O | 3-Tetrahydrofuranyl | |
| 4.15 | SMe | Cl | O | 3-Tetrahydrofuranyl | |
| 4.16 | SO₂Me | SO₂Me | O | 3-Tetrahydrofuranyl | |
| 4.17 | SO₂Me | SO₂Et | O | 3-Tetrahydrofuranyl | |
| 4.18 | SO₂Me | Cl | O | 3-Tetrahydrofuranyl | |
| 4.19 | NO₂ | SO₂Me | O | 3-Tetrahydrofuranyl | |
| 4.20 | NO₂ | SO₂Et | O | 3-Tetrahydrofuranyl | |
| 4.21 | NO₂ | Cl | O | 3-Tetrahydrofuranyl | |
| 4.22 | Cl | SO₂Me | O | 4-Tetrahydropyranyl | |
| 4.23 | Cl | SO₂Et | O | 4-Tetrahydropyranyl | |
| 4.24 | Cl | Cl | O | 4-Tetrahydropyranyl | |
| 4.25 | Br | SO₂Me | O | 4-Tetrahydropyranyl | |
| 4.26 | Br | SO₂Et | O | 4-Tetrahydropyranyl | |
| 4.27 | Br | Cl | O | 4-Tetrahydropyranyl | |
| 4.28 | I | SO₂Me | O | 4-Tetrahydropyranyl | |
| 4.29 | **I** | SO₂Et | O | 4-Tetrahydropyranyl | |
| 4.30 | I | Cl | O | 4-Tetrahydropyranyl | |
| 4.31 | Me | SO₂Me | O | 4-Tetrahydropyranyl | |
| 4.32 | Me | SO₂Et | O | 4-Tetrahydropyranyl | |
| 4.33 | Me | Cl | O | 4-Tetrahydropyranyl | |
| 4.34 | SMe | SO₂Me | O | 4-Tetrahydropyranyl | |
| 4.35 | SMe | SO₂Et | O | 4-Tetrahydropyranyl | |
| 4.36 | SMe | Cl | O | 4-Tetrahydropyranyl | |
| 4.37 | SO₂Me | SO₂Me | O | 4-Tetrahydropyranyl | |
| 4.38 | SO₂Me | SO₂Et | O | 4-Tetrahydropyranyl | |
| 4.39 | SO₂Me | Cl | O | 4-Tetrahydropyranyl | |
| 4.40 | NO₂ | SO₂Me | O | 4-Tetrahydropyranyl | |
| 4.41 | NO₂ | SO₂Et | O | 4-Tetrahydropyranyl | |
| 4.42 | NO₂ | Cl | O | 4-Tetrahydropyranyl | |
| 4.43 | Cl | SO₂Me | O | 3-Tetrahydropyranyl | |
| 4.44 | Cl | SO₂Et | O | 3-Tetrahydropyranyl | |
| 4.45 | Cl | Cl | O | 3-Tetrahydropyranyl | |
| 4.46 | Br | SO₂Me | O | 3-Tetrahydropyranyl | |
| 4.47 | Br | SO₂Et | O | 3-Tetrahydropyranyl | |
| 4.48 | Br | Cl | O | 3-Tetrahydropyranyl | |
| 4.49 | I | SO₂Me | O | 3-Tetrahydropyranyl | |
| 4.50 | I | SO₂Et | O | 3-Tetrahydropyranyl | |
| 4.51 | I | Cl | O | 3-Tetrahydropyranyl | |
| 4.52 | Me | SO₂Me | O | 3-Tetrahydropyranyl | |
| 4.53 | Me | SO₂Et | O | 3-Tetrahydropyranyl | |
| 4.54 | Me | Cl | O | 3-Tetrahydropyranyl | |
| 4.55 | SMe | SO₂Me | O | 3-Tetrahydropyranyl | |
| 4.56 | SMe | SO₂Et | O | 3-Tetrahydropyranyl | |
| 4.57 | SMe | Cl | O | 3-Tetrahydropyranyl | |
| 4.58 | SO₂Me | SO₂Me | O | 3-Tetrahydropyranyl | |
| 4.59 | SO₂Me | SO₂Et | O | 3-Tetrahydropyranyl | |
| 4.60 | SO₂Me | Cl | O | 3-Tetrahydropyranyl | |
| 4.61 | NO₂ | SO₂Me | O | 3-Tetrahydropyranyl | |
| 4.62 | NO₂ | SO₂Et | O | 3-Tetrahydropyranyl | |
| 4.63 | NO₂ | Cl | O | 3-Tetrahydropyranyl | |
| 4.64 | Cl | SO₂Me | O | 1,3-Dioxan-5 yl | |
| 4.65 | Cl | SO₂Et | O | 1,3-Dioxan-5 yl | |
| 4.66 | Cl | Cl | O | 1,3-Dioxan-5 yl | |
| 4.67 | Br | SO₂Me | O | 1,3-Dioxan-5 yl | |
| 4.68 | Br | SO₂Et | O | 1,3-Dioxan-5 yl | |
| 4.69 | Br | Cl | O | 1,3-Dioxan-5 yl | |
| 4.70 | I | SO₂Me | O | 1,3-Dioxan-5 yl | |
| 4.71 | I | SO₂Et | O | 1,3-Dioxan-5 yl | |
| 4.72 | I | Cl | O | 1,3-Dioxan-5 yl | |
| 4.73 | Me | SO₂Me | O | 1,3-Dioxan-5 yl | |
| 4.74 | Me | SO₂Et | O | 1,3-Dioxan-5 yl | |
| 4.75 | Me | Cl | O | 1,3-Dioxan-5 yl | |
| 4.76 | SMe | SO₂Me | O | 1,3-Dioxan-5 yl | |
| 4.77 | SMe | SO₂Et | O | 1,3-Dioxan-5 yl | |
| 4.78 | SMe | Cl | O | 1,3-Dioxan-5 yl | |
| 4.79 | SO₂Me | SO₂Me | O | 1,3-Dioxan-5 yl | |
| 4.80 | SO₂Me | SO₂Et | O | 1,3-Dioxan-5 yl | |
| 4.81 | SO₂Me | Cl | O | 1,3-Dioxan-5 yl | |
| 4.82 | NO₂ | SO₂Me | O | 1,3-Dioxan-5 yl | |
| 4.83 | NO₂ | SO₂Et | O | 1,3-Dioxan-5 yl | |
| 4.84 | NO₂ | Cl | O | 1,3-Dioxan-5 yl | |
| 4.85 | Cl | SO₂Me | O | γ-butyrolacton-2-yl | |
| 4.86 | Cl | SO₂Et | O | γ-butyrolacton-2-yl | |
| 4.87 | Cl | Cl | O | γ-butyrolacton-2-yl | |
| 4.88 | Br | SO₂Me | O | γ-butyrolacton-2-yl | |
| 4.89 | Br | SO₂Et | O | γ-butyrolacton-2-yl | |
| 4.90 | Br | Cl | O | γ-butyrolacton-2-yl | |
| 4.91 | I | SO₂Me | O | γ-butyrolacton-2-yl | |
| 4.92 | I | SO₂Et | O | γ-butyrolacton-2-yl | |
| 4.93 | I | Cl | O | γ-butyrolacton-2-yl | |
| 4.94 | Me | SO₂Me | O | γ-butyrolacton-2-yl | |
| 4.95 | Me | SO₂Et | O | γ-butyrolacton-2-yl | |
| 4.96 | Me | Cl | O | γ-butyrolacton-2-yl | |
| 4.97 | SMe | SO₂Me | O | γ-butyrolacton-2-yl | |
| 4.98 | SMe | SO₂Et | O | γ-butyrolacton-2-yl | |
| 4.99 | SMe | Cl | O | γ-butyrolacton-2-yl | |
| 4.100 | SO₂Me | SO₂Me | O | γ-butyrolacton-2-yl | |
| 4.101 | SO₂Me | SO₂Et | O | γ-butyrolacton-2-yl | |
| 4.102 | SO₂Me | Cl | O | γ-butyrolacton-2-yl | |
| 4.103 | NO₂ | SO₂Me | O | γ-butyrolacton-2-yl | |
| 4.104 | NO₂ | SO₂Et | O | γ-butyrolacton-2-yl | |
| 4.105 | NO₂ | Cl | O | γ-butyrolacton-2-yl | |
| 4.106 | Cl | SO₂Me | S | 3-Tetrahydrofuranyl | |
| 4.107 | Cl | SO₂Et | S | 3-Tetrahydrofuranyl | |
| 4.108 | Cl | Cl | S | 3-Tetrahydrofuranyl | |
| 4.109 | Br | SO₂Me | S | 3-Tetrahydrofuranyl | |
| 4.110 | Br | SO₂Et | S | 3-Tetrahydrofuranyl | |
| 4.111 | Br | Cl | S | 3-Tetrahydrofuranyl | |
| 4.112 | I | SO₂Me | S | 3-Tetrahydrofuranyl | |
| 4.113 | I | SO₂Et | S | 3-Tetrahydrofuranyl | |
| 4.114 | I | Cl | S | 3-Tetrahydrofuranyl | |
| 4.115 | Me | SO₂Me | S | 3-Tetrahydrofuranyl | |
| 4.116 | Me | SO₂Et | S | 3-Tetrahydrofuranyl | |
| 4.117 | Me | Cl | S | 3-Tetrahydrofuranyl | |
| 4.118 | SMe | SO₂Me | S | 3-Tetrahydrofuranyl | |
| 4.119 | SMe | SO₂Et | S | 3-Tetrahydrofuranyl | |
| 4.120 | SMe | Cl | S | 3-Tetrahydrofuranyl | |
| 4.121 | SO₂Me | SO₂Me | S | 3-Tetrahydrofuranyl | |
| 4.122 | SO₂Me | SO₂Et | S | 3-Tetrahydrofuranyl | |
| 4.123 | SO₂Me | Cl | S | 3-Tetrahydrofuranyl | |
| 4.124 | NO₂ | SO₂Me | S | 3-Tetrahydrofuranyl | |
| 4.125 | NO₂ | SO₂Et | S | 3-Tetrahydrofuranyl | |
| 4.126 | NO₂ | Cl | S | 3-Tetrahydrofuranyl | |
| 4.127 | Cl | SO₂Me | S | 4-Tetrahydropyranyl | |
| 4.128 | Cl | SO₂Et | S | 4-Tetrahydropyranyl | |
| 4.129 | Cl | Cl | S | 4-Tetrahydropyranyl | |
| 4.130 | Br | SO₂Me | S | 4-Tetrahydropyranyl | |
| 4.131 | Br | SO₂Et | S | 4-Tetrahydropyranyl | |
| 4.132 | Br | Cl | S | 4-Tetrahydropyranyl | |
| 4.133 | I | SO₂Me | S | 4-Tetrahydropyranyl | |
| 4.134 | I | SO₂Et | S | 4-Tetrahydropyranyl | |
| 4.135 | I | Cl | S | 4-Tetrahydropyranyl | |
| 4.136 | Me | SO₂Me | S | 4-Tetrahydropyranyl | |
| 4.137 | Me | SO₂Et | S | 4-Tetrahydropyranyl | |
| 4.138 | Me | Cl | S | 4-Tetrahydropyranyl | |
| 4.139 | SMe | SO₂Me | S | 4-Tetrahydropyranyl | |
| 4.140 | SMe | SO₂Et | S | 4-Tetrahydropyranyl | |
| 4.141 | SMe | Cl | S | 4-Tetrahydropyranyl | |
| 4.142 | SO₂Me | SO₂Me | S | 4-Tetrahydropyranyl | |
| 4.143 | SO₂Me | SO₂Et | S | 4-Tetrahydropyranyl | |
| 4.144 | SO₂Me | Cl | S | 4-Tetrahydropyranyl | |
| 4.145 | NO₂ | SO₂Me | S | 4-Tetrahydropyranyl | |
| 4.146 | NO₂ | SO₂Et | S | 4-Tetrahydropyranyl | |
| 4.147 | NO₂ | Cl | S | 4-Tetrahydropyranyl | |
| 4.148 | Cl | SO₂Me | S | 3-Tetrahydropyranyl | |
| 4.149 | Cl | SO₂Et | S | 3-Tetrahydropyranyl | |
| 4.150 | Cl | Cl | S | 3-Tetrahydropyranyl | |
| 4.151 | Br | SO₂Me | S | 3-Tetrahydropyranyl | |
| 4.152 | Br | SO₂Et | S | 3-Tetrahydropyranyl | |
| 4.153 | Br | Cl | S | 3-Tetrahydropyranyl | |
| 4.154 | I | SO₂Me | S | 3-Tetrahydropyranyl | |
| 4.155 | I | SO₂Et | S | 3-Tetrahydropyranyl | |
| 4.156 | I | Cl | S | 3-Tetrahydropyranyl | |
| 4.157 | Me | SO₂Me | S | 3-Tetrahydropyranyl | |
| 4.158 | Me | SO₂Et | S | 3-Tetrahydropyranyl | |
| 4.159 | Me | Cl | S | 3-Tetrahydropyranyl | |
| 4.160 | SMe | SO₂Me | S | 3-Tetrahydropyranyl | |
| 4.161 | SMe | SO₂Et | S | 3-Tetrahydropyranyl | |
| 4.162 | SMe | Cl | S | 3-Tetrahydropyranyl | |
| 4.163 | SO₂Me | SO₂Me | S | 3-Tetrahydropyranyl | |
| 4.164 | SO₂Me | SO₂Et | S | 3-Tetrahydropyranyl | |
| 4.165 | SO₂Me | Cl | S | 3-Tetrahydropyranyl | |
| 4.166 | NO₂ | SO₂Me | S | 3-Tetrahydropyranyl | |
| 4.167 | NO₂ | SO₂Et | S | 3-Tetrahydropyranyl | |
| 4.168 | NO₂ | Cl | S | 3-Tetrahydropyranyl | |
| 4.169 | Cl | SO₂Me | S | 1,3-Dioxan-5 yl | |
| 4.170 | Cl | SO₂Et | S | 1,3-Dioxan-5 yl | |
| 4.171 | Cl | Cl | S | 1,3-Dioxan-5 yl | |
| 4.172 | Br | SO₂Me | S | 1,3-Dioxan-5 yl | |
| 4.173 | Br | SO₂Et | S | 1,3-Dioxan-5 yl | |
| 4.174 | Br | Cl | S | 1,3-Dioxan-5 yl | |
| 4.175 | I | SO₂Me | S | 1,3-Dioxan-5 yl | |
| 4.176 | I | SO₂Et | S | 1,3-Dioxan-5 yl | |
| 4.177 | I | Cl | S | 1,3-Dioxan-5 yl | |
| 4.178 | Me | SO₂Me | S | 1,3-Dioxan-5 yl | |
| 4.179 | Me | SO₂Et | S | 1,3-Dioxan-5 yl | |
| 4.180 | Me | Cl | S | 1,3-Dioxan-5 yl | |
| 4.181 | SMe | SO₂Me | S | 1,3-Dioxan-5 yl | |
| 4.182 | SMe | SO₂Et | S | 1,3-Dioxan-5 yl | |
| 4.183 | SMe | Cl | S | 1,3-Dioxan-5 yl | |
| 4.184 | SO₂Me | SO₂Me | S | 1,3-Dioxan-5 yl | |
| 4.185 | SO₂Me | SO₂Et | S | 1,3-Dioxan-5 yl | |
| 4.186 | SO₂Me | Cl | S | 1,3-Dioxan-5 yl | |
| 4.187 | NO₂ | SO₂Me | S | 1,3-Dioxan-5 yl | |
| 4.188 | NO₂ | SO₂Et | S | 1,3-Dioxan-5 yl | |
| 4.189 | NO₂ | Cl | S | 1,3-Dioxan-5 yl | |
| 4.190 | Cl | SO₂Me | S | γ-butyrolacton-2-yl | |
| 4.191 | Cl | SO₂Et | S | γ-butyrolacton-2-yl | |
| 4.192 | Cl | Cl | S | γ-butyrolacton-2-yl | |
| 4.193 | Br | SO₂Me | S | γ-butyrolacton-2-yl | |
| 4.194 | Br | SO₂Et | S | γ-butyrolacton-2-yl | |
| 4.195 | Br | Cl | S | γ-butyrolacton-2-yl | |
| 4.196 | I | SO₂Me | S | γ-butyrolacton-2-yl | |
| 4.197 | I | SO₂Et | S | γ-butyrolacton-2-yl | |
| 4.198 | I | Cl | S | γ-butyrolacton-2-yl | |
| 4.199 | Me | SO₂Me | S | γ-butyrolacton-2-yl | |
| 4.200 | Me | SO₂Et | S | γ-butyrolacton-2-yl | |
| 4.201 | Me | Cl | S | γ-butyrolacton-2-yl | |
| 4.202 | SMe | SO₂Me | S | γ-butyrolacton-2-yl | |
| 4.203 | SMe | SO₂Et | S | γ-butyrolacton-2-yl | |
| 4.204 | SMe | Cl | S | γ-butyrolacton-2-yl | |
| 4.205 | SO₂Me | SO₂Me | S | γ-butyrolacton-2-yl | |
| 4.206 | SO₂Me | SO₂Et | S | γ-butyrolacton-2-yl | |
| 4.207 | SO₂Me | Cl | S | γ-butyrolacton-2-yl | |
| 4.208 | NO₂ | SO₂Me | S | γ-butyrolacton-2-yl | |
| 4.209 | NO₂ | SO₂Et | S | γ-butyrolacton-2-yl | |
| 4.210 | NO₂ | Cl | S | γ-butyrolacton-2-yl | |

**Tabelle 5: Erfindungsgemäße Verbindungen der allgemeinen Formel (I), worin die Substituenten und Symbole folgende Bedeutungen haben:**

| | | | | | |
|---|---|---|---|---|---|
| | R³ = H | | Q = Q2 | | R⁶ = H |
| | R⁷ = Me | | R⁸ = H | | |
| 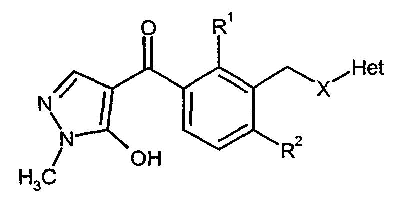 | | | | | |

| **Nr.** | **R¹** | **R²** | **X** | **Het** | **Physikal. Daten** |
|---|---|---|---|---|---|
| 5.1 | Cl | SO₂Me | O | 3-Tetrahydrofuranyl | |
| 5.2 | Cl | SO₂Et | O | 3-Tetrahydrofuranyl | |
| 5.3 | Cl | Cl | O | 3-Tetrahydrofuranyl | |
| 5.4 | Br | SO₂Me | O | 3-Tetrahydrofuranyl | |
| 5.5 | Br | SO₂Et | O | 3-Tetrahydrofuranyl | |
| 5.6 | Br | Cl | O | 3-Tetrahydrofuranyl | |
| 5.7 | I | SO₂Me | O | 3-Tetrahydrofuranyl | |
| 5.8 | I | SO₂Et | O | 3-Tetrahydrofuranyl | |
| 5.9 | I | Cl | O | 3-Tetrahydrofuranyl | |
| 5.10 | Me | SO₂Me | O | 3-Tetrahydrofuranyl | |
| 5.11 | Me | SO₂Et | O | 3-Tetrahydrofuranyl | |
| 5.12 | Me | Cl | O | 3-Tetrahydrofuranyl | |
| 5.13 | SMe | SO₂Me | O | 3-Tetrahydrofuranyl | |
| 5.14 | SMe | SO₂Et | O | 3-Tetrahydrofuranyl | |
| 5.15 | SMe | Cl | O | 3-Tetrahydrofuranyl | |
| 5.16 | SO₂Me | SO₂Me | O | 3-Tetrahydrofuranyl | |
| 5.17 | SO₂Me | SO₂Et | O | 3-Tetrahydrofuranyl | |
| 5.18 | SO₂Me | Cl | O | 3-Tetrahydrofuranyl | |
| 5.19 | NO₂ | SO₂Me | O | 3-Tetrahydrofuranyl | |
| 5.20 | NO₂ | SO₂Et | O | 3-Tetrahydrofuranyl | |
| 5.21 | NO₂ | Cl | O | 3-Tetrahydrofuranyl | |
| 5.22 | Cl | SO₂Me | O | 4-Tetrahydropyranyl | |
| 5.23 | Cl | SO₂Et | O | 4-Tetrahydropyranyl | |
| 5.24 | Cl | Cl | O | 4-Tetrahydropyranyl | |
| 5.25 | Br | SO₂Me | O | 4-Tetrahydropyranyl | |
| 5.26 | Br | SO₂Et | O | 4-Tetrahydropyranyl | |
| 5.27 | Br | Cl | O | 4-Tetrahydropyranyl | |
| 5.28 | I | SO₂Me | O | 4-Tetrahydropyranyl | |
| 5.29 | I | SO₂Et | O | 4-Tetrahydropyranyl | |
| 5.30 | I | Cl | O | 4-Tetrahydropyranyl | |
| 5.31 | Me | SO₂Me | O | 4-Tetrahydropyranyl | |
| 5.32 | Me | SO₂Et | O | 4-Tetrahydropyranyl | |
| 5.33 | Me | Cl | O | 4-Tetrahydropyranyl | |
| 5.34 | SMe | SO₂Me | O | 4-Tetrahydropyranyl | |
| 5.35 | SMe | SO₂Et | O | 4-Tetrahydropyranyl | |
| 5.36 | SMe | Cl | O | 4-Tetrahydropyranyl | |
| 5.37 | SO₂Me | SO₂Me | O | 4-Tetrahydropyranyl | |
| 5.38 | SO₂Me | SO₂Et | O | 4-Tetrahydropyranyl | |
| 5.39 | SO₂Me | Cl | O | 4-Tetrahydropyranyl | |
| 5.40 | NO₂ | SO₂Me | O | 4-Tetrahydropyranyl | |
| 5.41 | NO₂ | SO₂Et | O | 4-Tetrahydropyranyl | |
| 5.42 | NO₂ | Cl | O | 4-Tetrahydropyranyl | |
| 5.43 | Cl | SO₂Me | O | 3-Tetrahydropyranyl | |
| 5.44 | Cl | SO₂Et | O | 3-Tetrahydropyranyl | |
| 5.45 | Cl | Cl | O | 3-Tetrahydropyranyl | |
| 5.46 | Br | SO₂Me | O | 3-Tetrahydropyranyl | |
| 5.47 | Br | SO₂Et | O | 3-Tetrahydropyranyl | |
| 5.48 | Br | Cl | O | 3-Tetrahydropyranyl | |
| 5.49 | I | SO₂Me | O | 3-Tetrahydropyranyl | |
| 5.50 | I | O₂Et | O | 3-Tetrahydropyranyl | |
| 5.51 | I | Cl | O | 3-Tetrahydropyranyl | |
| 5.52 | Me | SO₂Me | O | 3-Tetrahydropyranyl | |
| 5.53 | Me | SO₂Et | O | 3-Tetrahydropyranyl | |
| 5.54 | Me | Cl | O | 3-Tetrahydropyranyl | |
| 5.55 | SMe | SO₂Me | O | 3-Tetrahydropyranyl | |
| 5.56 | SMe | SO₂Et | O | 3-Tetrahydropyranyl | |
| 5.57 | SMe | Cl | O | 3-Tetrahydropyranyl | |
| 5.58 | SO₂Me | SO₂Me | O | 3-Tetrahydropyranyl | |
| 5.59 | SO₂Me | SO₂Et | O | 3-Tetrahydropyranyl | |
| 5.60 | SO₂Me | Cl | O | 3-Tetrahydropyranyl | |
| 5.61 | NO₂ | SO₂Me | O | 3-Tetrahydropyranyl | |
| 5.62 | NO₂ | SO₂Et | O | 3-Tetrahydropyranyl | |
| 5.63 | NO₂ | Cl | O | 3-Tetrahydropyranyl | |
| 5.64 | Cl | SO₂Me | O | 1,3-Dioxan-5 yl | |
| 5.65 | Cl | SO₂Et | O | 1,3-Dioxan-5 yl | |
| 5.66 | Cl | Cl | O | 1,3-Dioxan-5 yl | |
| 5.67 | Br | SO₂Me | O | 1,3-Dioxan-5 yl | |
| 5.68 | Br | SO₂Et | O | 1,3-Dioxan-5 yl | |
| 5.69 | Br | Cl | O | 1,3-Dioxan-5 yl | |
| 5.70 | I | SO₂Me | O | 1,3-Dioxan-5 yl | |
| 5.71 | I | SO₂Et | O | 1,3-Dioxan-5 yl | |
| 5.72 | I | Cl | O | 1,3-Dioxan-5 yl | |
| 5.73 | Me | SO₂Me | O | 1,3-Dioxan-5 yl | |
| 5.74 | Me | SO₂Et | O | 1,3-Dioxan-5 yl | |
| 5.75 | Me | Cl | O | 1,3-Dioxan-5 yl | |
| 5.76 | SMe | SO₂Me | O | 1,3-Dioxan-5 yl | |
| 5.77 | SMe | SO₂Et | O | 1,3-Dioxan-5 yl | |
| 5.78 | SMe | Cl | O | 1,3-Dioxan-5 yl | |
| 5.79 | SO₂Me | SO₂Me | O | 1,3-Dioxan-5 yl | |
| 5.80 | SO₂Me | SO₂Et | O | 1,3-Dioxan-5 yl | |
| 5.81 | SO₂Me | Cl | O | 1,3-Dioxan-5 yl | |
| 5.82 | NO₂ | SO₂Me | O | 1,3-Dioxan-5 yl | |
| 5.83 | NO₂ | SO₂Et | O | 1,3-Dioxan-5 yl | |
| 5.84 | NO₂ | Cl | O | 1,3-Dioxan-5 yl | |
| 5.85 | Cl | SO₂Me | O | γ-butyrolacton-2-yl | |
| 5.86 | Cl | SO₂Et | O | γ-butyrolacton-2-yl | |
| 5.87 | Cl | Cl | O | γ-butyrolacton-2-yl | |
| 5.88 | Br | SO₂Me | O | γ-butyrolacton-2-yl | |
| 5.89 | Br | SO₂Et | O | γ-butyrolacton-2-yl | |
| 5.90 | Br | Cl | O | γ-butyrolacton-2-yl | |
| 5.91 | I | SO₂Me | O | γ-butyrolacton-2-yl | |
| 5.92 | I | SO₂Et | O | γ-butyrolacton-2-yl | |
| 5.93 | I | Cl | O | γ-butyrolacton-2-yl | |
| 5.94 | Me | SO₂Me | O | γ-butyrolacton-2-yl | |
| 5.95 | Me | SO₂Et | O | γ-butyrolacton-2-yl | |
| 5.96 | Me | Cl | O | γ-butyrolacton-2-yl | |
| 5.97 | SMe | SO₂Me | O | γ-butyrolacton-2-yl | |
| 5.98 | SMe | SO₂Et | O | γ-butyrolacton-2-yl | |
| 5.99 | SMe | Cl | O | γ-butyrolacton-2-yl | |
| 5.100 | SO₂Me | SO₂Me | O | γ-butyrolacton-2-yl | |
| 5.101 | SO₂Me | SO₂Et | O | γ-butyrolacton-2-yl | |
| 5.102 | SO₂Me | Cl | O | γ-butyrolacton-2-yl | |
| 5.103 | NO₂ | SO₂Me | O | γ-butyrolacton-2-yl | |
| 5.104 | NO₂ | SO₂Et | O | γ-butyrolacton-2-yl | |
| 5.105 | NO₂ | Cl | O | γ-butyrolacton-2-yl | |
| 5.106 | Cl | SO₂Me | S | 3-Tetrahydrofuranyl | |
| 5.107 | Cl | SO₂Et | S | 3-Tetrahydrofuranyl | |
| 5.108 | Cl | Cl | S | 3-Tetrahydrofuranyl | |
| 5.109 | Br | SO₂Me | S | 3-Tetrahydrofuranyl | |
| 5.110 | Br | SO₂Et | S | 3-Tetrahydrofuranyl | |
| 5.111 | Br | Cl | S | 3-Tetrahydrofuranyl | |
| 5.112 | I | SO₂Me | S | 3-Tetrahydrofuranyl | |
| 5.113 | I | SO₂Et | S | 3-Tetrahydrofuranyl | |
| 5.114 | I | Cl | S | 3-Tetrahydrofuranyl | |
| 5.115 | Me | SO₂Me | S | 3-Tetrahydrofuranyl | |
| 5.116 | Me | SO₂Et | S | 3-Tetrahydrofuranyl | |
| 5.117 | Me | Cl | S | 3-Tetrahydrofuranyl | |
| 5.118 | SMe | SO₂Me | S | 3-Tetrahydrofuranyl | |
| 5.119 | SMe | SO₂Et | S | 3-Tetrahydrofuranyl | |
| 5.120 | SMe | Cl | S | 3-Tetrahydrofuranyl | |
| 5.121 | SO₂Me | SO₂Me | S | 3-Tetrahydrofuranyl | |
| 5.122 | SO₂Me | SO₂Et | S | 3-Tetrahydrofuranyl | |
| 5.123 | SO₂Me | Cl | S | 3-Tetrahydrofuranyl | |
| 5.124 | NO₂ | SO₂Me | S | 3-Tetrahydrofuranyl | |
| 5.125 | NO₂ | SO₂Et | S | 3-Tetrahydrofuranyl | |
| 5.126 | NO₂ | Cl | S | 3-Tetrahydrofuranyl | |
| 5.127 | Cl | SO₂Me | S | 4-Tetrahydropyranyl | |
| 5.128 | Cl | SO₂Et | S | 4-Tetrahydropyranyl | |
| 5.129 | Cl | Cl | S | 4-Tetrahydropyranyl | |
| 5.130 | Br | SO₂Me | S | 4-Tetrahydropyranyl | |
| 5.131 | Br | SO₂Et | S | 4-Tetrahydropyranyl | |
| 5.132 | Br | Cl | S | 4-Tetrahydropyranyl | |
| 5.133 | I | SO₂Me | S | 4-Tetrahydropyranyl | |
| 5.134 | I | SO₂Et | S | 4-Tetrahydropyranyl | |
| 5.135 | I | Cl | S | 4-Tetrahydropyranyl | |
| 5.136 | Me | SO₂Me | S | 4-Tetrahydropyranyl | |
| 5.137 | Me | SO₂Et | S | 4-Tetrahydropyranyl | |
| 5.138 | Me | Cl | S | 4-Tetrahydropyranyl | |
| 5.139 | SMe | SO₂Me | S | 4-Tetrahydropyranyl | |
| 5.140 | SMe | SO₂Et | S | 4-Tetrahydropyranyl | |
| 5.141 | SMe | Cl | S | 4-Tetrahydropyranyl | |
| 5.142 | SO₂Me | SO₂Me | S | 4-Tetrahydropyranyl | |
| 5.143 | SO₂Me | SO₂Et | S | 4-Tetrahydropyranyl | |
| 5.144 | SO₂Me | Cl | S | 4-Tetrahydropyranyl | |
| 5.145 | NO₂ | SO₂Me | S | 4-Tetrahydropyranyl | |
| 5.146 | NO₂ | SO₂Et | S | 4-Tetrahydropyranyl | |
| 5.147 | NO₂ | Cl | S | 4-Tetrahydropyranyl | |
| 5.148 | Cl | SO₂Me | S | 3-Tetrahydropyranyl | |
| 5.149 | Cl | SO₂Et | S | 3-Tetrahydropyranyl | |
| 5.150 | Cl | Cl | S | 3-Tetrahydropyranyl | |
| 5.151 | Br | SO₂Me | S | 3-Tetrahydropyranyl | |
| 5.152 | Br | SO₂Et | S | 3-Tetrahydropyranyl | |
| 5.153 | Br | Cl | S | 3-Tetrahydropyranyl | |
| 5.154 | I | SO₂Me | S | 3-Tetrahydropyranyl | |
| 5.155 | I | SO₂Et | S | 3-Tetrahydropyranyl | |
| 5.156 | I | Cl | S | 3-Tetrahydropyranyl | |
| 5.157 | Me | SO₂Me | S | 3-Tetrahydropyranyl | |
| 5.158 | Me | SO₂Et | S | 3-Tetrahydropyranyl | |
| 5.159 | Me | Cl | S | 3-Tetrahydropyranyl | |
| 5.160 | SMe | SO₂Me | S | 3-Tetrahydropyranyl | |
| 5.161 | SMe | SO₂Et | S | 3-Tetrahydropyranyl | |
| 5.162 | SMe | Cl | S | 3-Tetrahydropyranyl | |
| 5.163 | SO₂Me | SO₂Me | S | 3-Tetrahydropyranyl | |
| 5.164 | SO₂Me | SO₂Et | S | 3-Tetrahydropyranyl | |
| 5.165 | SO₂Me | Cl | S | 3-Tetrahydropyranyl | |
| 5.166 | NO₂ | SO₂Me | S | 3-Tetrahydropyranyl | |
| 5.167 | NO₂ | SO₂Et | S | 3-Tetrahydropyranyl | |
| 5.168 | NO₂ | Cl | S | 3-Tetrahydropyranyl | |
| 5.169 | Cl | SO₂Me | S | 1,3-Dioxan-5 yl | |
| 5.170 | Cl | SO₂Et | S | 1,3-Dioxan-5 yl | |
| 5.171 | Cl | Cl | S | 1,3-Dioxan-5 yl | |
| 5.172 | Br | SO₂Me | S | 1,3-Dioxan-5 yl | |
| 5.173 | Br | SO₂Et | S | 1,3-Dioxan-5 yl | |
| 5.174 | Br | Cl | S | 1,3-Dioxan-5 yl | |
| 5.175 | I | SO₂Me | S | 1,3-Dioxan-5 yl | |
| 5.176 | I | SO₂Et | S | 1,3-Dioxan-5 yl | |
| 5.177 | I | Cl | S | 1,3-Dioxan-5 yl | |
| 5.178 | Me | SO₂Me | S | 1,3-Dioxan-5 yl | |
| 5.179 | Me | SO₂Et | S | 1,3-Dioxan-5 yl | |
| 5.180 | Me | Cl | S | 1,3-Dioxan-5 yl | |
| 5.181 | SMe | SO₂Me | S | 1,3-Dioxan-5 yl | |
| 5.182 | SMe | SO₂Et | S | 1,3-Dioxan-5 yl | |
| 5.183 | SMe | Cl | S | 1,3-Dioxan-5 yl | |
| 5.184 | SO₂Me | SO₂Me | S | 1,3-Dioxan-5 yl | |
| 5.185 | SO₂Me | SO₂Et | S | 1,3-Dioxan-5 yl | |
| 5.186 | SO₂Me | Cl | S | 1,3-Dioxan-5 yl | |
| 5.187 | NO₂ | SO₂Me | S | 1,3-Dioxan-5 yl | |
| 5.188 | NO₂ | SO₂Et | S | 1,3-Dioxan-5 yl | |
| 5.189 | NO₂ | Cl | S | 1,3-Dioxan-5 yl | |
| 5.190 | Cl | SO₂Me | S | γ-butyrolacton-2-yl | |
| 5.191 | Cl | SO₂Et | S | γ-butyrolacton-2-yl | |
| 5.192 | Cl | Cl | S | γ-butyrolacton-2-yl | |
| 5.193 | Br | SO₂Me | S | γ-butyrolacton-2-yl | |
| 5.194 | Br | SO₂Et | S | γ-butyrolacton-2-yl | |
| 5.195 | Br | Cl | S | γ-butyrolacton-2-yl | |
| 5.196 | I | SO₂Me | S | γ-butyrolacton-2-yl | |
| 5.197 | I | SO₂Et | S | γ-butyrolacton-2-yl | |
| 5.198 | I | Cl | S | γ-butyrolacton-2-yl | |
| 5.199 | Me | SO₂Me | S | γ-butyrolacton-2-yl | |
| 5.200 | Me | SO₂Et | S | γ-butyrolacton-2-yl | |
| 5.201 | Me | Cl | S | γ-butyrolacton-2-yl | |
| 5.202 | SMe | SO₂Me | S | γ-butyrolacton-2-yl | |
| 5.203 | SMe | SO₂Et | S | γ-butyrolacton-2-yl | |
| 5.204 | SMe | Cl | S | γ-butyrolacton-2-yl | |
| 5.205 | SO₂Me | SO₂Me | S | γ-butyrolacton-2-yl | |
| 5.206 | SO₂Me | SO₂Et | S | γ-butyrolacton-2-yl | |
| 5.207 | SO₂Me | Cl | S | γ-butyrolacton-2-yl | |
| 5.208 | NO₂ | SO₂Me | S | γ-butyrolacton-2-yl | |
| 5.209 | NO₂ | SO₂Et | S | γ-butyrolacton-2-yl | |
| 5.210 | NO₂ | Cl | S | γ-butyrolacton-2-yl | |

**Tabelle 6: Erfindungsgemäße Verbindungen der allgemeinen Formel (I), worin die Substituenten und Symbole folgende Bedeutungen haben:**

| | | | | | |
|---|---|---|---|---|---|
| | R³ = H | | Q = Q2 | | R⁶ = H |
| | R⁷ = Et | | R⁸ = H | | |
| 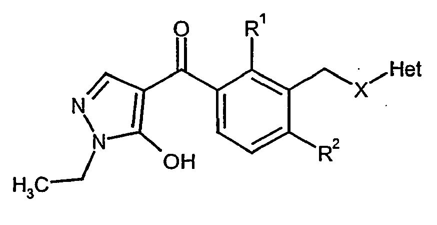 | | | | | |

| **Nr.** | **R¹** | **R²** | **X** | **Het** | **Physikal. Daten** |
|---|---|---|---|---|---|
| 6.1 | Cl | SO₂Me | O | 3-Tetrahydrofuranyl | |
| 6.2 | Cl | SO₂Et | O | 3-Tetrahydrofuranyl | |
| 6.3 | Cl | Cl | O | 3-Tetrahydrofuranyl | |
| 6.4 | Br | SO₂Me | O | 3-Tetrahydrofuranyl | |
| 6.5 | Br | SO₂Et | O | 3-Tetrahydrofuranyl | |
| 6.6 | Br | Cl | O | 3-Tetrahydrofuranyl | |
| 6.7 | I | SO₂Me | O | 3-Tetrahydrofuranyl | |
| 6.8 | I | SO₂Et | O | 3-Tetrahydrofuranyl | |
| 6.9 | I | Cl | O | 3-Tetrahydrofuranyl | |
| 6.10 | Me | SO₂Me | O | 3-Tetrahydrofuranyl | |
| 6.11 | Me | SO₂Et | O | 3-Tetrahydrofuranyl | |
| 6.12 | Me | Cl | O | 3-Tetrahydrofuranyl | |
| 6.13 | SMe | SO₂Me | O | 3-Tetrahydrofuranyl | |
| 6.14 | SMe | SO₂Et | O | 3-Tetrahydrofuranyl | |
| 6.15 | SMe | Cl | O | 3-Tetrahydrofuranyl | |
| 6.16 | SO₂Me | SO₂Me | O | 3-Tetrahydrofuranyl | |
| 6.17 | SO₂Me | SO₂Et | O | 3-Tetrahydrofuranyl | |
| 6.18 | SO₂Me | Cl | O | 3-Tetrahydrofuranyl | |
| 6.19 | NO₂ | SO₂Me | O | 3-Tetrahydrofuranyl | |
| 6.20 | NO₂ | SO₂Et | O | 3-Tetrahydrofuranyl | |
| 6.21 | NO₂ | Cl | O | 3-Tetrahydrofuranyl | |
| 6.22 | Cl | SO₂Me | O | 4-Tetrahydropyranyl | |
| 6.23 | Cl | SO₂Et | O | 4-Tetrahydropyranyl | |
| 6.24 | Cl | Cl | O | 4-Tetrahydropyranyl | |
| 6.25 | Br | SO₂Me | O | 4-Tetrahydropyranyl | |
| 6.26 | Br | SO₂Et | O | 4-Tetrahydropyranyl | |
| 6.27 | Br | Cl | O | 4-Tetrahydropyranyl | |
| 6.28 | I | SO₂Me | O | 4-Tetrahydropyranyl | |
| 6.29 | I | SO₂Et | O | 4-Tetrahydropyranyl | |
| 6.30 | I | Cl | O | 4-Tetrahydropyranyl | |
| 6.31 | Me | SO₂Me | O | 4-Tetrahydropyranyl | |
| 6.32 | Me | SO₂Et | O | 4-Tetrahydropyranyl | |
| 6.33 | Me | Cl | O | 4-Tetrahydropyranyl | |
| 6.34 | SMe | SO₂Me | O | 4-Tetrahydropyranyl | |
| 6.35 | SMe | SO₂Et | O | 4-Tetrahydropyranyl | |
| 6.36 | SMe | Cl | O | 4-Tetrahydropyranyl | |
| 6.37 | SO₂Me | SO₂Me | O | 4-Tetrahydropyranyl | |
| 6.38 | SO₂Me | SO₂Et | O | 4-Tetrahydropyranyl | |
| 6.39 | SO₂Me | Cl | O | 4-Tetrahydropyranyl | |
| 6.40 | NO₂ | SO₂Me | O | 4-Tetrahydropyranyl | |
| 6.41 | NO₂ | SO₂Et | O | 4-Tetrahydropyranyl | |
| 6.42 | NO₂ | Cl | O | 4-Tetrahydropyranyl | |
| 6.43 | Cl | SO₂Me | O | 3-Tetrahydropyranyl | |
| 6.44 | Cl | SO₂Et | O | 3-Tetrahydropyranyl | |
| 6.45 | Cl | Cl | O | 3-Tetrahydropyranyl | |
| 6.46 | Br | SO₂Me | O | 3-Tetrahydropyranyl | |
| 6.47 | Br | SO₂Et | O | 3-Tetrahydropyranyl | |
| 6.48 | Br | Cl | O | 3-Tetrahydropyranyl | |
| 6.49 | I | SO₂Me | O | 3-Tetrahydropyranyl | |
| 6.50 | I | SO₂Et | O | 3-Tetrahydropyranyl | |
| 6.51 | I | Cl | O | 3-Tetrahydropyranyl | |
| 6.52 | Me | SO₂Me | O | 3-Tetrahydropyranyl | |
| 6.53 | Me | SO₂Et | O | 3-Tetrahydropyranyl | |
| 6.54 | Me | Cl | O | 3-Tetrahydropyranyl | |
| 6.55 | SMe | SO₂Me | O | 3-Tetrahydropyranyl | |
| 6.56 | SMe | SO₂Et | O | 3-Tetrahydropyranyl | |
| 6.57 | SMe | Cl | O | 3-Tetrahydropyranyl | |
| 6.58 | SO₂Me | SO₂Me | O | 3-Tetrahydropyranyl | |
| 6.59 | SO₂Me | SO₂Et | O | 3-Tetrahydropyranyl | |
| 6.60 | SO₂Me | Cl | O | 3-Tetrahydropyranyl | |
| 6.61 | NO₂ | SO₂Me | O | 3-Tetrahydropyranyl | |
| 6.62 | NO₂ | SO₂Et | O | 3-Tetrahydropyranyl | |
| 6.63 | NO₂ | Cl | O | 3-Tetrahydropyranyl | |
| 6.64 | Cl | SO₂Me | O | 1,3-Dioxan-5 yl | |
| 6.65 | Cl | SO₂Et | O | 1,3-Dioxan-5 yl | |
| 6.66 | Cl | Cl | O | 1,3-Dioxan-5 yl | |
| 6.67 | Br | SO₂Me | O | 1,3-Dioxan-5 yl | |
| 6.68 | Br | SO₂Et | O | 1,3-Dioxan-5 yl | |
| 6.69 | Br | Cl | O | 1,3-Dioxan-5 yl | |
| 6.70 | I | SO₂Me | O | 1,3-Dioxan-5 yl | |
| 6.71 | I | SO₂Et | O | 1,3-Dioxan-5 yl | |
| 6.72 | I | Cl | O | 1,3-Dioxan-5 yl | |
| 6.73 | Me | SO₂Me | O | 1,3-Dioxan-5 yl | |
| 6.74 | Me | SO₂Et | O | 1,3-Dioxan-5 yl | |
| 6.75 | Me | Cl | O | 1,3-Dioxan-5 yl | |
| 6.76 | SMe | SO₂Me | O | 1,3-Dioxan-5 yl | |
| 6.77 | SMe | SO₂Et | O | 1,3-Dioxan-5 yl | |
| 6.78 | SMe | Cl | O | 1,3-Dioxan-5 yl | |
| 6.79 | SO₂Me | SO₂Me | O | 1,3-Dioxan-5 yl | |
| 6.80 | SO₂Me | SO₂Et | O | 1,3-Dioxan-5 yl | |
| 6.81 | SO₂Me | Cl | O | 1,3-Dioxan-5 yl | |
| 6.82 | NO₂ | SO₂Me | O | 1,3-Dioxan-5 yl | |
| 6.83 | NO₂ | SO₂Et | O | 1,3-Dioxan-5 yl | |
| 6.84 | NO₂ | Cl | O | 1,3-Dioxan-5 yl | |
| 6.85 | Cl | SO₂Me | O | γ-butyrolacton-2-yl | |
| 6.86 | Cl | SO₂Et | O | γ-butyrolacton-2-yl | |
| 6.87 | Cl | Cl | O | γ-butyrolacton-2-yl | |
| 6.88 | Br | SO₂Me | O | γ-butyrolacton-2-yl | |
| 6.89 | Br | SO₂Et | O | γ-butyrolacton-2-yl | |
| 6.90 | Br | Cl | O | γ-butyrolacton-2-yl | |
| 6.91 | I | SO₂Me | O | γ-butyrolacton-2-yl | |
| 6.92 | I | SO₂Et | O | γ-butyrolacton-2-yl | |
| 6.93 | I | Cl | O | γ-butyrolacton-2-yl | |
| 6.94 | Me | SO₂Me | O | γ-butyrolacton-2-yl | |
| 6.95 | Me | SO₂Et | O | γ-butyrolacton-2-yl | |
| 6.96 | Me | Cl | O | γ-butyrolacton-2-yl | |
| 6.97 | SMe | SO₂Me | O | γ-butyrolacton-2-yl | |
| 6.98 | SMe | SO₂Et | O | γ-butyrolacton-2-yl | |
| 6.99 | SMe | Cl | O | γ-butyrolacton-2-yl | |
| 6.100 | SO₂Me | SO₂Me | O | γ-butyrolacton-2-yl | |
| 6.101 | SO₂Me | SO₂Et | O | γ-butyrolacton-2-yl | |
| 6.102 | SO₂Me | Cl | O | γ-butyrolacton-2-yl | |
| 6.103 | NO₂ | SO₂Me | O | γ-butyrolacton-2-yl | |
| 6.104 | NO₂ | S_{O}2Et | O | γ-butyrolacton-2-yl | |
| 6.105 | NO₂ | Cl | O | γ-butyrolacton-2-yl | |
| 6.106 | Cl | SO₂Me | S | 3-Tetrahydrofuranyl | |
| 6.107 | Cl | SO₂Et | S | 3-Tetrahydrofuranyl | |
| 6.108 | Cl | Cl | S | 3-Tetrahydrofuranyl | |
| 6.109 | Br | SO₂Me | S | 3-Tetrahydrofuranyl | |
| 6.110 | Br | SO₂Et | S | 3-Tetrahydrofuranyl | |
| 6.111 | Br | Cl | S | 3-Tetrahydrofuranyl | |
| 6.112 | I | SO₂Me | S | 3-Tetrahydrofuranyl | |
| 6.113 | I | SO₂Et | S | 3-Tetrahydrofuranyl | |
| 6.114 | I | Cl | S | 3-Tetrahydrofuranyl | |
| 6.115 | Me | SO₂Me | S | 3-Tetrahydrofuranyl | |
| 6.116 | Me | SO₂Et | S | 3-Tetrahydrofuranyl | |
| 6.117 | Me | Cl | S | 3-Tetrahydrofuranyl | |
| 6.118 | SMe | SO₂Me | S | 3-Tetrahydrofuranyl | |
| 6.119 | SMe | SO₂Et | S | 3-Tetrahydrofuranyl | |
| 6.120 | SMe | Cl | S | 3-Tetrahydrofuranyl | |
| 6.121 | SO₂Me | SO₂Me | S | 3-Tetrahydrofuranyl | |
| 6.122 | SO₂Me | SO₂Et | S | 3-Tetrahydrofuranyl | |
| 6.123 | SO₂Me | Cl | S | 3-Tetrahydrofuranyl | |
| 6.124 | NO₂ | SO₂Me | S | 3-Tetrahydrofuranyl | |
| 6.125 | NO₂ | SO₂Et | S | 3-Tetrahydrofuranyl | |
| 6.126 | NO₂ | Cl | S | 3-Tetrahydrofuranyl | |
| 6.127 | Cl | SO₂Me | S | 4-Tetrahydropyranyl | |
| 6.128 | Cl | SO₂Et | S | 4-Tetrahydropyranyl | |
| 6.129 | Cl | Cl | S | 4-Tetrahydropyranyl | |
| 6.130 | Br | SO₂Me | S | 4-Tetrahydropyranyl | |
| 6.131 | Br | SO₂Et | S | 4-Tetrahydropyranyl | |
| 6.132 | Br | Cl | S | 4-Tetrahydropyranyl | |
| 6.133 | I | SO₂Me | S | 4-Tetrahydropyranyl | |
| 6.134 | I | SO₂Et | S | 4-Tetrahydropyranyl | |
| 6.135 | I | Cl | S | 4-Tetrahydropyranyl | |
| 6.136 | Me | SO₂Me | S | 4-Tetrahydropyranyl | |
| 6.137 | Me | SO₂Et | S | 4-Tetrahydropyranyl | |
| 6.138 | Me | Cl | S | 4-Tetrahydropyranyl | |
| 6.139 | SMe | SO₂Me | S | 4-Tetrahydropyranyl | |
| 6.140 | SMe | SO₂Et | S | 4-Tetrahydropyranyl | |
| 6.141 | SMe | Cl | S | 4-Tetrahydropyranyl | |
| 6.142 | SO₂Me | SO₂Me | S | 4-Tetrahydropyranyl | |
| 6.143 | SO₂Me | SO₂Et | S | 4-Tetrahydropyranyl | |
| 6.144 | SO₂Me | Cl | S | 4-Tetrahydropyranyl | |
| 6.145 | NO₂ | SO₂Me | S | 4-Tetrahydropyranyl | |
| 6.146 | NO₂ | SO₂Et | S | 4-Tetrahydropyranyl | |
| 6.147 | NO₂ | Cl | S | 4-Tetrahydropyranyl | |
| 6.148 | Cl | SO₂Me | S | 3-Tetrahydropyranyl | |
| 6.149 | Cl | SO₂Et | S | 3-Tetrahydropyranyl | |
| 6.150 | Cl | Cl | S | 3-Tetrahydropyranyl | |
| 6.151 | Br | SO₂Me | S | 3-Tetrahydropyranyl | |
| 6.152 | Br | SO₂Et | S | 3-Tetrahydropyranyl | |
| 6.153 | Br | Cl | S | 3-Tetrahydropyranyl | |
| 6.154 | I | SO₂Me | S | 3-Tetrahydropyranyl | |
| 6.155 | I | SO₂Et | S | 3-Tetrahydropyranyl | |
| 6.156 | I | Cl | S | 3-Tetrahydropyranyl | |
| 6.157 | Me | SO₂Me | S | 3-Tetrahydropyranyl | |
| 6.158 | Me | SO₂Et | S | 3-Tetrahydropyranyl | |
| 6.159 | Me | Cl | S | 3-Tetrahydropyranyl | |
| 6.160 | SMe | SO₂Me | S | 3-Tetrahydropyranyl | |
| 6.161 | SMe | SO₂Et | S | 3-Tetrahydropyranyl | |
| 6.162 | SMe | Cl | S | 3-Tetrahydropyranyl | |
| 6.163 | SO₂Me | SO₂Me | S | 3-Tetrahydropyranyl | |
| 6.164 | SO₂Me | SO₂Et | S | 3-Tetrahydropyranyl | |
| 6.165 | SO₂Me | Cl | S | 3-Tetrahydropyranyl | |
| 6.166 | NO₂ | SO₂Me | S | 3-Tetrahydropyranyl | |
| 6.167 | NO₂ | SO₂Et | S | 3-Tetrahydropyranyl | |
| 6.168 | NO₂ | Cl | S | 3-Tetrahydropyranyl | |
| 6.169 | Cl | SO₂Me | S | 1,3-Dioxan-5 yl | |
| 6.170 | Cl | SO₂Et | S | 1,3-Dioxan-5 yl | |
| 6.171 | Cl | Cl | S | 1,3-Dioxan-5 yl | |
| 6.172 | Br | SO₂Me | S | 1,3-Dioxan-5 yl | |
| 6.173 | Br | SO₂Et | S | 1,3-Dioxan-5 yl | |
| 6.174 | Br | Cl | S | 1,3-Dioxan-5 yl | |
| 6.175 | I | SO₂Me | S | 1,3-Dioxan-5 yl | |
| 6.176 | I | SO₂Et | S | 1,3-Dioxan-5 yl | |
| 6.177 | I | Cl | S | 1,3-Dioxan-5 yl | |
| 6.178 | Me | SO₂Me | S | 1,3-Dioxan-5 yl | |
| 6.179 | Me | SO₂Et | S | 1,3-Dioxan-5 yl | |
| 6.180 | Me | Cl | S | 1,3-Dioxan-5 yl | |
| 6.181 | SMe | SO₂Me | S | 1,3-Dioxan-5 yl | |
| 6.182 | SMe | SO₂Et | S | 1,3-Dioxan-5 yl | |
| 6.183 | SMe | Cl | S | 1,3-Dioxan-5 yl | |
| 6.184 | SO₂Me | SO₂Me | S | 1,3-Dioxan-5 yl | |
| 6.185 | SO₂Me | SO₂Et | S | 1,3-Dioxan-5 yl | |
| 6.186 | SO₂Me | Cl | S | 1,3-Dioxan-5 yl | |
| 6.187 | NO₂ | SO₂Me | S | 1,3-Dioxan-5 yl | |
| 6.188 | NO₂ | SO₂Et | S | 1,3-Dioxan-5 yl | |
| 6.189 | NO₂ | Cl | S | 1,3-Dioxan-5 yl | |
| 6.190 | Cl | SO₂Me | S | γ-butyrolacton-2-yl | |
| 6.191 | Cl | SO₂Et | S | γ-butyrolacton-2-yl | |
| 6.192 | Cl | Cl | S | γ-butyrolacton-2-yl | |
| 6.193 | Br | SO₂Me | S | γ-butyrolacton-2-yl | |
| 6.194 | Br | SO₂Et | S | γ-butyrolacton-2-yl | |
| 6.195 | Br | Cl | S | γ-butyrolacton-2-yl | |
| 6.196 | I | SO₂Me | S | γ-butyrolacton-2-yl | |
| 6.197 | I | SO₂Et | S | γ-butyrolacton-2-yl | |
| 6.198 | I | Cl | S | γ-butyrolacton-2-yl | |
| 6.199 | Me | SO₂Me | S | γ-butyrolacton-2-yl | |
| 6.200 | Me | SO₂Et | S | γ-butyrolacton-2-yl | |
| 6.201 | Me | Cl | S | γ-butyrolacton-2-yl | |
| 6.202 | SMe | SO₂Me | S | γ-butyrolacton-2-yl | |
| 6.203 | SMe | SO₂Et | S | γ-butyrolacton-2-yl | |
| 6.204 | SMe | Cl | S | γ-butyrolacton-2-yl | |
| 6.205 | SO₂Me | SO₂Me | S | γ-butyrolacton-2-yl | |
| 6.206 | SO₂Me | SO₂Et | S | γ-butyrolacton-2-yl | |
| 6.207 | SO₂Me | Cl | S | γ-butyrolacton-2-yl | |
| 6.208 | NO₂ | SO₂Me | S | γ-butyrolacton-2-yl | |
| 6.209 | NO₂ | SO₂Et | S | γ-butyrolacton-2-yl | |
| 6.210 | NO₂ | Cl | S | γ-butyrolacton-2-yl | |

**Tabelle 7: Erfindungsgemäße Verbindungen der allgemeinen Formel (I), worin die Substituenten und Symbole folgende Bedeutungen haben:**

| | | | | | |
|---|---|---|---|---|---|
| | R³ = H | | Q = Q2 | | R⁷ = Me |
| | R⁸ = H | | R⁶ = Cyclopropyl | | |
| 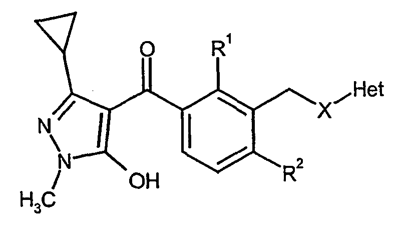 | | | | | |

| **Nr** | **R¹** | **R²** | **X** | **Het** | **Physikal. Daten** |
|---|---|---|---|---|---|
| 7.1 | Cl | SO₂Me | O | 3-Tetrahydrofuranyl | |
| 7.2 | Cl | SO₂Et | O | 3-Tetrahydrofuranyl | |
| 7.3 | Cl | Cl | O | 3-Tetrahydrofuranyl | |
| 7.4 | Br | SO₂Me | O | 3-Tetrahydrofuranyl | |
| 7.5 | Br | SO₂Et | O | 3-Tetrahydrofuranyl | |
| 7.6 | Br | Cl | O | 3-Tetrahydrofuranyl | |
| 7.7 | I | SO₂Me | O | 3-Tetrahydrofuranyl | |
| 7.8 | I | SO₂Et | O | 3-Tetrahydrofuranyl | |
| 7.9 | I | Cl | O | 3-Tetrahydrofuranyl | |
| 7.10 | Me | SO₂Me | O | 3-Tetrahydrofuranyl | |
| 7.11 | Me | SO₂Et | O | 3-Tetrahydrofuranyl | |
| 7.12 | Me | Cl | O | 3-Tetrahydrofuranyl | |
| 7.13 | SMe | SO₂Me | O | 3-Tetrahydrofuranyl | |
| 7.14 | SMe | SO₂Et | O | 3-Tetrahydrofuranyl | |
| 7.15 | SMe | Cl | O | 3-Tetrahydrofuranyl | |
| 7.16 | SO₂Me | SO₂Me | O | 3-Tetrahydrofuranyl | |
| 7.17 | SO₂Me | SO₂Et | O | 3-Tetrahydrofuranyl | |
| 7.18 | SO₂Me | Cl | O | 3-Tetrahydrofuranyl | |
| 7.19 | NO₂ | SO₂Me | O | 3-Tetrahydrofuranyl | |
| 7.20 | NO₂ | SO₂Et | O | 3-Tetrahydrofuranyl | |
| 7.21 | NO₂ | Cl | O | 3-Tetrahydrofuranyl | |
| 7.22 | Cl | SO₂Me | O | 4-Tetrahydropyranyl | |
| 7.23 | Cl | SO₂Et | O | 4-Tetrahydropyranyl | |
| 7.24 | Cl | Cl | O | 4-Tetrahydropyranyl | |
| 7.25 | Br | SO₂Me | O | 4-Tetrahydropyranyl | |
| 7.26 | Br | SO₂Et | O | 4-Tetrahydropyranyl | |
| 7.27 | Br | Cl | O | 4-Tetrahydropyranyl | |
| 7.28 | I | SO₂Me | O | 4-Tetrahydropyranyl | |
| 7.29 | I | SO₂Et | O | 4-Tetrahydropyranyl | |
| 7.30 | I | Cl | O | 4-Tetrahydropyranyl | |
| 7.31 | Me | SO₂Me | O | 4-Tetrahydropyranyl | |
| 7.32 | Me | SO₂Et | O | 4-Tetrahydropyranyl | |
| 7.33 | Me | Cl | O | 4-Tetrahydropyranyl | |
| 7.34 | SMe | SO₂Me | O | 4-Tetrahydropyranyl | |
| 7.35 | SMe | SO₂Et | O | 4-Tetrahydropyranyl | |
| 7.36 | SMe | Cl | O | 4-Tetrahydropyranyl | |
| 7.37 | SO₂Me | SO₂Me | O | 4-Tetrahydropyranyl | |
| 7.38 | SO₂Me | SO₂Et | O | 4-Tetrahydropyranyl | |
| 7.39 | SO₂Me | Cl | O | 4-Tetrahydropyranyl | |
| 7.40 | NO₂ | SO₂Me | O | 4-Tetrahydropyranyl | |
| 7.41 | NO₂ | SO₂Et | O | 4-Tetrahydropyranyl | |
| 7.42 | NO₂ | Cl | O | 4-Tetrahydropyranyl | |
| 7.43 | Cl | SO₂Me | O | 3-Tetrahydropyranyl | |
| 7.44 | Cl | SO₂Et | O | 3-Tetrahydropyranyl | |
| 7.45 | Cl | Cl | O | 3-Tetrahydropyranyl | |
| 7.46 | Br | SO₂Me | O | 3-Tetrahydropyranyl | |
| 7.47 | Br | SO₂Et | O | 3-Tetrahydropyranyl | |
| 7.48 | Br | Cl | O | 3-Tetrahydropyranyl | |
| 7.49 | I | SO₂Me | O | 3-Tetrahydropyranyl | |
| 7.50 | I | SO₂Et | O | 3-Tetrahydropyranyl | |
| 7.51 | I | Cl | O | 3-Tetrahydropyranyl | |
| 7.52 | Me | SO₂Me | O | 3-Tetrahydropyranyl | |
| 7.53 | Me | SO₂Et | O | 3-Tetrahydropyranyl | |
| 7.54 | Me | Cl | O | 3-Tetrahydropyranyl | |
| 7.55 | SMe | SO₂Me | O | 3-Tetrahydropyranyl | |
| 7.56 | SMe | SO₂Et | O | 3-Tetrahydropyranyl | |
| 7.57 | SMe | Cl | O | 3-Tetrahydropyranyl | |
| 7.58 | SO₂Me | SO₂Me | O | 3-Tetrahydropyranyl | |
| 7.59 | SO₂Me | SO₂Et | O | 3-Tetrahydropyranyl | |
| 7.60 | SO₂Me | Cl | O | 3-Tetrahydropyranyl | |
| 7.61 | NO₂ | SO₂Me | O | 3-Tetrahydropyranyl | |
| 7.62 | NO₂ | SO₂Et | O | 3-Tetrahydropyranyl | |
| 7.63 | NO₂ | Cl | O | 3-Tetrahydropyranyl | |
| 7.64 | Cl | SO₂Me | O | 1,3-Dioxan-5 yl | |
| 7.65 | Cl | SO₂Et | O | 1,3-Dioxan-5 yl | |
| 7.66 | Cl | Cl | O | 1,3-Dioxan-5 yl | |
| 7.67 | Br | SO₂Me | O | 1,3-Dioxan-5 yl | |
| 7.68 | Br | SO₂Et | O | 1,3-Dioxan-5 yl | |
| 7.69 | Br | Cl | O | 1,3-Dioxan-5 yl | |
| 7.70 | I | SO₂Me | O | 1,3-Dioxan-5 yl | |
| 7.71 | I | SO₂Et | O | 1,3-Dioxan-5 yl | |
| 7.72 | I | Cl | O | 1,3-Dioxan-5 yl | |
| 7.73 | Me | SO₂Me | O | 1,3-Dioxan-5 yl | |
| 7.74 | Me | SO₂Et | O | 1,3-Dioxan-5 yl | |
| 7.75 | Me | Cl | O | 1,3-Dioxan-5 yl | |
| 7.76 | SMe | SO₂Me | O | 1,3-Dioxan-5 yl | |
| 7.77 | SMe | SO₂Et | O | 1,3-Dioxan-5 yl | |
| 7.78 | SMe | Cl | O | 1,3-Dioxan-5 yl | |
| 7.79 | SO₂Me | SO₂Me | O | 1,3-Dioxan-5 yl | |
| 7.80 | SO₂Me | SO₂Et | O | 1,3-Dioxan-5 yl | |
| 7.81 | SO₂Me | Cl | O | 1,3-Dioxan-5 yl | |
| 7.82 | NO₂ | SO₂Me | O | 1,3-Dioxan-5 yl | |
| 7.83 | NO₂ | SO₂Et | O | 1,3-Dioxan-5 yl | |
| 7.84 | NO₂ | Cl | O | 1,3-Dioxan-5 yl | |
| 7.85 | Cl | SO₂Me | O | γ-butyrolacton-2-yl | |
| 7.86 | Cl | SO₂Et | O | γ-butyrolacton-2-yl | |
| 7.87 | Cl | Cl | O | γ-butyrolacton-2-yl | |
| 7.88 | Br | SO₂Me | O | γ-butyrolacton-2-yl | |
| 7.89 | Br | SO₂Et | O | γ-butyrolacton-2-yl | |
| 7.90 | Br | Cl | O | γ-butyrolacton-2-yl | |
| 7.91 | I | SO₂Me | O | γ-butyrolacton-2-yl | |
| 7.92 | I | SO₂Et | O | γ-butyrolacton-2-yl | |
| 7.93 | I | Cl | O | γ-butyrolacton-2-yl | |
| 7.94 | Me | SO₂Me | O | γ-butyrolacton-2-yl | |
| 7.95 | Me | SO₂Et | O | γ-butyrolacton-2-yl | |
| 7.96 | Me | Cl | O | γ-butyrolacton-2-yl | |
| 7.97 | SMe | SO₂Me | O | γ-butyrolacton-2-yl | |
| 7.98 | SMe | SO₂Et | O | γ-butyrolacton-2-yl | |
| 7.99 | SMe | Cl | O | γ-butyrolacton-2-yl | |
| 7.100 | SO₂Me | SO₂Me | O | γ-butyrolacton-2-yl | |
| 7.101 | SO₂Me | SO₂Et | O | γ-butyrolacton-2-yl | |
| 7.102 | SO₂Me | Cl | O | γ-butyrolacton-2-yl | |
| 7.103 | NO₂ | SO₂Me | O | γ-butyrolacton-2-yl | |
| 7.104 | NO₂ | SO₂Et | O | γ-butyrolacton-2-yl | |
| 7.105 | NO₂ | Cl | O | γ-butyrolacton-2-yl | |
| 7.106 | Cl | SO₂Me | S | 3-Tetrahydrofuranyl | |
| 7.107 | Cl | SO₂Et | S | 3-Tetrahydrofuranyl | |
| 7.108 | Cl | Cl | S | 3-Tetrahydrofuranyl | |
| 7.109 | Br | SO₂Me | S | 3-Tetrahydrofuranyl | |
| 7.110 | Br | SO₂Et | S | 3-Tetrahydrofuranyl | |
| 7.111 | Br | Cl | S | 3-Tetrahydrofuranyl | |
| 7.112 | I | SO₂Me | S | 3-Tetrahydrofuranyl | |
| 7.113 | I | SO₂Et | S | 3-Tetrahydrofuranyl | |
| 7.114 | I | Cl | S | 3-Tetrahydrofuranyl | |
| 7.115 | Me | SO₂Me | S | 3-Tetrahydrofuranyl | |
| 7.116 | Me | SO₂Et | S | 3-Tetrahydrofuranyl | |
| 7.117 | Me | Cl | S | 3-Tetrahydrofuranyl | |
| 7.118 | SMe | SO₂Me | S | 3-Tetrahydrofuranyl | |
| 7.119 | SMe | SO₂Et | S | 3-Tetrahydrofuranyl | |
| 7.120 | SMe | Cl | S | 3-Tetrahydrofuranyl | |
| 7.121 | SO₂Me | SO₂Me | S | 3-Tetrahydrofuranyl | |
| 7.122 | SO₂Me | SO₂Et | S | 3-Tetrahydrofuranyl | |
| 7.123 | SO₂Me | Cl | S | 3-Tetrahydrofuranyl | |
| 7.124 | NO₂ | SO₂Me | S | 3-Tetrahydrofuranyl | |
| 7.125 | NO₂ | SO₂Et | S | 3-Tetrahydrofuranyl | |
| 7.126 | NO₂ | Cl | S | 3-Tetrahydrofuranyl | |
| 7.127 | Cl | SO₂Me | S | 4-Tetrahydropyranyl | |
| 7.128 | Cl | SO₂Et | S | 4-Tetrahydropyranyl | |
| 7.129 | Cl | Cl | S | 4-Tetrahydropyranyl | |
| 7.130 | Br | SO₂Me | S | 4-Tetrahydropyranyl | |
| 7.131 | Br | SO₂Et | S | 4-Tetrahydropyranyl | |
| 7.132 | Br | Cl | S | 4-Tetrahydropyranyl | |
| 7.133 | I | SO₂Me | S | 4-Tetrahydropyranyl | |
| 7.134 | I | SO₂Et | S | 4-Tetrahydropyranyl | |
| 7.135 | I | Cl | S | 4-Tetrahydropyranyl | |
| 7.136 | Me | SO₂Me | S | 4-Tetrahydropyranyl | |
| 7.137 | Me | SO₂Et | S | 4-Tetrahydropyranyl | |
| 7.138 | Me | Cl | S | 4-Tetrahydropyranyl | |
| 7.139 | SMe | SO₂Me | S | 4-Tetrahydropyranyl | |
| 7.140 | SMe | SO₂Et | S | 4-Tetrahydropyranyl | |
| 7.141 | SMe | Cl | S | 4-Tetrahydropyranyl | |
| 7.142 | SO₂Me | SO₂Me | S | 4-Tetrahydropyranyl | |
| 7.143 | SO₂Me | SO₂Et | S | 4-Tetrahydropyranyl | |
| 7.144 | SO₂Me | Cl | S | 4-Tetrahydropyranyl | |
| 7.145 | NO₂ | SO₂Me | S | 4-Tetrahydropyranyl | |
| 7.146 | NO₂ | SO₂Et | S | 4-Tetrahydropyranyl | |
| 7.147 | NO₂ | Cl | S | 4-Tetrahydropyranyl | |
| 7.148 | Cl | SO₂Me | S | 3-Tetrahydropyranyl | |
| 7.149 | Cl | SO₂Et | S | 3-Tetrahydropyranyl | |
| 7.150 | Cl | Cl | S | 3-Tetrahydropyranyl | |
| 7.151 | Br | SO₂Me | S | 3-Tetrahydropyranyl | |
| 7.152 | Br | SO₂Et | S | 3-Tetrahydropyranyl | |
| 7.153 | Br | Cl | S | 3-Tetrahydropyranyl | |
| 7.154 | I | SO₂Me | S | 3-Tetrahydropyranyl | |
| 7.155 | I | SO₂Et | S | 3-Tetrahydropyranyl | |
| 7.156 | I | Cl | S | 3-Tetrahydropyranyl | |
| 7.157 | Me | SO₂Me | S | 3-Tetrahydropyranyl | |
| 7.158 | Me | SO₂Et | S | 3-Tetrahydropyranyl | |
| 7.159 | Me | Cl | S | 3-Tetrahydropyranyl | |
| 7.160 | SMe | SO₂Me | S | 3-Tetrahydropyranyl | |
| 7.161 | SMe | SO₂Et | S | 3-Tetrahydropyranyl | |
| 7.162 | SMe | Cl | S | 3-Tetrahydropyranyl | |
| 7.163 | SO₂Me | SO₂Me | S | 3-Tetrahydropyranyl | |
| 7.164 | SO₂Me | SO₂Et | S | 3-Tetrahydropyranyl | |
| 7.165 | SO₂Me | Cl | S | 3-Tetrahydropyranyl | |
| 7.166 | NO₂ | SO₂Me | S | 3-Tetrahydropyranyl | |
| 7.167 | NO₂ | SO₂Et | S | 3-Tetrahydropyranyl | |
| 7.168 | NO₂ | Cl | S | 3-Tetrahydropyranyl | |
| 7.169 | Cl | SO₂Me | S | 1,3-Dioxan-5 yl | |
| 7.170 | Cl | SO₂Et | S | 1,3-Dioxan-5 yl | |
| 7.171 | Cl | Cl | S | 1,3-Dioxan-5 yl | |
| 7.172 | Br | SO₂Me | S | 1,3-Dioxan-5 yl | |
| 7.173 | Br | SO₂Et | S | 1,3-Dioxan-5 yl | |
| 7.174 | Br | Cl | S | 1,3-Dioxan-5 yl | |
| 7.175 | I | SO₂Me | S | 1,3-Dioxan-5 yl | |
| 7.176 | I | SO₂Et | S | 1,3-Dioxan-5 yl | |
| 7.177 | I | Cl | S | 1,3-Dioxan-5 yl | |
| 7.178 | Me | SO₂Me | S | 1,3-Dioxan-5 yl | |
| 7.179 | Me | SO₂Et | S | 1,3-Dioxan-5 yl | |
| 7.180 | Me | Cl | S | 1,3-Dioxan-5 yl | |
| 7.181 | SMe | SO₂Me | S | 1,3-Dioxan-5 yl | |
| 7.182 | SMe | SO₂Et | S | 1,3-Dioxan-5 yl | |
| 7.183 | SMe | Cl | S | 1,3-Dioxan-5 yl | |
| 7.184 | SO₂Me | SO₂Me | S | 1,3-Dioxan-5 yl | |
| 7.185 | SO₂Me | SO₂Et | S | 1,3-Dioxan-5 yl | |
| 7.186 | SO₂Me | Cl | S | 1,3-Dioxan-5 yl | |
| 7.187 | NO₂ | SO₂Me | S | 1,3-Dioxan-5 yl | |
| 7.188 | NO₂ | SO₂Et | S | 1,3-Dioxan-5 yl | |
| 7.189 | NO₂ | Cl | S | 1,3-Dioxan-5 yl | |
| 7.190 | Cl | SO₂Me | S | γ-butyrolacton-2-yl | |
| 7.191 | Cl | SO₂Et | S | γ-butyrolacton-2-yl | |
| 7.192 | Cl | Cl | S | γ-butyrolacton-2-yl | |
| 7.193 | Br | SO₂Me | S | γ-butyrolacton-2-yl | |
| 7.194 | Br | SO₂Et | S | γ-butyrolacton-2-yl | |
| 7.195 | Br | Cl | S | γ-butyrolacton-2-yl | |
| 7.196 | I | SO₂Me | S | γ-butyrolacton-2-yl | |
| 7.197 | I | SO₂Et | S | γ-butyrolacton-2-yl | |
| 7.198 | I | Cl | S | γ-butyrolacton-2-yl | |
| 7.199 | Me | SO₂Me | S | γ-butyrolacton-2-yl | |
| 7.200 | Me | SO₂Et | S | γ-butyrolacton-2-yl | |
| 7.201 | Me | Cl | S | γ-butyrolacton-2-yl | |
| 7.202 | SMe | SO₂Me | S | γ-butyrolacton-2-yl | |
| 7.203 | SMe | SO₂Et | S | γ-butyrolacton-2-yl | |
| 7.204 | SMe | Cl | S | γ-butyrolacton-2-yl | |
| 7.205 | SO₂Me | SO₂Me | S | γ-butyrolacton-2-yl | |
| 7.206 | SO₂Me | SO₂Et | S | γ-butyrolacton-2-yl | |
| 7.207 | SO₂Me | Cl | S | γ-butyrolacton-2-yl | |
| 7.208 | NO₂ | SO₂Me | S | γ-butyrolacton-2-yl | |
| 7.209 | NO₂ | SO₂Et | S | γ-butyrolacton-2-yl | |
| 7.210 | NO₂ | Cl | S | γ-butyrolacton-2-yl | |

**Tabelle 8: Erfindungsgemäße Verbindungen der allgemeinen Formel (1), worin die Substituenten und Symbole folgende Bedeutungen haben:**

| | | | | | |
|---|---|---|---|---|---|
| | R¹ = Cl | | R² = SO₂Me | | R³ = H |
| | Q = Q2 | | X = 0 | | |
| 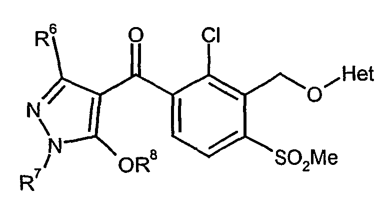 | | | | | |

| **Nr.** | **R⁵** | **R⁷** | **R⁸** | **Het** | **Physikal. Daten** |
|---|---|---|---|---|---|
| 8.1 | H | Me | SO₂Me | 3-Tetrahydrofuranyl | |
| 8.2 | H | Me | SO₂Me | 3-Tetrahydrofuranyl | |
| 8.3 | H | Me | SO₂-nPr | 3-Tetrahydrofuranyl | |
| 8.4 | H | Me | SO₂-nBu | 3-Tetrahydrofuranyl | |
| 8.5 | H | Me | SO₂Ph | 3-Tetrahydrofuranyl | |
| 8.6 | H | Me | CO₂Me | 3-Tetrahydrofuranyl | |
| 8.7 | H | Me | CO₂Et | 3-Tetrahydrofuranyl | |
| 8.8 | H | Me | CO₂-nPr | 3-Tetrahydrofuranyl | |
| 8.9 | H | Me | CO₂-nBu | 3-Tetrahydrofuranyl | |
| 8.10 | H | Me | CO₂Ph | 3-Tetrahydrofuranyl | |
| 8.11 | H | Me | Me | 3-Tetrahydrofuranyl | |
| 8.12 | H | Me | Et | 3-Tetrahydrofuranyl | |
| 8.13 | H | Me | nPr | 3-Tetrahydrofuranyl | |
| 8.14 | H | Me | nBu | 3-Tetrahydrofuranyl | |
| 8.15 | Me | Me | SO₂Me | 3-Tetrahydrofuranyl | |
| 8.16 | Me | Me | SO₂Me | 3-Tetrahydrofuranyl | |
| 8.17 | Me | Me | SO₂-nPr | 3-Tetrahydrofuranyl | |
| 8.18 | Me | Me | SO₂-nBu | 3-Tetrahydrofuranyl | |
| 8.19 | Me | Me | SO₂Ph | 3-Tetrahydrofuranyl | |
| 8.20 | Me | Me | CO₂Me | 3-Tetrahydrofuranyl | |
| 8.21 | Me | Me | CO₂Et | 3-Tetrahydrofuranyl | |
| 8.22 | Me | Me | CO₂-nPr | 3-Tetrahydrofuranyl | |
| 8.23 | Me | Me | CO₂-nBu | 3-Tetrahydrofuranyl | |
| 8.24 | Me | Me | CO₂Ph | 3-Tetrahydrofuranyl | |
| 8.25 | Me | Me | Me | 3-Tetrahydrofuranyl | |
| 8.26 | Me | Me | Et | 3-Tetrahydrofuranyl | |
| 8.27 | Me | Me | nPr | 3-Tetrahydrofuranyl | |
| 8.28 | Me | Me | nBu | 3-Tetrahydrofuranyl | |
| 8.29 | H | Et | SO₂Me | 3-Tetrahydrofuranyl | |
| 8.30 | H | Et | SO₂Me | 3-Tetrahydrofuranyl | |
| 8.31 | H | Et | SO₂-nPr | 3-Tetrahydrofuranyl | |
| 8.32 | H | Et | SO₂-nBu | 3-Tetrahydrofuranyl | |
| 8.33 | H | Et | SO₂Ph | 3-Tetrahydrofuranyl | |
| 8.34 | H | Et | CO₂Me | 3-Tetrahydrofuranyl | |
| 8.35 | H | Et | CO₂Et | 3-Tetrahydrofuranyl | |
| 8.36 | H | Et | CO₂-nPr | 3-Tetrahydrofuranyl | |
| 8.37 | H | Et | CO₂-nBu | 3-Tetrahydrofuranyl | |
| 8.38 | H | Et | CO₂Ph | 3-Tetrahydrofuranyl | |
| 8.39 | H | Et | Me | 3-Tetrahydrofuranyl | |
| 8.40 | H | Et | Et | 3-Tetrahydrofuranyl | |
| 8.41 | H | Et | nPr | 3-Tetrahydrofuranyl | |
| 8.42 | H | Et | nBu | 3-Tetrahydrofuranyl | |
| 8.43 | cPr | Me | SO₂Me | 3-Tetrahydrofuranyl | |
| 8.44 | cPr | Me | SO₂Me | 3-Tetrahydrofuranyl | |
| 8.45 | cPr | Me | SO₂-nPr | 3-Tetrahydrofuranyl | |
| 8.46 | cPr | Me | SO₂-nBu | 3-Tetrahydrofuranyl | |
| 8.47 | cPr | Me | SO₂Ph | 3-Tetrahydrofuranyl | |
| 8.48 | cPr | Me | CO₂Me | 3-Tetrahydrofuranyl | |
| 8.49 | cPr | Me | CO₂Et | 3-Tetrahydrofuranyl | |
| 8.50 | cPr | Me | CO₂-nPr | 3-Tetrahydrofuranyl | |
| 8.51 | cPr | Me | CO₂-nBu | 3-Tetrahydrofuranyl | |
| 8.52 | cPr | Me | CO₂Ph | 3-Tetrahydrofuranyl | |
| 8.53 | cPr | Me | Me | 3-Tetrahydrofuranyl | |
| 8.54 | cPr | Me | Et | 3-Tetrahydrofuranyl | |
| 8.55 | cPr | Me | nPr | 3-Tetrahydrofuranyl | |
| 8.56 | cPr | Me | nBu | 3-Tetrahydrofuranyl | |
| 8.57 | H | Me | SO₂Me | 3-Tetrahydropyranyl | |
| 8.58 | H | Me | SO₂Me | 3-Tetrahydropyranyl | |
| 8.59 | H | Me | SO₂-nPr | 3-Tetrahydropyranyl | |
| 8.60 | H | Me | SO₂-nBu | 3-Tetrahydropyranyl | |
| 8.61 | H | Me | SO₂Ph | 3-Tetrahydropyranyl | |
| 8.62 | H | Me | CO₂Me | 3-Tetrahydropyranyl | |
| 8.63 | H | Me | CO₂Et | 3-Tetrahydropyranyl | |
| 8.64 | H | Me | CO₂-nPr | 3-Tetrahydropyranyl | |
| 8.65 | H | Me | CO₂-nBu | 3-Tetrahydropyranyl | |
| 8.66 | H | Me | CO₂Ph | 3-Tetrahydropyranyl | |
| 8.67 | H | Me | Me | 3-Tetrahydropyranyl | |
| 8.68 | H | Me | Et | 3-Tetrahydropyranyl | |
| 8.69 | H | Me | nPr | 3-Tetrahydropyranyl | |
| 8.70 | H | Me | nBu | 3-Tetrahydropyranyl | |
| 8.71 | Me | Me | SO₂Me | 3-Tetrahydropyranyl | |
| 8.72 | Me | Me | SO₂Me | 3-Tetrahydropyranyl | |
| 8.73 | Me | Me | SO₂-nPr | 3-Tetrahydropyranyl | |
| 8.74 | Me | Me | SO₂-nBu | 3-Tetrahydropyranyl | |
| 8.75 | Me | Me | SO₂Ph | 3-Tetrahydropyranyl | |
| 8.76 | Me | Me | CO₂Me | 3-Tetrahydropyranyl | |
| 8.77 | Me | Me | CO₂Et | 3-Tetrahydropyranyl | |
| 8.78 | Me | Me | CO₂-nPr | 3-Tetrahydropyranyl | |
| 8.79 | Me | Me | CO₂-nBu | 3-Tetrahydropyranyl | |
| 8.80 | Me | Me | CO₂Ph | 3-Tetrahydropyranyl | |
| 8.81 | Me | Me | Me | 3-Tetrahydropyranyl | |
| 8.82 | Me | Me | Et | 3-Tetrahydropyranyl | |
| 8.83 | Me | Me | nPr | 3-Tetrahydropyranyl | |
| 8.84 | Me | Me | nBu | 3-Tetrahydropyranyl | |
| 8.85 | H | Et | SO₂Me | 3-Tetrahydropyranyl | |
| 8.86 | H | Et | SO₂Me | 3-Tetrahydropyranyl | |
| 8.87 | H | Et | SO₂-nPr | 3-Tetrahydropyranyl | |
| 8.88 | H | Et | SO₂-nBu | 3-Tetrahydropyranyl | |
| 8.89 | H | Et | SO₂Ph | 3-Tetrahydropyranyl | |
| 8.90 | H | Et | CO₂Me | 3-Tetrahydropyranyl | |
| 8.91 | H | Et | CO₂Et | 3-Tetrahydropyranyl | |
| 8.92 | H | Et | CO₂-nPr | 3-Tetrahydropyranyl | |
| 8.93 | H | Et | CO₂-nBu | 3-Tetrahydropyranyl | |
| 8.94 | H | Et | CO₂Ph | 3-Tetrahydropyranyl | |
| 8.95 | H | Et | Me | 3-Tetrahydropyranyl | |
| 8.96 | H | Et | Et | 3-Tetrahydropyranyl | |
| 8.97 | H | Et | nPr | 3-Tetrahydropyranyl | |
| 8.98 | H | Et | nBu | 3-Tetrahydropyranyl | |
| 8.99 | cPr | Me | SO₂Me | 3-Tetrahydropyranyl | |
| 8.100 | cPr | Me | SO₂Me | 3-Tetrahydropyranyl | |
| 8.101 | cPr | Me | SO₂-nPr | 3-Tetrahydropyranyl | |
| 8.102 | cPr | Me | SO₂-nBu | 3-Tetrahydropyranyl | |
| 8.103 | cPr | Me | SO₂Ph | 3-Tetrahydropyranyl | |
| 8.104 | cPr | Me | CO₂Me | 3-Tetrahydropyranyl | |
| 8.105 | cPr | Me | CO₂Et | 3-Tetrahydropyranyl | |
| 8.106 | cPr | Me | CO₂-nPr | 3-Tetrahydropyranyl | |
| 8.107 | cPr | Me | CO₂-nBu | 3-Tetrahydropyranyl | |
| 8.108 | cPr | Me | CO₂Ph | 3-Tetrahydropyranyl | |
| 8.109 | cPr | Me | Me | 3-Tetrahydropyranyl | |
| 8.110 | cPr | Me | Et | 3-Tetrahydropyranyl | |
| 8.111 | cPr | Me | nPr | 3-Tetrahydropyranyl | |
| 8.112 | cPr | Me | nBu | 3-Tetrahydropyranyl | |
| 8.113 | H | Me | SO₂Me | 1,3-Dioxan-5 yl | |
| 8.114 | H | Me | SO₂Me | 1,3-Dioxan-5 yl | |
| 8.115 | H | Me | SO₂-nPr | 1,3-Dioxan-5 yl | |
| 8.116 | H | Me | SO₂-nBu | 1,3-Dioxan-5 yl | |
| 8.117 | H | Me | SO₂Ph | 1,3-Dioxan-5 yl | |
| 8.118 | H | Me | CO₂Me | 1,3-Dioxan-5 yl | |
| 8.119 | H | Me | CO₂Et | 1,3-Dioxan-5 yl | |
| 8.120 | H | Me | CO₂-nPr | 1,3-Dioxan-5 yl | |
| 8.121 | H | Me | CO₂-nBu | 1,3-Dioxan-5 yl | |
| 8.122 | H | Me | CO₂Ph | 1,3-Dioxan-5 yl | |
| 8.123 | H | Me | Me | 1,3-Dioxan-5 yl | |
| 8.124 | H | Me | Et | 1,3-Dioxan-5 yl | |
| 8.125 | H | Me | nPr | 1,3-Dioxan-5 yl | |
| 8.126 | H | Me | nBu | 1,3-Dioxan-5 yl | |
| 8.127 | Me | Me | SO₂Me | 1,3-Dioxan-5 yl | |
| 8.128 | Me | Me | SO₂Me | 1,3-Dioxan-5 yl | |
| 8.129 | Me | Me | SO₂-nPr | 1,3-Dioxan-5 yl | |
| 8.130 | Me | Me | SO₂-nBu | 1,3-Dioxan-5 yl | |
| 8.131 | Me | Me | SO₂Ph | 1,3-Dioxan-5 yl | |
| 8.132 | Me | Me | CO₂Me | 1,3-Dioxan-5 yl | |
| 8.133 | Me | Me | CO₂Et | 1,3-Dioxan-5 yl | |
| 8.134 | Me | Me | CO₂-nPr | 1,3-Dioxan-5 yl | |
| 8.135 | Me | Me | CO₂-nBu | 1,3-Dioxan-5 yl | |
| 8.136 | Me | Me | CO₂Ph | 1,3-Dioxan-5 yl | |
| 8.137 | Me | Me | Me | 1,3-Dioxan-5 yl | |
| 8.138 | Me | Me | Et | 1,3-Dioxan-5 yl | |
| 8.139 | Me | Me | nPr | 1,3-Dioxan-5 yl | |
| 8.140 | Me | Me | nBu | 1,3-Dioxan-5 yl | |
| 8.141 | H | Et | SO₂Me | 1,3-Dioxan-5 yl | |
| 8.142 | H | Et | SO₂Me | 1,3-Dioxan-5 yl | |
| 8.143 | H | Et | SO₂-nPr | 1,3-Dioxan-5 yl | |
| 8.144 | H | Et | SO₂-nBu | 1,3-Dioxan-5 yl | |
| 8.145 | H | Et | SO₂Ph | 1,3-Dioxan-5 yl | |
| 8.146 | H | Et | CO₂Me | 1,3-Dioxan-5 yl | |
| 8.147 | H | Et | CO₂Et | 1,3-Dioxan-5 yl | |
| 8.148 | H | Et | CO₂-nPr | 1,3-Dioxan-5 yl | |
| 8.149 | H | Et | CO₂-nBu | 1,3-Dioxan-5 yl | |
| 8.150 | H | Et | CO₂Ph | 1,3-Dioxan-5 yl | |
| 8.151 | H | Et | Me | 1,3-Dioxan-5 yl | |
| 8.152 | H | Et | Et | 1,3-Dioxan-5 yl | |
| 8.153 | H | Et | nPr | 1,3-Dioxan-5 yl | |
| 8.154 | H | Et | nBu | 1,3-Dioxan-5 yl | |
| 8.155 | cPr | Me | SO₂Me | 1,3-Dioxan-5 yl | |
| 8.156 | cPr | Me | SO₂Me | 1,3-Dioxan-5 yl | |
| 8.157 | cPr | Me | SO₂-nPr | 1,3-Dioxan-5 yl | |
| 8.158 | cPr | Me | SO₂-nBu | 1,3-Dioxan-5 yl | |
| 8.159 | cPr | Me | SO₂Ph | 1,3-Dioxan-5 yl | |
| 8.160 | cPr | Me | CO₂Me | 1,3-Dioxan-5 yl | |
| 8.161 | cPr | Me | CO₂Et | 1,3-Dioxan-5 yl | |
| 8.162 | cPr | Me | CO₂-nPr | 1,3-Dioxan-5 yl | |
| 8.163 | cPr | Me | CO₂-nBu | 1,3-Dioxan-5 yl | |
| 8.164 | cPr | Me | CO₂Ph | 1,3-Dioxan-5 yl | |
| 8.165 | cPr | Me | Me | 1,3-Dioxan-5 yl | |
| 8.166 | cPr | Me | Et | 1,3-Dioxan-5 yl | |
| 8.167 | cPr | Me | nPr | 1,3-Dioxan-5 yl | |
| 8.168 | cPr | Me | nBu | 1,3-Dioxan-5 yl | |
| 8.169 | H | Me | SO₂Me | γ-butyrolacton-2-yl | |
| 8.170 | H | Me | SO₂Me | γ-butyrolacton-2-yl | |
| 8.171 | H | Me | SO₂-nPr | γ-butyrolacton-2-yl | |
| 8.172 | H | Me | SO₂-nBu | γ-butyrolacton-2-yl | |
| 8.173 | H | Me | SO₂Ph | γ-butyrolacton-2-yl | |
| 8.174 | H | Me | CO₂Me | γ-butyrolacton-2-yl | |
| 8.175 | H | Me | CO₂Et | γ-butyrolacton-2-yl | |
| 8.176 | H | Me | CO₂-nPr | γ-butyrolacton-2-yl | |
| 8.177 | H | Me | CO₂-nBu | γ-butyrolacton-2-yl | |
| 8.178 | H | Me | CO₂Ph | γ-butyrolacton-2-yl | |
| 8.179 | H | Me | Me | γ-butyrolacton-2-yl | |
| 8.180 | H | Me | Et | γ-butyrolacton-2-yl | |
| 8.181 | H | Me | nPr | γ-butyrolacton-2-yl | |
| 8.182 | H | Me | nBu | γ-butyrolacton-2-yl | |
| 8.183 | Me | Me | SO₂Me | γ-butyrotacton-2-yl | |
| 8.184 | Me | Me | SO₂Me | γ-butyrolacton-2-yl | |
| 8.185 | Me | Me | SO₂-nPr | γ-butyrolacton-2-yl | |
| 8.186 | Me | Me | SO₂-nBu | γ-butyrolacton-2-yl | |
| 8.187 | Me | Me | SO₂Ph | γ-butyrolacton-2-yl | |
| 8.188 | Me | Me | CO₂Me | γ-butyrolacton-2-yl | |
| 8.189 | Me | Me | CO₂Et | γ-butyrolacton-2-yl | |
| 8.190 | Me | Me | CO₂-nPr | γ-butyrolacton-2-yl | |
| 8.191 | Me | Me | CO₂-nBu | γ-butyrolacton-2-yl | |
| 8.192 | Me | Me | CO₂Ph | γ-butyrolacton-2-yl | |
| 8.193 | Me | Me | Me | γ-butyrolacton-2-yl | |
| 8.194 | Me | Me | Et | γ-butyrolacton-2-yl | |
| 8.195 | Me | Me | nPr | γ-butyrolacton-2-yl | |
| 8.196 | Me | Me | nBu | γ-butyrolacton-2-yl | |
| 8.197 | H | Et | SO₂Me | γ-butyrolacton-2-yl | |
| 8.198 | H | Et | SO₂Me | γ-butyrolacton-2-yl | |
| 8.199 | H | Et | SO₂-nPr | γ-butyrolacton-2-yl | |
| 8.200 | H | Et | SO₂-nBu | γ-butyrolacton-2-yl | |
| 8.201 | H | Et | SO₂Ph | γ-butyrolacton-2-yl | |
| 8.202 | H | Et | CO₂Me | γ-butyrolacton-2-yl | |
| 8.203 | H | Et | CO₂Et | γ-butyrolacton-2-yl | |
| 8.204 | H | Et | CO₂-nPr | γ-butyrolacton-2-yl | |
| 8.205 | H | Et | CO₂-nBu | γ-butyrolacton-2-yl | |
| 8.206 | H | Et | CO₂Ph | γ-butyrolacton-2-yl | |
| 8.207 | H | Et | Me | γ-butyrolacton-2-yl | |
| 8.208 | H | Et | Et | γ-butyrolacton-2-yl | |
| 8.209 | H | Et | nPr | γ-butyrolacton-2-yl | |
| 8.210 | H | Et | nBu | γ-butyrolacton-2-yl | |
| 8.211 | cPr | Me | SO₂Me | γ-butyrolacton-2-yl | |
| 8.212 | cPr | Me | SO₂Me | γ-butyroacton-2-yl | |
| 8.213 | cPr | Me | SO₂-nPr | γ-butyrolacton-2-yl | |
| 8.214 | cPr | Me | SO₂-nBu | γ-butyrolacton-2-yl | |
| 8.215 | cPr | Me | SO₂Ph | γ-butyrolacton-2-yl | |
| 8.216 | cPr | Me | CO₂Me | γ-butyrolacton-2-yl | |
| 8.217 | cPr | Me | CO₂Et | γ-butyrolacton-2-yl | |
| 8.218 | cPr | Me | CO₂-nPr | γ-butyrolacton-2-yl | |
| 8.219 | cPr | Me | CO₂-nBu | γ-butyrolacton-2-yl | |
| 8.220 | cPr | Me | CO₂Ph | γ-butyrolacton-2-yl | |
| 8.221 | cPr | Me | Me | γ-butyrolacton-2-yl | |
| 8.222 | cPr | Me | Et | γ-butyrolacton-2-yl | |
| 8.223 | cPr | Me | nPr | γ-butyrolacton-2-yl | |
| 8.224 | cPr | Me | nBu | γ-butyrolacton-2-yl | |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | |
|---|---|
| 75 | Gew.-Teile einer Verbindung der allgemeinen Formel (I), |
| 10 | " ligninsulfonsaures Calcium, |
| 5 | " Natriumlaurylsulfat, |
| 3 | " Polyvinylalkohol und |
| 7 | " Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | |
|---|---|
| 25 | Gew.-Teile einer Verbindung der allgemeinen Formel (I), |
| 5 | " 2,2'-dinaphthylmethan-6,6'-disulfonsaures Natrium, |
| 2 | " oleoylmethyltaurinsaures Natrium, |
| 1 | " Polyvinylalkohol, |
| 17 | " Calciumcarbonat und |
| 50 | " Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen von mono- und dikotylen Unkrautpflanzen werden in Papptöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 I/ha in in verschiedenen Dosierungen auf die Oberfläche der Abdeckerde appliziert. Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen.
Nach 3 bis 4 Wochen Standzeit der Versuchspflanzen im Gewächshaus unter optimalen Wachstumsbedingungen wird die Wirkung der Verbindungen bonitiert. Dabei weisen die erfindungsgemäßen Verbindungen eine hervorragende Wirksamkeit gegen ein breites Spektrum wirtschaftlich wichtiger mono- und dikotyler Schadpflanzen auf.
So zeigt beispielweise die erfindungsgemäße Verbindung der Nr. 1.1 bei einer Dosierung von 320 g/ha eine mindestens 90%-ige Wirkung gegen die Schadpflanzen Galium aparine, Matricaria inodora, Stellaria media, Chenopodium album, Veronica persica und Abutilon theophrasti auf.

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha in verschiedenen Dosierungen auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen bonitiert. Dabei weisen die erfindungsgemäßen Verbindungen eine hervorragende Wirksamkeit gegen ein breites Spektrum wirtschaftlich wichtiger mono- und dikotyler Schadpflanzen auf.
So zeigt beispielweise die erfindungsgemäße Verbindung der Nr. 3.1 bei einer Dosierung von 320 g/ha eine mindestens 80%-ige Wirkung gegen die Schadpflanzen Sinapis arvensis, Avena fatua, Amaranthus retroflexus und Setaria viridis auf.

### 3. Kulturpflanzenverträglichkeit

In weiteren Versuchen im Gewächshaus werden Samen von Gerste und mono- und dikotyler Schadpflanzen in sandigem Lehmboden ausgelegt, mit Erde abgedeckt und im Gewächshaus aufgestellt, bis die Pflanzen zwei bis drei echte Blätter entwickelt haben. Die Behandlung mit den erfindungsgemäßen Verbindungen der Formel (I) erfolgt dann wie oben unter Punkt 2 beschrieben. Vier bis fünf Wochen nach der Applikation und Standzeit im Gewächshaus wird mittels optischer Bonitur festgestellt, daß die erfindungsgemäßen Verbindungen eine hervorragende Verträglichkeit gegenüber wichtigen Kulturpflanzen, insbesondere Weizen, Mais und Reis, aufweisen.
So zeigt beispielweise die erfindungsgemäße Verbindung der Nr. 1.1 bei einer Dosierung von 50 g/ha eine mindestens 95%-ige Wirkung gegen die Schadpflanzen Echinochloa crus galli, Sagittaria pygmaea, Cyperus serotinus und Scirpus juncoides auf, wobei gleichzeitig keine Schädigung der Kulturpflanze Reis verursacht wird.
Die erfindungsgemäße Verbindung der Nr. 1.85 zeigt bei einer Dosierung von 320 g/ha eine mindestens 90%-ige Wirkung gegen die Schadpflanzen Stellaria media, Veronica persica, Chenopodium album und Abutilon theophrasti auf, wobei gleichzeitig keine Schädigung der Kulturpflanzen Reis, Weizen und Mais verursacht wird.

## Patentansprüche

1. Verbindungen der Formel (I) oder deren Salze 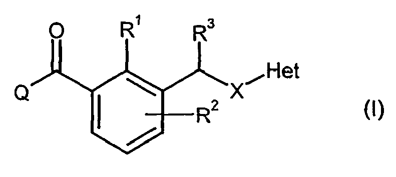 worin die Reste und Indizes folgende Bedeutungen haben:
R¹, R² bedeuten unabhängig voneinander Wasserstoff, Mercapto, Nitro, Halogen, Cyano, Rhodano, C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₃-C₆-Cycloalkyl, -OR⁴, OCOR⁴, OSO₂R⁴, S(O)ₙR⁴, SO₂OR⁴, SO₂N(R⁴)₂, NR⁴SO₂R⁴, NR⁴COR⁴, C₁-C₆-Alkyl-S(O)ₙR⁴, C₁-C₆-Alkyl-OR⁴, C₁-C₆-Alkyl-OCOR⁴, C₁-C₆-Alkyl-OSO₂R⁴, C₁-C₆-Alkyl-SO₂OR⁴, C₁-C₆-Alkyl-SO₂N(R⁴)₂ oder C₁-C₆-Alkyl-NR⁴COR⁴;
R³ bedeutet Wasserstoff, C₁-C₆-Alkyl, C₂-C₆-Alkenyl oder C₂-C₆-Alkinyl;
R⁴ bedeutet Wasserstoff, C₁-C₆-Alkyl, C₂-C₆-Alkenyl, C₂-C₆-Alkinyl, C₃-C₆-Cycloalkyl, Phenyl oder Phenyl- C₁-C₆-alkyl, wobei die sechs letztgenannten Reste durch s Reste der Gruppe Hydroxy, Mercapto, Amino, Cyano, Nitro, Rhodano, OR³, SR³, N(R³)₂, =NOR³, OCOR³, SCOR³, NR³COR³, CO₂R³, COSR³, CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl und C₁-C₄-Alkylsulfonyl substituiert sind;
Het bedeutet eine vollständig gesättigte heterocyclische Gruppe, deren Ringatome aus KohlenstofF- und Sauerstoffatomen bestehen, wobei
die Gesamtzahl der Ringatome p beträgt,
die Anzahl der Sauerstoffatome r beträgt,
die Anzahl der Kohlenstoffatome (p-r) beträgt, und
Het durch n Reste R⁵ substituiert sein kann;
n bedeutet 0, 1 oder 2;
p bedeutet 5, 6 oder 7;
r bedeutet 1 oder 2;
s bedeutet 0, 1, 2 oder 3;
X bedeutet 0 oder S(O)ₙ;
R⁵ bedeutet Hydroxy, Mercapto, Amino, Cyano, Nitro, Halogen, Formyl, C₁-C₆-Alkylamino, C₁-C₆-Dialkylamino, C₁-C₆-Alkoxycarbonyl, C₁-C₆-Alkylcarbonyl, C₁-C₄-Alkylcarbonyloxy, C₁-C₆-Alkyl, C₁-C₆-Halogenalkyl, C₁-C₆-Alkylthio, C₁-C₆-Halogenalkylthio, C₁-C₆-Alkoxy, C₁-C₆-Halogenalkoxy oder R⁵ bildet zusammen mit dem Kohlenstoffatom, an dem es gebunden ist, eine Carbonylgruppe;
Q bedeutet einen Rest der Gruppe Q1 oder Q2; 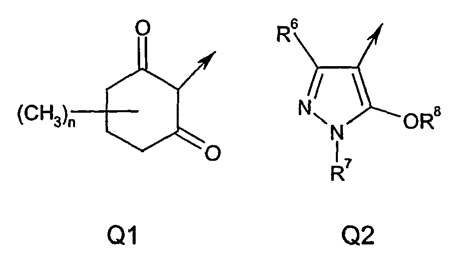
R⁶, R⁷ bedeuten unabhängig voneinander Wasserstoff, C₁-C₆-Alkyl, C₁-C₆-Halogenalkyl oder C₃-C₆-Cyclopropyl;
R⁸ bedeutet Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenyloxycarbonyl oder Phenylsulfonyl, wobei der Phenylkern der vier letztgenannten Reste durch s
Reste aus der Gruppe Halogen, Nitro, Cyano, C₁-C₆-Alkyl, C₁-C₆-Halogenalkyl, C₁-C₆-Alkoxy und C₁-C₆-Halogenalkoxy substituiert ist.

2. Verbindungen nach Anspruch 1, worin
R¹, R² bedeuten unabhängig voneinander Wasserstoff, Nitro, Halogen, C₁-C₄-Alkyl, C₁-C₄-Halogenalkyl, C₂-C₆-Alkenyl, C₂-C₆-Halogenalkenyl, C₂-C₆-Alkinyl, C₂-C₆-Halogenalkinyl, C₃-C₆-Cycloalkyl, -OR⁴ S(O)ₙR⁴, SO₂OR⁴, SO₂N(R⁴)₂, NR⁴SO₂R⁴ oder C₁-C₆-Alkyl-S(O)ₙR⁴;
R⁴ bedeutet Wasserstoff, C₁-C₄-Alkyl, C₂-C₄-Alkenyl, C₂-C₄-Alkinyl, C₃-C₆-Cycloalkyl, Phenyl oder Phenyl- C₁-C₄-alkyl, wobei die sechs letztgenannten Reste durch s Reste der Gruppe Cyano, Nitro, R³, OR³, SR³ oder N(R³)₂ substituiert sind.

3. Verbindungen nach Anspruch 1 oder 2, worin
R³ bedeutet Wasserstoff;
R⁵ bedeutet Cyano, Nitro, Halogen, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₆-Alkoxy, C₁-C₆-Halogenalkoxy oder R⁵ bildet zusammen mit dem Kohlenstoffatom, an dem es gebunden ist, eine Carbonylgruppe.

4. Verbindungen nach einem der Ansprüche 1 bis 3, worin
R⁶, R⁷ bedeuten unabhängig voneinander Wasserstoff oder C₁-C₄-Alkyl;
R⁸ bedeutet Wasserstoff, C₁-C₄-Alkyl, C₁-C₄-Halogenalkyl, C₁-C₄-Alkylcarbonyl, C₁-C₄-Halogenalkylcarbonyl, C₁-C₄-Alkoxycarbonyl, C₁-C₄-Alkylsulfonyl, C₁-C₄-Halogenalkylsulfonyl, Phenylcarbonyl, Phenylcarbonylmethyl, Phenyloxycarbonyl oder Phenylsulfonyl, wobei der Phenylkern der vier letztgenannten Reste durch s Reste aus der Gruppe Halogen Nitro, Cyano, C₁-C₄-Alkyl, C₁-C₄-Halogenalkyl, C₁-C₄-Alkoxy und C₁-C₄-Halogenalkoxy substituiert ist.

5. Verbindungen nach einem der Ansprüche 1 bis 4, worin
R¹ bedeutet Chlor, Brom, Iod, Nitro, Methyl, Thiomethyl, Thioethyl, Methylsulfonyl, Ethylsulfonyl oder Methoxy;
R² bedeutet Brom, Chlor, Methylsulfonyl oder Ethylsulfonyl;
R² befindet sich in der 4-Position des Phenylrings;
R⁸ bedeutet Wasserstoff,
Het bedeutet 3-Tetrahydrofuranyl, 3-Tetrahydropyranyl, 4-Tetrahydropyranyl, 1,3-Dioxan-5-yl oder γ-Butyrolacton-2-yl.

6. Herbizide Mittel, **gekennzeichnet durch** einen herbizid wirksamen Gehalt an mindestens einer Verbindung der allgemeinen Formel (I) gemäß einem der Ansprüche 1 bis 5

7. Herbizide Mittel nach Anspruch 6 in Mischung mit Formulierungshilfsmitteln.

8. Verfahren zur Bekämpfung unerwünschter Pflanzen, **dadurch gekennzeichnet, daß** man eine wirksame Menge mindestens einer Verbindung der allgemeinen Formel (1) nach einem der Ansprüche 1 bis 5 oder eines herbiziden Mittels nach Anspruch 6 oder 7 auf die Pflanzen oder auf den Ort des unerwünschten Pflanzenwachstums appliziert.

9. Verwendung von Verbindungen der allgemeinen Formel (I) nach einem der Ansprüche 1 bis 5 oder von herbiziden Mitteln nach Anspruch 6 oder 7 zur Bekämpfung unerwünschter Pflanzen.

10. Verwendung nach Anspruch 9, **dadurch gekennzeichnet, daß** die Verbindungen der allgemeinen Formel (I) zur Bekämpfung unerwünschter Pflanzen in Kulturen von Nutzpflanzen eingesetzt werden.

11. Verwendung nach Anspruch 10, **dadurch gekennzeichnet, daß** die Nutzpflanzen transgene Nutzpflanzen sind.

## Claims

1. A compound of the formula (I) or a salt thereof 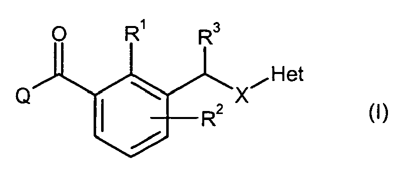 in which the radicals and indices are as defined below:
R¹, R² independently of one another are hydrogen, mercapto, nitro, halogen, cyano, thiocyanato, C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cydoalkyl, -OR⁴, OCOR⁴, OSO₂R⁴, S(O)ₙR⁴, SO₂OR⁴, SO₂N(R⁴)₂, NR⁴SO₂R⁴, NR⁴COR⁴, C₁-C₆-alkyl-S(O)nR⁴, C₁-C₆-alkyl-OR⁴, C₁-C₆-alkyl-OCOR⁴, C₁-C₆-alkyl-OSO₂R⁴, C₁-C₆-alkyl-SO₂OR⁴, C₁-C₆-alkyl-SO₂N(R⁴)₂ or C₁-C₆-alkyl-NR⁴COR⁴;
R³ is hydrogen, C₁-C₆-alkyl, C₂-C₆-alkenyl or C₂-C₆-alkynyl;
R⁴ is hydrogen, C₁-C₆-alkyl, C₂-C₆-alkenyl, C₂-C₆-alkynyl, C₃-C₆-cydoalkyl, phenyl or phenyl-C₁-C₆-alkyl, where the six last-mentioned radicals are substituted by s radicals selected from the group consisting of hydroxy, mercapto, amino, cyano, nitro, thiocyanato, OR³, SR³, N(R³)₂, =NOR³, OCOR³, SCOR³, NR³COR³, CO₂R³, COSR³, CON(R³)₂, C₁-C₄-alkyliminooxy, C₁-C₄-alkoxyamino, C₁-C₄-alkylcarbonyl, C₁-C₄-alkoxy-C₂-C₆-alkoxycarbonyl and C₁-C₄-alkylsulfonyl;
Het is a fully saturated heterocyclic group whose ring atoms consist of carbon and oxygen atoms, where
the total number of ring atoms is p,
the number of oxygen atoms is r,
the number of carbon atoms is (p-r) and
Het may be substituted by n radicals R⁵;
n is 0, 1 or 2;
p is 5, 6 or 7;
r is 1 or 2;
s is 0, 1, 2 or 3;
X is O or S(O)ₙ;
R⁵ is hydroxy, mercapto, amino, cyano, nitro, halogen, formyl, C₁-C₆-alkylamino, C₁-C₆-dialkylamino, C₁-C₆-alkoxycarbonyl, C₁-C₆-alkylcarbonyl, C₁-C₄-alkylcarbonyloxy, C₁-C₆-alkyl, C₁-C₆-haloalkyl, C₁-C₆-alkylthio, C₁-C₆-haloalkylthio, C₁-C₆-alkoxy, C₁-C₆-haloalkoxy or R⁵ together with the carbon atom to which it is attached forms a carbonyl group;
Q is a radical of group Q1 or Q2; 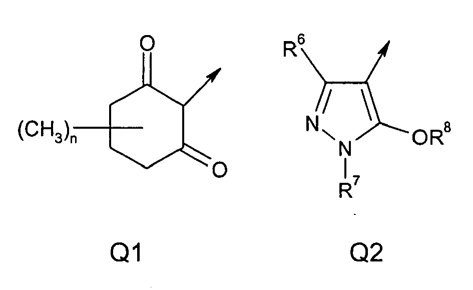
R⁶, R⁷ independently of one another are hydrogen, C₁-C₆-alkyl, C₁-C₆-haloalkyl or C₃-C₆-cyclopropyl;
R⁸ is hydrogen, C₁-C₆-alkyl, C₁-C₆-haloalkyl, C₁-C₆-alkylcarbonyl, C₁-C₆-haloalkylcarbonyl, C₁-C₆-alkoxycarbonyl, C₁-C₆-alkylsulfonyl, C₁-C₆-haloalkylsulfonyl, phenylcarbonyl, phenylcarbonylmethyl, phenyloxycarbonyl or phenylsulfonyl, where the phenyl ring of the four last-mentioned radicals is substituted by s radicals selected from the group consisting of halogen, nitro, cyano, C₁-C₆-alkyl, C₁-C₆-haloalkyl, C₁-C₆-alkoxy and C₁-C₆-haloalkoxy.

2. A compound as claimed in claim 1, in which
R¹, R² independently of one another are hydrogen, nitro, halogen, C₁-C₄-alkyl, C₁-C₄-haloalkyl, C₂-C₆-alkenyl, C₂-C₆-haloalkenyl, C₂-C₆-alkynyl, C₂-C₆-haloalkynyl, C₃-C₆-cydoalkyl, -OR⁴, S(O)ₙR⁴, SO₂OR⁴, SO₂N(R⁴)₂, NR⁴SO₂R⁴ or C₁-C₆-alkyl-S(O)ₙR⁴;
R⁴ is hydrogen, C₁-C₄-alkyl, C₂-C₄-alkenyl, C₂-C₄-alkynyl, C₃-C₆-cydoalkyl, phenyl or phenyl-C₁-C₄-alkyl, where the six last-mentioned radicals are substituted by s radicals selected from the group consisting of cyano, nitro, R³, OR³, SR³ and N(R³)₂.

3. A compound as claimed in claim 1 or 2, in which
R³ is hydrogen;
R⁵ is cyano, nitro, halogen, C₁-C₄-alkoxycarbonyl, C₁-C₄-alkylcarbonyl, C₁-C₄-alkylcarbonyloxy, C₁-C₄-alkyl, C₁-C₄-haloalkyl, C₁-C₄-alkylthio, C₁-C₄-haloalkylthio, C₁-C₆-alkoxy, C₁-C₆-haloalkoxy, or R⁵ together with the carbon atom to which it is attached forms a carbonyl group.

4. A compound as claimed in any of claims 1 to 3, in which
R⁶, R⁷ independently of one another are hydrogen or C₁-C₄-alkyl;
R⁸ is hydrogen, C₁-C₄-alkyl, C₁-C₄-haloalkyl, C₁-C₄-alkylcarbonyl, C₁-C₄-haloalkylcarbonyl, C₁-C₄-alkoxycarbonyl, C₁-C₄-alkylsulfonyl, C₁-C₄-haloalkylsulfonyl, phenylcarbonyl, phenylcarbonylmethyl, phenyloxycarbonyl or phenylsulfonyl, where the phenyl ring of the four last-mentioned radicals is substituted by s radicals selected from the group consisting of halogen, nitro, cyano, C₁-C₄-alkyl, C₁-C₄-haloalkyl, C₁-C₄-alkoxy and C₁-C₄-haloalkoxy.

5. A compound as claimed in any of claims 1 to 4, in which
R¹ is chlorine, bromine, iodine, nitro, methyl, thiomethyl, thioethyl, methylsulfonyl, ethylsulfonyl or methoxy;
R² is bromine, chlorine, methylsulfonyl or ethylsulfonyl;
_{R}² is located in the 4-position of the phenyl ring;
R⁸ is hydrogen,
Het is 3-tetrahydrofuranyl, 3-tetrahydropyranyl, 4-tetrahydropyranyl, 1,3-dioxan-5-yl or γ-butyrolacton-2-yl.

6. A herbicidal composition which comprises a herbicidally effective amount of at least one compound of the formula (I) as claimed in any of claims 1 to 5.

7. A herbicidal composition as claimed in claim 6 in a mixture with formulation auxiliaries.

8. A method for controlling unwanted plants, which comprises applying an effective amount of at least one compound of the formula (I) as claimed in any of claims 1 to 5 or of a herbicidal composition as claimed in claim 6 or 7 to the plants or the site of the unwanted vegetation.

9. The use of a compound of the formula (I) as claimed in any of claims 1 to 5 or of a herbicidal composition as claimed in claim 6 or 7 for controlling unwanted plants.

10. The use as claimed in claim 9, wherein the compound of the formula (I) is used for controlling unwanted plants in crops of useful plants.

11. The use as claimed in claim 10, wherein the useful plants are transgenic useful plants.

## Revendications

1. Composés de formule (I) ou leurs sels 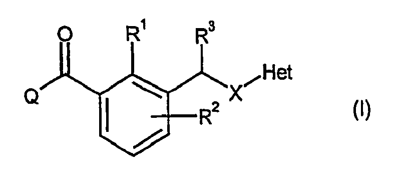 dans lesquels les radicaux et indices ont les significations suivantes :
R¹, R² désignent, indépendamment l'un de l'autre, un atome d'hydrogène, un groupe mercapto, nitro, halogène, cyano, rhodano, alkyle en C₁ à C₆, halogéno-alkyle en C₁ à C₆, alcényle en C₂ à C₆, halogéno-alcényle en C₂ à C₆, alcynyle en C₂ à C₆, halogéno-alcynyle en C₃ à C₆, cycloalkyle en C₃ à C₆, -OR⁴, OCOR⁴, OSO₂R⁴, S(O)ₙR⁴, SO₂OR⁴, SO₂N(R⁴)₂, NR⁴SO₂R⁴, NR⁴COR⁴, alkyle en C₁ à C₆-S(O)ₙR⁴, alkyle en C₁ à C₆-OR⁴, alkyle en C₁ à C₆-OCOR⁴, alkyle en C₁ à C₆-OSO₂R⁴, alkyle en C₁ à C₆-SO₂OR⁴, alkyle en C₁ à C₆-SO*₂*N*(*R⁴)₂ ou alkyle en C₁ à C₆-NR⁴COR⁴ ;
R³ désigne un atome d'hydrogène, un groupe alkyle en C₁ à C₆, alcényle en C₂ à C₆ ou alcynyle en C₂ à C₆ ;
R⁴ désigne un atome d'hydrogène, un groupe alkyle en C₁ à C₆, alcényle en C₂ à C₆, alcynyle en C₂ à C₆, cycloalkyle en C₃ à C₆, phényle ou phényl-alkyle en C₁ à C₆, les six derniers radicaux cités étant substitués par s radicaux des groupes hydroxy, mercapto, amino, cyano, nitro, rhodano, OR³, SR³, N(R³)₂, =NOR³, OCOR³, SCOR³, NR³COR³, CO₂R³, COSR³, CON(R³)₂, alkylimino-oxy en C₁ à C₄, alcoxy-amino en C₁ à C₄, alkylcarbonyle en C₁ à C₄, alcoxy en C₁ à C₄-alcoxycarbonyle en C₂ à C₆ et alkylsulfonyle en C₁ à C₄ ;
Het désigne un groupe hétérocyclique entièrement insaturé, dont les atomes cycliques sont des atomes de carbone et d'oxygène, dans lequel
le nombre total d'atomes cycliques est de p,
le nombre d'atomes d'oxygène est de r,
le nombre d'atomes de carbone est de (p-r), et
Het peut être substitué par n radicaux R⁵ ;
n vaut 0, 1 ou 2 ;
p vaut 5, 6 ou 7 ;
r vaut 1 ou 2 ;
s vaut 0, 1, 2 ou 3 ;
X désigne O ou S(O)ₙ ;
R⁵ désigne un groupe hydroxy, mercapto, amino, cyano, nitro, halogène, formyle, alkylamino en C₁ à C₆, dialkylamino en C₁ à C₆, alcoxycarbonyle en C₁ à C₆, alkylcarbonyle en C₁ à C₆, alkylcarbonyloxy en C₁ à C₄, alkyle en C₁ à C₆, halogéno-alkyle en C₁ à C₆, alkylthio en C₁ à C₆, halogéno-alkylthio en C₁ à C₆, alcoxy en C₁ à C₆, halogéno-alcoxy en C₁ à C₆ ou R⁵ forme, conjointement avec l'atome de carbone auquel il est lié, un groupe carbonyle ;
Q désigne un radical du groupe Q1 ou Q2 ; 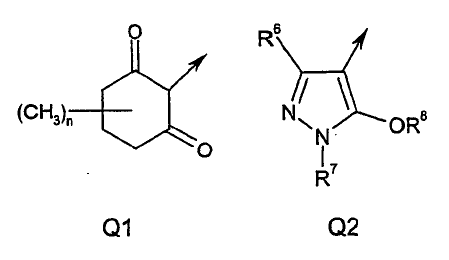
R⁶ et R⁷ désignent indépendamment l'un de l'autre, un atome d'hydrogène, un groupe alkyle en C₁ à C₆, halogéno-alkyle en C₁ à C₆ ou cyclopropyle en C₃ à C₆ ;
R⁸ désigne un atome d'hydrogène, un groupe alkyle en C₁ à C₆, halogéno-alkyle en C₁ à C₆, alkylcarbonyle en C₁ à C₆, halogéno-alkylcarbonyle en C₁ à C₆, alcoxycarbonyle en C₁ à C₆, alkylsulfonyle en C₁ à C₆, halogéno-alkylsulfonyle en C₁ à C₆, phénylcarbonyle, phénylcarbonylméthyle, phénylcarbonyle ou phénylsulfonyle, le noyau phényle des quatre derniers radicaux cités étant substitué par s radicaux du groupe comprenant un atome d'halogène, un groupe nitro, cyano, alkyle en C₁ à C₆, halogéno-alkyle en C₁ à C₆, alcoxy en C₁ à C₆ et halogéno-alcoxy en C₁ à C₆.

2. Composé selon la revendication 1, dans lequel
R¹, R² désignent indépendamment les uns des autres un atome d'hydrogène, un groupe nitro, un atome d'halogène, un groupe alkyle en C₁ à C₄, halogéno-alkyle en C₁ à C₄, alcényle en C₂ à C₆, halogéno-alkyle en C₂ à C₆, alcynyle en C₂ à C₆, halogéno-alcynyle en C₂ à C₆, cycloalkyle en C₃ à C₆, -OR⁴, S(O)ₙR⁴, SO₂OR⁴, SO₂N(R⁴)₂, NR⁴SO₂R⁴ ou alkyle en C₁ à C₆-S (O)ₙR⁴ ;
R⁴ désigne un atome d'hydrogène, un groupe alkyle en C₁ à C₄, alcényle en C₂ à C₄, alcynyle en C₂ à C₄, cycloalkyle en C₃ à C₆, phényle ou phényl-alkyle en C₁ à C₄, dans lequel les six derniers radicaux cités sont substitués par s radicaux du groupe cyano, nitro, R³, OR³ ou N(R³)₂.

3. Composés selon la revendication 1 ou 2, dans lesquels
R³ désigne un atome d'hydrogène ;
R⁵ désigne un groupe cyano, nitro, halogène, alcoxycarbonyle en C₁ à C₄, alkylcarbonyle en C₁ à C₄, alkylcarbonyloxy en C₁ à C₄, alkyle en C₁ à C₄, halogéno-alkyle en C₁ à C₄, alkylthio en C₁ à C₄, halogéno-alkylthio en C₁ à C₄, alcoxy en C₁ à C₆, halogéno-alcoxy en C₁ à C₆ ou R⁵ forme conjointement avec l'atome de carbone auquel il est lié, un groupe carbonyle.

4. Composés selon l'une quelconque des revendications 1 à 3, dans lesquels
R⁶, R⁷ désignent indépendamment l'un de l'autre un atome d'hydrogène ou un groupe alkyle en C₁ à C₄ ;
R⁸ désigne un atome d'hydrogène, un groupe alkyle en C₁ à C₄, halohéno-alkyle en C₁ à C₄, alkylcarbonyle en C₁ à C₄, halogéno-alkylcarbonyle en C₁ à C₄, alcoxycarbonyle en C₁ à C₄, alkylsulfonyle en C₁ à C₄, halogénoalkylsulfonyle en C₁ à C₄, phénylcarbonyle, phénylcarbonylméthyle, phényloxycarbonyle ou phénylsulfonyle, le noyau phényle des quatre derniers radicaux cités étant substitué par s radicaux du groupe comprenant un atome d'halogène, un groupe nitro, cyano, alkyle en C₁ à C₄, halogéno-alkyle en C₁ à C₄, alcoxy en C₁ à C₄ et halogéno-alcoxy en C₁ à C₄.

5. Composés selon l'une quelconque des revendications 1 à 4, dans lesquels
R¹ désigne un atome de chlore, de brome, d'iode, un groupe nitro, méthyle, thiométhyle, thioéthyle, méthylsulfonyle, éthylsulfonyle ou méthoxy,
R² désigne un atome de brome, de chlore, un groupe méthylsulfonyle ou éthylsulfonyle ;
R² se trouve en position 4 du cycle phényle;
R⁸ désigne un atome d'hydrogène,
Het désigne un groupe 3-tétrahydrofuranyle, 3-tétrahydropyranyle, 4-tétrahydropyranyle, 1,3-dioxan-5-yle ou γ-butyrolacton-2-yle.

6. Agent herbicide, **caractérisé par** une teneur efficace d'un point de vue herbicide, d'au moins un composé de formule générale (I) selon l'une quelconque des revendications 1 à 5.

7. Agent herbicide selon la revendication 6, en mélange avec des adjuvants de formulation.

8. Procédé pour lutter contre les plantes indésirables, **caractérisé en ce que** l'on applique une quantité efficace d'au moins un composé de formule générale (I) selon l'une quelconque des revendications 1 à 5 ou d'un agent herbicide selon la revendication 6 ou 7 sur les plantes ou sur le lieu de croissance indésirable des plantes.

9. Utilisation de composés de formule générale (I) selon l'une quelconque des revendications 1 à 5, ou d'agents herbicides selon la revendication 6 ou 7, pour lutter contre les plantes indésirables.

10. Utilisation selon la revendication 9, **caractérisée en ce que** les composés de formule générale (I) sont utilisés pour lutter contre les plantes indésirables dans des cultures de plantes utiles.

11. Utilisation selon la revendication 10, **caractérisée en ce que** les plantes utiles sont des plantes utiles transgéniques.
